(19) Europäisches Patentamt
European Patent Office
Office européen des brevets

(11) Veröffentlichungsnummer: **0 161 218 B1**

(12) **EUROPÄISCHE PATENTSCHRIFT**

(45) Veröffentlichungstag der Patentschrift: **26.06.91**

(21) Anmeldenummer: **85810208.0**

(22) Anmeldetag: **06.05.85**

(51) Int. Cl.5: **C07D 491/052**, C07D 495/04, C07D 491/153, C07D 495/14, A61K 31/435, //(C07D491/052, 311:00,221:00),(C07D495/04, 335:00,221:00)

# (54) Benzo-(pyrano und thiopyrano)-pyridine.

(30) Priorität: **10.05.84 US 609037**

(43) Veröffentlichungstag der Anmeldung: **13.11.85 Patentblatt 85/46**

(45) Bekanntmachung des Hinweises auf die Patenterteilung: **26.06.91 Patentblatt 91/26**

(84) Benannte Vertragsstaaten: **AT BE CH DE FR GB IT LI LU NL SE**

(56) Entgegenhaltungen:
**GB-A- 1 143 819**
**US-A- 3 514 464**

(73) Patentinhaber: **CIBA-GEIGY AG**
**Klybeckstrasse 141**
**CH-4002 Basel(CH)**

(72) Erfinder: **Hutchison, Alan Jeffrey**
**35 Lynwood Road**
**Verona New Jersey 07044(US)**

**EP 0 161 218 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft neue 4H-[1]-Benzopyrano[3,4-b]pyridin- und 4H-[1]-Benzothiopyrano[3,4-b]pyridin-Verbindungen der Formel I,

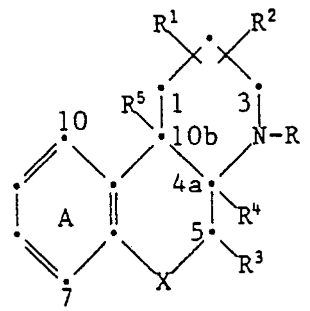

(I)

worin X Sauerstoff oder Schwefel bedeutet, Ring A unsubstituiert oder durch einen bis zwei gleiche oder verschiedene Substituenten aus der Gruppe Hydroxy, Hydroxy-$C_{1-4}$-alkyl, verethertes Hydroxy, $C_{1-4}$-Alkoxy-$C_{1-4}$-alkyl, Acyloxy, $C_{1-4}$-Alkanoyloxy-$C_{1-4}$-alkyl, Halogen, $C_{1-4}$-Alkyl, Trifluormethyl, Amino, Mono- oder Di-$C_{1-4}$-Alkylamino und Acylamino substituiert ist, oder Ring A durch ein $C_{1-4}$-Alkylendioxy substituiert ist, R Wasserstoff, $C_{1-4}$-Alkyl, Benzyl oder Phenethyl bedeutet, $R^1$ für Wasserstoff, $C_{1-4}$-Alkyl, $C_{1-4}$-Alkylthio-$C_{1-4}$-alkyl, Amino, Acylamino, (Amino, Mono- oder Di-$C_{1-4}$-alkylamino)-$C_{1-4}$-alkyl, Carboxy, $C_{1-4}$-Alkoxycarbonyl, Carbamoyl oder Mono- oder Di-$C_{1-4}$-alkylcarbamoyl steht, sowie $R^2$ bis $R^5$ Wasserstoff oder $C_{1-4}$-Alkyl bedeuten, ferner deren Dehydroderivate mit einer Doppelbindung in 1,2-Position oder in 1,10b-Position, wobei in letzterem Fall $R^5$ nicht vorhanden ist; deren Salze, insbesondere pharmazeutisch annehmbare Salze; Verfahren zur Herstellung dieser Verbindungen, pharmazeutische Präparate, die diese Verbindungen enthalten, sowie ihre therapeutische Anwendung.

Die 1,10b-Dehydroderivate der Verbindungen der Formel I weisen die Formel Ia, die 1,2-Dehydroderivate der Verbindungen der Formel I die Formel Ib auf,

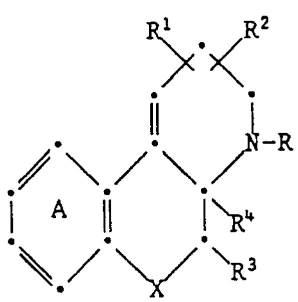

(Ia)

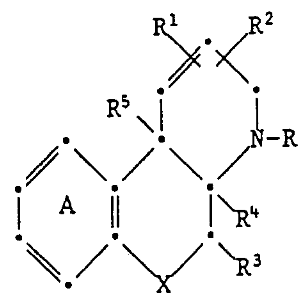

(Ib)

worin X, Ring A, R und $R^1$ bis $R^5$ wie vorstehend definiert sind.

Im Stand der Technik sind schon andere 4H-[1]-Benzopyranopyridin-Verbindungen mit Wirkungen auf das Zentralnervensystem beschrieben. Die aus US-Patent 3 514 464 und aus GB-Patent 1 143 819 bekannten Verbindungen unterscheiden sich von den vorliegenden Verbindungen jedoch mindestens in der Position des Stickstoffs im annellierten Pyridinring (in Position 2 oder 3 statt 4) und in der Substitution an C-5 in Nachbarschaft zum Heteroatom X (zwei geminale Niederalkylreste statt mindestens ein Wasserstoffatom).

Eine besondere Ausführungsform der Erfindung wird durch Verbindungen der Formeln I, Ia und Ib dargestellt, in denen X Sauerstoff bedeutet, sowie durch deren Salze. Eine andere Ausführungsform der Erfindung wird durch Verbindungen der Formeln I, Ia und Ib dargestellt, in denen X Schwefel bedeutet, sowie durch deren Salze.

Bevorzugt sind Verbindungen der Formeln I, Ia und Ib, worin X Sauerstoff oder Schwefel bedeutet, Ring A unsubstituiert oder durch einen bis zwei Substituenten aus der Gruppe Hydroxy, Acyloxy, $C_{1-4}$-Alkoxy, Hydroxymethyl, $C_{1-4}$-Alkyl, $C_{1-4}$-Alkanoyloxymethyl, $C_{1-4}$-Alkoxymethyl, Halogen und Trifluormethyl substituiert ist, oder Ring A durch $C_{1-4}$-Alkylendioxy monosubstituiert ist und R und $R^1$ bis $R^5$ Wasserstoff oder $C_{1-4}$-Alkyl bedeuten, sowie pharmazeutisch annehmbare Salze davon.

Weiterhin bevorzugt sind Verbindungen der Formel II,

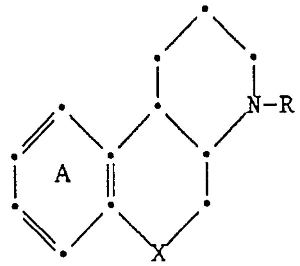

(II)

worin X Sauerstoff oder Schwefel bedeutet, Ring A unsubstituiert oder durch einen bis zwei gleiche oder verschiedene Substituenten aus der Gruppe Hydroxy, Hydroxymethyl, Acyloxy, $C_{1-4}$-Alkanoyloxymethyl, $C_{1-4}$-Alkyl, $C_{1-4}$-Alkoxy und Halogen substituiert ist und R $C_{1-4}$-Alkyl bedeutet, deren 1,10b-Dehydroderivate und pharmazeutisch annehmbare Salze davon.

Besonders bevorzugt sind Verbindungen der Formel II, worin X Sauerstoff bedeutet, Ring A durch Hydroxy, $C_{1-4}$-Alkanoyloxy, Benzoyloxy oder Pyridylcarbonyloxy monosubstituiert ist und R $C_{1-4}$-Alkyl bedeutet, sowie pharmazeutisch annehmbare Salze davon.

Eine weitere bevorzugte Ausführungsform der Erfindung bezieht sich auf Verbindungen der Formel III,

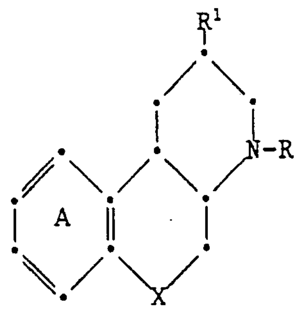

(III)

worin X Sauerstoff oder Schwefel bedeutet, Ring A unsubstituiert oder durch einen bis zwei gleiche oder verschiedene Substituenten aus der Gruppe Hydroxy, Hydroxymethyl, Acyloxy, $C_{1-4}$-Alkanoyloxymethyl, $C_{1-4}$-Alkoxy und Halogen substituiert ist, R $C_{1-4}$-Alkyl bedeutet, $R^1$ für $C_{1-4}$-Alkylthio-$C_{1-4}$-alkyl, $C_{1-4}$-Alkanoylamino, (Amino, Mono- oder Di-$C_{1-4}$-alkylamino)-$C_{1-4}$-alkyl, Carboxy, $C_{1-4}$-Alkoxycarbonyl, Carbamoyl oder Mono- oder Di-$C_{1-4}$-Alkylcarbamoyl steht, und pharmazeutisch annehmbare Salze davon.

Die Verbindungen der Formeln I, Ib, II oder III können cis- oder trans-verknüpfte Ringe aufweisen. Bevorzugt sind die entsprechenden Verbindungen mit einer trans-4a,10b-Ringverknüpfung.

Je nach der Art von $R^1$ bis $R^5$ und der sich daraus ergebenden Anzahl asymmetrischer Kohlenstoffatome können die Verbindungen der Formeln I, II oder III auch in Form einer Anzahl von Racematen und optischen Antipoden davon vorliegen. So können die erfindungsgemässen Verbindungen als Stereoisomere, z.B. geometrische Isomere, als Racemate, reine Enantiomere oder Mischungen davon auftreten. Alle diese Isomere oder Gemische von Isomeren gehören zum Gegenstand der Erfindung.

Die verwendeten allgemeinen Definitionen haben im Rahmen der vorliegenden Erfindung die folgenden Bedeutungen: Der Terminus "$C_{1-4}$-", der vor- und nachstehend im Zusammenhang mit organischen Resten oder Verbindungen verwendet wird, definiert solche mit bis zu und einschliesslich 4 und insbesondere ein oder zwei Kohlenstoffatomen.

$C_{1-4}$-Alkyl enthält 1 bis 4 Kohlenstoffatome und bedeutet z.B. Ethyl, Propyl, Butyl, insbesondere Methyl.

$C_{1-4}$-Alkoxy enthält 1 bis 4 Kohlenstoffatome und bedeutet z.B. Ethoxy, Propoxy oder insbesondere Methoxy.

$C_{1-4}$-Alkylthio enthält 1 bis 4 Kohlenstoffatome und bedeutet z.B. Ethylthio, Propylthio oder insbesondere Methylthio.

$C_{1-4}$-Alkoxycarbonyl enthält 1 bis 4 Kohlenstoffatome im Alkoxyteil und bedeutet z.B. Methoxycarbonyl, Propoxycarbonyl, Isopropoxycarbonyl oder insbesondere Ethoxycarbonyl.

Acyl in Acyloxy bedeutet $C_{1-4}$-Alkanoyl, Aroyl, $C_{1-4}$-Alkoxycarbonyl, Carbamoyl oder Mono- oder Di-$C_{1-4}$-Alkylcarbamoyl. Acyl in Acylamino bedeutet $C_{1-4}$-Alkanoyl, Benzoyl oder $C_{1-4}$-Alkoxycarbonyl. $C_{1-4}$-

Alkanoyl bedeutet vorzugsweise Acetyl, Propionyl oder Butyryl.

Aroyl bedeutet Benzoyl, Benzolsulfonyl oder 2-, 3- oder 4-Pyridylcarbonyl, insbesondere Nicotinoyl.

$C_{1-4}$-Alkanoyloxy ist vorzugsweise Acetoxy oder Propionyloxy; $C_{1-4}$-Alkanoylamino ist vorzugsweise Acetamido oder Propionamido; Aroyloxy ist vorzugsweise Benzolsulfonyloxy, Benzoyloxy oder 2-, 3- oder 4-Pyridylcarbonyloxy, insbesondere Nicotinoyloxy.

$C_{1-4}$-Alkanoyloxy-$C_{1-4}$-alkyl ist vorzugsweise $C_{1-4}$-Alkanoyloxymethyl.

$C_{2-4}$-Alkyl, das eine zusätzliche ungesättigte Bindung aufweist, steht für $C_{2-4}$-Alkenyl oder $C_{2-4}$-Alkinyl.

Halogen ist vorzugsweise Fluor oder Chlor, kann aber auch Brom oder Iod sein.

$C_{2-4}$-Alkenyl bedeutet Alkenyl mit 2 bis 4 Kohlenstoffatomen, insbesondere Allyl oder Crotonyl.

$C_{2-4}$-Alkinyl bedeutet Alkinyl mit 2 bis 4 Kohlenstoffatomen, insbesondere Propargyl.

$C_{1-4}$-Alkylendioxy bedeutet vorzugsweise Ethylendioxy oder Methylendioxy.

Hydroxy-$C_{1-4}$-alkyl bedeutet vorzugsweise Hydroxymethyl, Hydroxyethyl oder Hydroxypropyl, insbesondere Hydroxymethyl.

Verethertes Hydroxy bedeutet $C_{1-4}$-Alkoxy, z.B. Methoxy oder Ethoxy, $C_{2-4}$-Alkenyloxy, z.B. Allyloxy, $C_{2-4}$-Alkinyloxy, z.B. Propargyloxy, $C_{3-6}$-Cycloalkyl-$C_{1-2}$-alkoxy, z.B. Cyclopropylmethoxy, oder Benzyloxy.

Acylamino bedeutet $C_{1-4}$-Alkanoylamino, Benzoylamino oder $C_{1-4}$-Alkoxycarbonylamino, worin die jeweiligen Gruppen die vorstehend definierten Bedeutungen haben.

Acyloxy bedeutet $C_{1-4}$-Alkanoyloxy, Aroyloxy, $C_{1-4}$-Alkoxycarbonyloxy, Carbamoyloxy oder Mono- oder Di-$C_{1-4}$-alkylcarbamoyloxy, worin die jeweiligen Gruppen die vorstehend definierten Bedeutungen haben.

Mono- oder Di-$C_{1-4}$-alkylamino ist vorzugsweise Mono- oder Di-(methyl, ethyl, propyl)-amino. Mono- oder Di-$C_{1-4}$-alkylcarbamoyl ist vorzugsweise Mono- oder Di-(methyl, ethyl, propyl)-carbamoyl.

Pharmazeutisch annehmbare Salze sind Säureadditionssalze, vorzugsweise solche von therapeutisch akzeptablen anorganischen oder organischen Säuren, wie starken Mineralsäuren, z.B. Halogenwasserstoffsäuren, wie Chlorwasserstoffsäure oder Bromwasserstoffsäure, Schwefelsäure, Phosphor- oder Salpetersäure, ferner solche von aliphatischen oder aromatischen Carbon- oder Sulfonsäuren, z.B. Ameisensäure, Essigsäure, Propionsäure, Bernsteinsäure, Glykolsäure, Milchsäure, Malonsäure, Weinsäure, Glukonsäure, Zitronensäure, Maleinsäure, Fumarsäure, Brenztraubensäure, Phenylessigsäure, Benzoesäure, 4-Aminobenzoesäure, Anthranilsäure, 4-Hydroxybenzoesäure, Salicylsäure, 4-Aminosalicylsäure, Pamoasäure, Nicotinsäure, Methansulfonsäure, Ethansulfonsäure, Hydroxyethansulfonsäure, Benzolsulfonsäure, p-Toluolsulfonsäure, Naphthalinsulfonsäure, Sulfanilsäure, Cyclohexylsulfaminsäure oder Ascorbinsäure.

Zusätzlich zu den pharmazeutisch annehmbaren Salzen sind auch Prodrug-Derivate ein weiterer Gegenstand dieser Erfindung, z.B. pharmazeutisch annehmbare Ester von Phenolen oder Alkoholen (Verbindungen der Formeln I, II oder III sowie Dehydroderivaten davon, deren Ring A durch Hydroxy oder Hydroxy-$C_{1-4}$-Alkyl substituiert ist) dieser Erfindung, die durch Solvolyse oder unter physiologischen Bedingungen in besagte Phenole oder Alkohole umgewandelt werden können.

Solche Prodrug-Derivate sind vorzugsweise $C_{1-7}$-Alkanoylester, z.B. Acetyl-, Isobutyryl- oder Pivaloylester, Aroylester, z.B. Benzoyl- oder Nicotinoylester, Carbamoylester (Urethane), z.B. Mono- oder Diethylcarbamoyl- oder Mono- oder Diethylcarbamoylester.

Die Verbindungen dieser Erfindung sind in bekannten in vitro und in vivo Testanordnungen wirksam, anhand derer sich Aussagen über die Wirksamkeit in der Behandlung von Krankheiten des zentralen Nervensystems (ZNS) von Säugern inklusive Menschen machen lassen. Selektive präsynaptische Dopaminrezeptoragonisten und adrenerge $\alpha_2$-Rezeptoragonisten können z.B. für die Behandlung von Dyskinesie, Parkinsonismus oder psychotischen Zuständen wie Schizophrenie verwendet werden. Serotoninrezeptoragonisten und $\alpha_2$-Rezeptorantagonisten können z.B. für die Behandlung von Depression, Wahrnehmungsschwächen und kleineren Gehirnmangelfunktionen eingesetzt werden.

Die Verbindungen dieser Erfindung besitzen daher wertvolle pharmakologische Eigenschaften in Säugern, insbesondere ZNS-modulierende oder psychopharmakologische Eigenschaften, z.B. vorzugsweise neuroleptische und/oder antidepressive Wirkungen unter anderem durch Modulierung präsynaptischer Dopaminrezeptoren, und/oder präsynaptischer adrenerger $\alpha_2$-Rezeptoren und/oder Serotoninrezeptoren im Gehirn. So ist ihre Eigenschaft, selektiv diepräsynaptischen Dopaminrezeptoren zu stimulieren (agonistisch), ein Zeichen für z.B. neuroleptische (antipsychotische) Aktivität, ihre Eigenschaft, Serotoninrezeptoren zu stimulieren (agonistisch) und jene, adrenerge $\alpha_2$-Rezeptoren zu blockieren (antagonistisch), ein Zeichen für z.B. antidepressive Aktivität.

Die vorstehend erwähnten Eigenschaften lassen sich in in vitro und in vivo Testanordnungen zeigen, wobei man vorzugsweise Säuger, z.B. Ratten, Hunde, Affen oder aus ihnen isolierte Organe, Gewebe oder

Präparate verwendet. Besagte Verbindungen können in vitro in Form von Lösungen, z.B. vorzugsweise wässrigen Lösungen, und in vivo enteral oder parenteral, vorzugsweise oral oder intravenös, z.B. in Gelatinekapseln, als Stärkesuspension oder in wässriger Lösung eingesetzt werden. Die Dosis kann in vitro im Bereich von Konzentrationen von $10^{-4}$ molar bis $10^{-9}$ molar liegen. Die Dosis in vivo kann zwischen ca. 0,01 und 50 mg/kg und Tag, vorzugsweise zwischen ca. 0,05 und 30 mg/kg und Tag, insbesondere zwischen ca. 0,1 und 20 mg/kg und Tag liegen.

Die Eigenschaft der Verbindungen dieser Erfindung, an präsynaptische Dopaminrezeptoren zu binden, die eine Regulierung solcher präsynaptischer Dopaminrezeptoren anzeigt, z.B. eine agonistische Aktivität, wird durch den Dopamin-Bindungstest in vitro gezeigt. Diese Methode beruht auf der Verdrängung des Dopaminagonisten 2-Amino-6,7-dihydroxy-1,2,3,4-tetrahydronaphthalin ($^3$H-ADTN) von Nukleus caudatum Membranen vom Kalb [Eur. J. Pharmacol. 55, 137 (1979)].

Die Aktivität als Agonist an präsynaptischen Dopaminrezeptoren (sie wird auch Dopamin-Autorezeptoragonistische Aktivität genannt) weist auf neuroleptische Aktivität der Verbindungen der Erfindung hin. Sie lässt sich nach dem Ratten-Gamma-Butyrolacton Modell (GBL) gemäss dem Verfahren von Walters und Roth, Naunyn Schmiedebergs Arch. Pharmacol. 296, 5 (1976) bestimmen. Nach diesem Modell inhibiert ein präsynaptischer Dopaminagonist die durch GBL verursachte Konzentrationserhöhung des Dopaminvorläufers DOPA nach Vorbehandlung mit 3-Hydroxybenzylhydrazin (NSD-1015), einem DOPA-Decarboxylaseinhibitor.

Die Selektivität der Verbindungen der Erfindung hinsichtlich ihrer Aktivität als präsynaptische Dopaminagonisten lässt sich in vitro durch Bindungsuntersuchungen bestimmen, bei denen $^3$H-Spiroperidol von postsynaptischen Dopaminrezeptoren verdrängt wird. Eine schwache Bindung in diesem Test weist auf Selektivität hin.

In vivo kann diese Selektivität dadurch bestimmt werden, dass man an der Ratte das Ausmass der Beeinflussung der durch Reserpin verursachten Hypomotilität misst. Das relative Fehlen solcher Beeinflussung bei wirksamen Dosen (z.B. im GBL-Modell) zeigt selektive präsynaptische Dopamin-agonistische Aktivität an.

Die Serotonin-Bindungseigenschaften der Verbindungen der Erfindung, die eine Aktivität im Zusammenhang mit agonistischer Serotoninrezeptor-Regulierung anzeigen, lassen sich durch einen in vitro Bindungstest nach dem Verfahren von Bennett und Snyder zeigen [Mol. Pharmacol. 12, 273 (1976)].

Die agonistische Serotoninrezeptor-Aktivität lässt sich in vivo bestimmen, indem man nach J. Med. Chem. 21, 864 (1978) die Abnahme der Konzentrationssteigerung von 5-Hydroxytryptophan im Rattenhirn misst, nachdem eine Testverbindung verabreicht wurde.

Die vorstehend geschilderten vorteilhaften Eigenschaften machen die Verbindungen der Erfindung wertvoll als therapeutische Stoffe mit psychotropen Eigenschaften. Sie weisen selektive ZNS-modulierende Aktivität auf und sind daher in Säugern nützlich, insbesondere als psychoaktive Mittel und, je nach ihren speziellen Wirkungen auf ZNS-Rezeptoren, z.B. als Neuroleptika (Antipsychotika) zur Behandlung psychotischer Zustände (Schizophrenie), als Psychostimulantien für die Behandlung von Depression, Wahrnehmungsschwächen (senile Demenz) und kleineren Gehirnmangelfunktionen, als Anxiolytika zur Behandlung von Angstzuständen, sowie als Appetitzügler zur Behandlung von Fettleibigkeit.

Insbesondere nützlich sind Verbindungen der Formel II, speziell die entsprechenden Isomere mit einer trans-Ringverknüpfung, oder die 1,10b-Dehydroderivate davon, in denen X Sauerstoff oder Schwefel bedeutet, R für $C_{3-4}$-Alkyl steht, Ring A in der 7-, 8- oder 9-Position durch Hydroxy, Acyloxy, Hydroxymethyl oder $C_{1-4}$-Alkanoyloxymethyl monosubstituiert ist, oder worin Ring A in 7,8-, 7,9- oder 8,9-Position disubstituiert ist, wobei ein Substituent Hydroxy oder Acyloxy und der andere Hydroxy, Acyloxy oder Halogen ist, und ihre pharmazeutisch anwendbaren Salze.

Bevorzugt sind Verbindungen der Formel II, speziell die entsprechenden Isomere mit einer trans-Ringverknüpfung, oder die 1,10b-Dehydroderivate davon, in denen X Sauerstoff oder Schwefel bedeutet, R für $C_{3-4}$-Alkyl steht, Ring A in der 7-, 8- oder 9-Position durch Hydroxy oder Acyloxy monosubstituiert ist, oder worin Ring A in 7,8-, 7,9- oder 8,9-Position disubstituiert ist, wobei ein Substituent Hydroxy und der andere Hydroxy oder Halogen ist, und ihre pharmazeutisch anwendbaren Salze.

Bevorzugt sind auch Verbindungen der Formel II, insbesondere deren Isomere mit trans-Ringverknüpfung, in denen X Sauerstoff bedeutet, R für n-Propyl oder n-Butyl steht und Ring A in der 7-, 8- oder 9-Position durch Hydroxy, $C_{1-4}$-Alkanoyloxy, Aroyloxy, Hydroxymethyl oder $C_{1-4}$-Alkanoyloxymethyl substituiert ist, und ihre pharmazeutisch annehmbaren Salze.

Bevorzugt sind auch Verbindungen der Formel II, insbesondere deren Isomere mit trans-Ringverknüpfung, in denen X Sauerstoff bedeutet, R für n-Propyl oder n-Butyl steht, Ring A in der 7-, 8- oder 9-Position durch Hydroxy, $C_{1-4}$-Alkanoyloxy oder Aroyloxy substituiert ist, und ihre pharmazeutisch annehmbaren Salze.

Stark bevorzugt sind Verbindungen der Formel II, insbesondere deren Isomere mit trans-Ringverknüpfung, in denen X Sauerstoff bedeutet, Ring A in der 7- oder 9-Position durch Hydroxy, $C_{1-4}$-Alkanoyloxy, Benzoyloxy oder Nicotinoyloxy monosubstituiert ist, R für n-Propyl oder n-Butyl steht, und ihre pharmazeutisch annehmbaren Salze.

Eine andere bevorzugte Ausführungsform der Erfindung betrifft Verbindungen der Formel II, insbesondere deren Isomere mit trans-Ringverknüpfung, in denen X Sauerstoff bedeutet, R für Methyl, Ethyl oder n-Propyl steht, Ring A insbesondere in der 7- oder 10-Position durch Hydroxy, $C_{1-4}$-Alkoxy, $C_{1-4}$-Alkanoyloxy, Benzoyloxy oder Nicotinoyloxy monosubstituiert ist, oder in denen Ring A vorzugsweise in 7,8-, 7,10- oder 8,10-Position disubstituiert ist, wobei ein Substituent Hydroxy oder $C_{1-4}$-Alkoxy und der andere $C_{1-4}$-Alkyl oder Halogen ist, und ihre pharmazeutisch annehmbaren Salze.

Weiter bevorzugt sind Verbindungen der Formel II, insbesondere deren Isomere mit trans-Ringverknüpfung, in denen X Sauerstoff bedeutet, R für Methyl oder n-Propyl steht, Ring A insbesondere in der 7- oder 10-Position durch Hydroxy, $C_{1-4}$-Alkoxy oder Benzoyloxy monosubstituiert ist, oder in denen Ring A vorzugsweise in 7,8-, 7,10- oder 8,10-Position disubstituiert ist, wobei ein Substituent Hydroxy oder $C_{1-4}$-Alkoxy und der andere $C_{1-4}$-Alkyl oder Halogen ist, und ihre pharmazeutisch annehmbaren Salze.

Die Verbindungen der Formeln I, Ia und Ib werden unter Anwendung konventioneller chemischer Verfahren hergestellt, indem man z.B.

a) eine Verbindung der Formel IV,

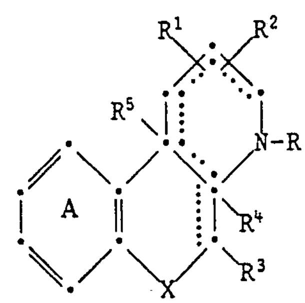

(IV)

in der gestrichelte Linien die Lage von einer, zwei oder drei nicht kumulierten Doppelbindungen angeben, X, eventuelle Substituenten von Ring A, R und $R^1$ bis $R^5$ wie vorstehend definiert sind, mit der Massgabe, dass $R^4$ und $R^5$ nur anwesend sind, wenn die Kohlenstoffatome, an die sie gebunden sind, nicht zu einer Doppelbindung gehören, mit einem geeigneten Reduktionsmittel reduziert, oder

b) eine Verbindung der Formel V,

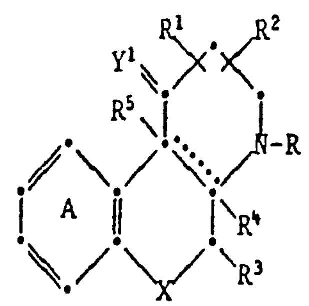

(V)

in der die gestrichelte Linie die Lage einer gegebenenfalls vorhandenen Doppelbindung angibt, X, eventuelle Substituenten von Ring A, R und $R^1$ bis $R^5$ wie vorstehend definiert sind, mit der Massgabe, dass $R^4$ und $R^5$ nur anwesend sind, wenn die Kohlenstoffatome, an die sie gebunden sind, nicht zu einer Doppelbindung gehören, und worin $Y^1$ Oxo oder geschütztes Oxo bedeutet, oder worin $Y^1$ verethertes, verestertes oder freies Hydroxy zusammen mit Wasserstoff bedeutet, mit einem geeigneten Reduktionsmittel und/oder Eliminierungsreagens behandelt, oder

c) eine Verbindung der Formel VI,

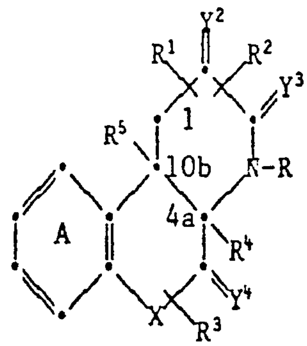

(VI),

worin X, eventuelle Substituenten von Ring A, R und $R^1$ bis $R^5$ wie vorstehend definiert sind, und worin einer der Reste $Y^2$, $Y^3$ und $Y^4$ Oxo oder geschütztes Oxo bedeutet, begleitet oder nicht begleitet von einer konjugierten endocyclischen Kohlenstoff-Kohlenstoff-Doppelbindung, oder für den Fall, dass $Y^3$ Oxo oder geschütztes Oxo bedeutet, auch begleitet von einer 1,10b- oder einer 4a,10b-Doppelbindung, und worin von den beiden verbleibenden Resten $Y^2$ bis $Y^4$, je nachdem, ob weitere Kohlenstoff-Kohlenstoff-Doppelbindungen vorhanden sind, $Y^2$ und $Y^3$ einen oder zwei Reste aus der Gruppe bestehend aus einem Rest $R^1$, einem Rest $R^2$ und Wasserstoff bedeuten, und/oder $Y^4$ für $R^3$ und Wasserstoff steht, mit einem geeigneten Reduktionsmittel reduziert, oder

d) in einer Verbindung der Formel VII,

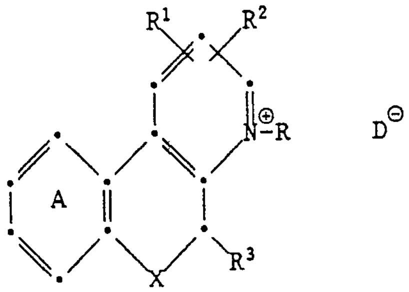

(VII)

worin X, eventuelle Substituenten von Ring A, R und $R^1$ bis $R^3$ wie vorstehend definiert sind, und $D^\ominus$ das Anion einer organischen oder anorganischen Säure ist, den Pyridiniumring mit einem geeigneten Reduktionsmittel reduziert, oder

e) eine Verbindung der Formel VIII,

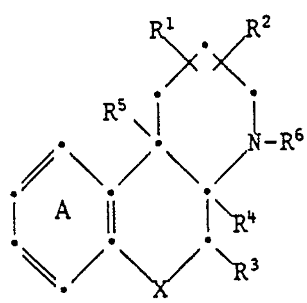

(VIII)

in der X, eventuelle Substituenten von Ring A und $R^1$ bis $R^5$ wie vorstehend definiert sind, und $R^6$ $C_{2-4}$-Alkenyl, $C_{2-4}$-Alkinyl, $C_{1-4}$-Alkanoyl oder Benzoyl bedeutet, mit einem geeigneten Reduktionsmittel reduziert, oder

f) eine Verbindung der Formel IX,

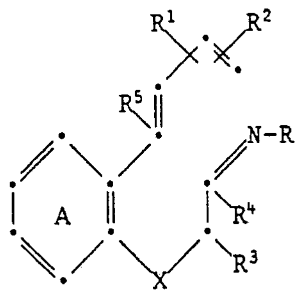

(IX)

in der X, eventuelle Substituenten von Ring A, R und $R^1$ bis $R^5$ wie vorstehend definiert sind, zu einer Verbindung der Formel Ib cyclisiert und das erhaltene Produkt gewünschtenfalls reduziert, oder

g) eine Verbindung der Formel X,

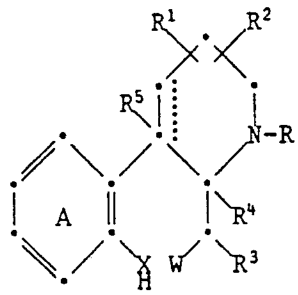

(X)

in der die gestrichelte Linie die Lage einer gegebenenfalls vorhandenen Doppelbindung angibt, X, eventuelle Substituenten von Ring A, R und $R^1$ bis $R^5$ wie vorstehend definiert sind, mit der Massgabe, dass $R^5$ nur anwesend ist, wenn an der durch die gestrichelte Linie bezeichneten Position eine Einfachbindung vorliegt, und worin W für reaktives verestertes Hydroxy steht, cyclisiert, oder

h) eine Verbindung der Formel XI,

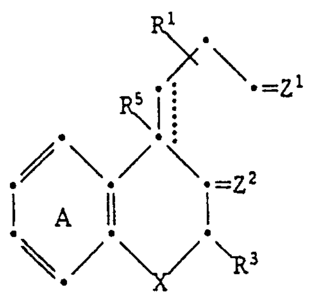

(XI)

in der die gestrichelte Linie die Lage einen gegebenenfalls vorhandenen Doppelbindung angibt, X, eventuelle Substituenten von Ring A, R und $R^1$ bis $R^5$ wie vorstehend definiert sind, und worin einer der Reste $Z^1$ und $Z^2$ Oxo oder reaktives verestertes Hydroxy W zusammen mit Wasserstoff oder $C_{1-4}$-Alkyl bedeutet, und der andere der beiden Reste $Z^1$ und $Z^2$ für -NHR zusammen mit Wasserstoff oder $C_{1-4}$-Alkyl steht, mit der Massgabe, dass $R^5$ nur anwesend ist, wenn an der durch die gestrichelte Linie bezeichneten Position eine Einfachbindung vorliegt, cyclisiert und das erhaltene Produkt gewünschtenfalls reduziert, oder

i) eine Verbindung der Formel XII,

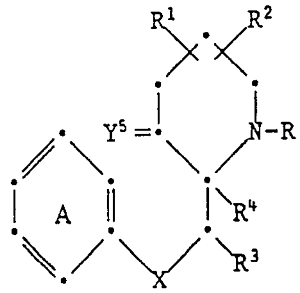

(XII)

in der X, eventuelle Substituenten von Ring A, R und $R^1$ bis $R^4$ wie vorstehend definiert sind, $Y^5$ für Oxo oder für reaktives verestertes Hydroxy W zusammen mit Wasserstoff oder $C_{1-4}$-Alkyl steht, cyclisiert und das erhaltene Produkt gewünschtenfalls reduziert, oder

j) eine Verbindung der Formel XIII,

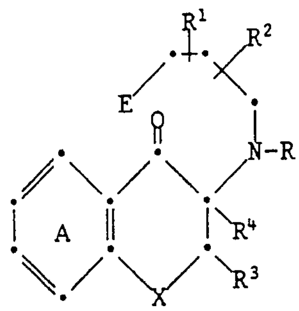

(XIII)

in der X, eventuelle Substituenten von Ring A, R und $R^1$ bis $R^4$ wie vorstehend definiert sind und E eine α-Carbanionen stabilisierende Abgangsgruppe ist, cyclisiert und das erhaltene Produkt gewünschtenfalls reduziert,

und indem man diese Verfahren, wenn nötig, unter zwischenzeitlichem Schutz von reaktionsfähigen funktionellen Gruppen, deren Reaktivität sich störend auswirken würde, durchführt, und dann die erhaltene Verbindung der Formel I, Ia oder Ib nach entsprechender Schutzgruppenabspaltung isoliert, und, wenn erwünscht, eine erhaltene Verbindung der Formel I, Ia oder Ib in eine andere Verbindung der Erfindung umwandelt, und/oder, wenn erwünscht, eine erhaltene freie Verbindung in ein Salz oder ein erhaltenes Salz in die entsprechende freie Verbindung oder ein anderes Salz umwandelt, und/oder, wenn erwünscht, ein erhaltenes Gemisch von Isomeren oder Racematen in die einzelnen Isomere oder Racemate auftrennt, und/oder, wenn erwünscht, ein erhaltenes Racemat in die optischen Antipoden auftrennt.

Eine reaktive veresterte Hydroxygruppe W in einem der vorstehend beschriebenen Verfahren ist durch eine starke Säure verestertes Hydroxy, insbesondere durch eine starke anorganische Säure, wie durch eine Halogenwasserstoffsäure, insbesondere Chlorwasserstoffsäure, Bromwasserstoffsäure oder Iodwasserstoffsäure, ferner Schwefelsäure, oder durch eine starke organische Säure, insbesondere eine starke organische Sulfonsäure, wie eine aliphatische oder aromatische Sulfonsäure, z.B. Methansulfonsäure, 4-Methylphenylsulfonsäure oder 4-Bromphenylsulfonsäure. Entsprechendes reaktives verestertes Hydroxy W ist insbesondere Halogen, wie Chlor, Brom oder Iod, oder aliphatisch oder aromatisch substituiertes Sulfonyloxy, z.B. Phenylsulfonyloxy oder 4-Methylphenylsulfonyloxy (Tosyloxy).

In Ausgangsverbindungen und Zwischenprodukten dafür, die wie vorstehend beschrieben in erfindungsgemässe Verbindungen übergeführt werden, sind vorhandene funktionelle Gruppen wie Carbonyl- (Formyl oder Keto), Carboxy-, Amino-, Hydroxy- und Mercapto-Gruppen gegebenenfalls durch übliche Schutzgruppen geschützt, die in der präparativen organischen Chemie geläufig sind. Geschützte Carbonyl-, Carboxy-, Amino-, Hydroxy- und Mercapto-Gruppen sind solche, die unter schonenden Bedingungen in die entsprechenden freien Gruppen übergeführt werden können, ohne dass das Gerüst des Moleküls zerstört wird oder andere unerwünschte Nebenreaktionen stattfinden.

Der Grund, aus dem Schutzgruppen eingeführt werden, liegt darin, dass unerwünschte Reaktionen der funktionellen Gruppen mit den Reagentien und unter den jeweils herrschenden Reaktionsbedingungen verhindert werden sollen. Ob für eine bestimmte Reaktion Schutzgruppen notwendig sind, und welche, ist

dem Fachmann geläufig. Es hängt von der Art der zu schützerden funktionellen Gruppe ab sowie von der Struktur und Stabilität des Moleküls, das diesen Substituenten trägt und von den Reaktionsbedingungen.

Bekannte Schutzgruppen, die diesen Anforderungen genügen sowie ihre Einführung und Abspaltung sind beispielsweise in den folgenden Werken beschrieben: J.F.W. Mc Omie, "Protective Groups in Organic Chemistry", Plenum Press, London, New York 1973; T.W. Greene, "Protective Groups in Organic Synthesis", Wiley, New York 1981; "The Peptides", Vol. I, Schroeder und Lübke, Academic Press, London, New York 1965; Houben-Weyl, "Methoden der Organischen Chemie", Vol. 15/1, Georg Thieme Verlag, Stuttgart 1974.

Verfahren a) wird nach im Stand der Technik bekannten Methoden zur Reduktion von Doppelbindungen ausgeführt, z.B. für Verbindungen der Formel IV, in denen X Sauerstoff bedeutet, mit Wasserstoff unter Hydrierungsbedingungen, vorzugsweise in Gegenwart eines Katalysators wie Palladium auf Kohle oder, wenn die zu reduzierende Verbindung ein Enamin ist, mit einem Reduktionsmittel wie Natriumcyanborhydrid unter bekannten Bedingungen, bei Raumtemperatur oder erhöhter Temperatur in einem polaren Lösungsmittel wie Isopropanol.

Auch die Ausgangsverbindungen der Formeln IV-XIII lassen sich durch Anwendung generell bekannter Methoden herstellen.

So lässt sich z.B. eine Ausgangsverbindung der Formel IV, in der $R^1$ Carboxy, $C_{1-4}$-Alkoxycarbonyl, Carbamoyl, oder Mono- oder Di-$C_{1-4}$-alkylcarbamoyl bedeutet, durch Cyclisieren einer Verbindung der Formel XIV herstellen,

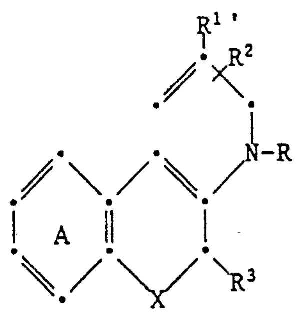

(XIV)

in der X, eventuelle Substituenten von Ring A, R, $R^2$ und $R^3$ wie vorstehend definiert sind und worin $R^{1'}$ Carboxy, $C_{1-4}$-Alkoxycarbonyl, Carbamoyl oder Mono- oder Di-$C_{1-4}$-alkylcarbamoyl bedeutet, beispielsweise durch Erhitzen in einem inerten Lösungsmittel wie Toluol. Man erhält auf diese Weise ein tricyclisches Produkt, ein 4a,10b-Dehydroderivat der Formel IV wie vorstehend definiert.

Ausgangsverbindungen der Formel XIV können in situ hergestellt werden, indem man geeignet substituierte 2H-[1]-Benzopyran-3-one mit geeignet substituierten α-(Aminomethyl)acrylsäure-Verbindungen kondensiert. 2H-[1]-Benzopyran-3-one (Chroman-3-one) sind bereits bekannt oder sie lassen sich auf bekannte Weise herstellen, z.B. nach J. Chem. Soc. 1610 (1948). Die Herstellung von Thiochroman-3-onen wird auf ähnliche Weise beschrieben, vergleiche z.B. J. Org. Chem. 34, 1566 (1969).

Auch Verbindungen der Formeln Ia und Ib sind Ausgangsmaterialien für Verfahren a).

Verfahren b) umfasst z.B. die Reduktion zum Alkohol und/oder einen Eliminierungsschritt zur Herstellung von Verbindungen der Formel Ia. Es lässt sich z.B. durch Hydrieren durchführen, wie mit einem Adams Katalysator in Essigsäure, oder durch Behandeln mit einem komplexen Metallhydrid-Reduktionsmittel, wie Lithiumaluminiumhydrid in Pyridin, Tetrahydrofuran oder Ether, oder Natriumborhydrid in Methanol.

Verbindungen der Formel V, in denen X und $Y^1$ Sauerstoff bedeuten und worin an der durch die gestrichelte Linie bezeichneten Position eine Doppelbindung vorliegt, können auch zuerst z.B. mit einem Metallhydrid wie Lithiumaluminiumhydrid in Pyridin selektiv zum entsprechenden 4a,10b-Dihydroderivat reduziert werden, dann in ein Thioketal übergeführt und, z.B. mit Raney-Nickel, entschwefelt werden.

Verbindungen der Formel I, insbesondere solche, in denen X Sauerstoff bedeutet, lassen sich auch aus Verbindungen der Formel V erhalten, in denen $Y^1$ als als Thioketal geschütztes Oxo vorliegt, z.B. durch Entschwefelung mit Raney-Nickel in Alkohol unter Erwärmen.

Ausgangsverbindungen der Formel V, in denen X und $Y^1$ Sauerstoff bedeuten und worin an der durch die gestrichelte Linie bezeichneten Position eine Doppelbindung vorliegt, lassen sich durch Kondensation eines gegebenenfalls substituierten 2H-[1]-Benzopyran-3-ons (Chroman-3-ons) mit beispielsweise einer gegebenenfalls substituierten 3-Aminopropionsäure, deren Carboxygruppe geschützt vorliegt, z.B. einem gegebenenfalls substituierten $C_{1-7}$-Alkylester von 3-Aminopropionsäure, z.B. in Gegenwart einer organischen Säure, wie Trifluoressigsäure, in einem inerten Lösungsmittel wie Toluol, herstellen.

Entsprechende Verbindungen der Formel V werden mit einem Reduktionsmittel wie einem komplexen Metallhydrid, z.B. Lithiumaluminiumhydrid, in Lösungsmitteln wie Pyridin, Diethylether und Tetrahydrofuran zum entsprechenden Alkohol der Formel V reduziert, in dem $Y^1$ Hydroxy zusammen mit Wasserstoff und $R^5$ Wasserstoff bedeuten. Wasserabspaltung auf an sich bekannte Weise ergibt eine entsprechende Verbindung der Formel Ia und/oder Ib, je nach angewendeten Reaktionsbedingungen. Beispielsweise liefert die Wassereliminierung mit Phosphoroxychlorid oder einer Mineralsäure bevorzugt Verbindungen der Formel Ia. Verbindungen der Formeln Ia oder Ib, in denen X Schwefel bedeutet, lassen sich ausgehend von entsprechenden Thiochroman-3-onen auf analoge Weise erhalten.

Verfahren c) bezieht sich in einer ersten Ausführungsform auf die Reduktion von Verbindungen der Formel VI, in denen $Y^4$ für Oxo oder geschütztes Oxo steht, insbesondere für Oxo, begleitet oder nicht begleitet von einer 4a,10b-Doppelbindung, und worin $Y^2$ und $Y^3$ die Bedeutung von zwei Resten aus der Gruppe bestehend aus einem Rest $R^1$, einem Rest $R^2$ und Wasserstoff aufweisen.

Dieses Verfahren wird vorzugsweise mit einem einfachen oder komplexen Metallhydrid als Reduktionsmittel ausgeführt, vorteilhafterweise in Gegenwart einer Lewissäure, z.B. mit Lithiumaluminiumhydrid oder Natriumborhydrid in Gegenwart von Bortrifluorid oder Aluminiumchlorid.

Entsprechende Ausgangsverbindungen der Formel VI, z.B. solche mit einer 4a,10b-Doppelbindung, lassen sich durch Kondensation eines gegebenenfalls substituierten Phenols (oder Thiophenols) mit einem $C_{1-7}$-Alkylester einer 3-Piperidon-2-carbonsäure in Gegenwart einer starken wasserfreien Säure, z.B. von Schwefelsäure, herstellen.

In einer zweiten Ausführungsform bezieht es sich auf die Reduktion von Verbindungen der Formel VI, in denen $Y^3$ für Oxo oder geschütztes Oxo steht, insbesondere für Oxo, begleitet oder nicht begleitet von einer 1,2-, 1,10b- oder einer 4a,10b-Doppelbindung, und worin $Y^2$ die Bedeutung von einem oder zwei Resten aus der Gruppe bestehend aus einem Rest $R^1$, einem Rest $R^2$ und Wasserstoff hat und $Y^4$ für $R^3$ und Wasserstoff steht. Ob $Y^2$ einen oder zwei monovalente Reste bedeutet, hängt davon ab, ob eine 1,2-Doppelbindung vorliegt.

Dieses Verfahren c) wird vorzugsweise durch Reduktion mit einem einfachen oder komplexen Hydrid-Reduktionsmittel durchgeführt, das für die Reduktion von Amidgruppen bekannt ist, z.B. Lithiumaluminiumhydrid oder Boran, in einem inerten Lösungsmittel, wie Tetrahydrofuran oder Diethylether, vorzugsweise bei Raumtemperatur oder erhöhter Temperatur. Wenn es sich bei einer Verbindung der Formel VI um ein Enamin handelt, kann die Kohlenstoff-Kohlenstoff-Doppelbindung gleichzeitig reduziert werden. Wenn andererseits eine Kohlenstoff-Kohlenstoff-Doppelbindung danach reduziert werden soll, kann das wie vorstehend unter Verfahren a) beschrieben geschehen.

Derartige Ausgangsverbindungen der Formel VI, z.B. solche, in denen $Y^3$ für Oxo steht, mit einer 4a,10b-Doppelbindung, können durch Kondensation eines Enaminderivates eines gegebenenfalls substituierten Chroman-3-ons oder Thiochroman-3-ons mit einem geeignet substituierten Acrylamid hergestellt werden, z.B. nach J. Med. Chem. 19, 987 (1976).

In einer dritten Ausführungsform bezieht sich Verfahren c) auf die Reduktion von Verbindungen der Formel VI, in denen $Y^2$ für Oxo oder geschütztes Oxo steht, im Falle von geschütztem Oxo insbesondere für als Thioketal geschütztes Oxo, begleitet oder nicht begleitet von einer 1,10b-Doppelbindung, und worin $Y^3$ die Bedeutung von zwei Resten aus der Gruppe bestehend aus einem Rest $R^1$, einem Rest $R^2$ und Wasserstoff aufweist und $Y^4$ für $R^3$ und Wasserstoff steht.

Dieses Verfahren wird wie für Verfahren b) beschrieben ausgeführt. Entsprechende Ausgangsverbindungen der Formel VI können durch Kondensation z.B. eines gegebenenfalls substituierten 3-Halogenchroman-4-ons (oder eines Thiochroman-4-ons) mit einem geeignet substituierten und (z.B. als Ketal) geschützten 1-Aminopropanon, anschliessende Freisetzung der Carbonylgruppe und Cyclisierung nach der allgemeinen Verfahrensweise, wie sie im J. Org. Chem. 44, 1108 (1979) beschrieben ist, erhalten werden. Diese Methode führt zu entsprechenden Verbindungen der Formel VI mit einer 1,10b-Doppelbindung.

Verfahren d) wird auf für die Reduktion von Pyridin- und Pyridiniumverbindungen an sich bekannte Weise durchgeführt, z.B. vorteilhafterweise durch katalytische Hydrierung zu Verbindungen der Formel I, oder auch durch Reduktion mit komplexen Metallhydriden, wie Natriumborhydrid oder Aluminiumhydrid zu Verbindungen der Formel Ia oder Ib.

Ausgangsverbindungen der Formel VII können durch Kondensation eines gegebenenfalls substituierten 2-Halogenphenols (oder -thiophenols) mit einem 3-Halogen-2-halogenmethylpyridin in basischem Medium, gefolgt von einer Reaktion von der Art der Ullmann-Reaktion in Gegenwart von Kupfer und abschliessende Quaternisierung mit beispielsweise einem $C_{1-4}$-Alkylhalogenid, hergestellt werden.

Verfahren e) umfasst einerseits die Reduktion einer Verbindung der Formel VIII, worin $R^6$ für $C_{2-4}$-Alkenyl oder für $C_{2-4}$-Alkinyl steht, zu einer Verbindung der Formel I, worin R für $C_{2-4}$-Alkyl steht.

Die Reduktion nach diesem Verfahren wird auf sich bekannte Weise durchgeführt, z.B. durch katalytische Hydrierung in Gegenwart eines Katalysators, wie Platin, Palladium oder Nickel in einem polaren Lösungsmittel bei atmosphärischem oder erhöhtem Druck, bei Raumtemperatur oder erhöhter Temperatur.

Die entsprechenden Ausgangsverbindungen der Formel VIII können z.B. hergestellt werden, indem man eine Verbindung der Formel I, II oder III, worin R Wasserstoff bedeutet, mit beispielsweise einem $C_{2-4}$-Alkinyl- oder -Alkenylhalogenid behandelt, wie Propargylbromid, Allylbromid, Allylchlorid, Crotylbromid und dergleichen.

Verfahren e) umfasst andererseits die Reduktion einer Verbindung der Formel VIII, worin $R^6$ für $C_{1-4}$-Alkanoyl oder Benzoyl steht. Diese Reduktion kann analog zu der für Verfahren c) beschriebenen durchgeführt werden, für den Fall, in dem $Y^3$ Oxo oder geschütztes Oxo bedeutet.

Verfahren f) wird in Anwesenheit einer starken Base wie Lithiumdiisopropylamid oder Lithiumamid in einem polaren Lösungsmittel wie Tetrahydrofuran durchgeführt, vorzugsweise bei Raumtemperatur oder unter Kühlen.

Ausgangsverbindungen der Formel IX können durch Kondensation eines gegebenenfalls substituierten o-(Butadienyl)-phenoxyacetaldehydes mit Ammoniak, einem $C_{1-4}$-Alkylamin oder einem Benzyl- oder Phenethylamin erhalten werden. Die dafür benötigte Verbindung lässt sich aus dem geeigneterweise geschützten o-Hydroxybenzaldehyd durch Wittig-Kondensation mit 3-Triphenylphosphoranyliden-1-propen, Abspalten der Schutzgruppe und nachfolgende Kondensation mit 2-Bromacetaldehyddiethylacetal herstellen.

Verfahren g) wird auf bekannte Weise ausgeführt, üblicherweise in Gegenwart eines Lösungsmittels oder einer Mischung von Lösungsmitteln und, wenn nötig, unter Kühlen oder Erwärmen, z.B. innerhalb eines Temperaturbereichs zwischen ungefähr -20° und 150°C, und/oder unter Schutzgas, z.B. in einer Stickstoffatmosphäre. Die Umsetzung wird vorteilhaft in Gegenwart einer Base ausgeführt, wie einer anorganischen Base, z.B. eines Alkalimetall- oder Erdalkalimetallcarbonats, -hydrids oder -hydroxides, oder einer organischen Base, wie eines Alkalimetall-$C_{1-7}$-alkoholats, oder eines tertiären Amins, wie Triethylamin oder Pyridin.

Ausgangsverbindungen der Formel X können durch Kondensation z.B. einer Grignard-Verbindung, die aus dem geeignet substituierten und geschützten 2-Halogenphenol oder 2-Halogenthiophenol gewonnen wird, mit der geeignet substituierten und geschützten 1,4,5,6-Tetrahydropyridin-2-carbonsäure erhalten werden. Man reduziert die als Primärprodukt erhaltene entsprechend substituierte und geschützte 3-(2-Hydroxy- oder 2-Mercaptophenyl)-piperidin-2-carbonsäure zum entsprechend substituierten 3-(2-Hydroxy- oder 2-Mercaptophenyl)-2-hydroxymethylpiperidin. Diese Verbindung wird dann in einen entsprechenden reaktiven Ester übergeführt, z.B. ein Chlor- oder Mesyloxyderivat.

Verfahren h) wird für den Fall, dass $Z^1$ oder $Z^2$ für Oxo steht, durch reduktive N-Alkylierung auf an sich bekannte Weise durchgeführt, z.B. durch katalytische Hydrierung mit Wasserstoff in Anwesenheit von Platin, Palladium oder Nickel als Katalysatoren, oder mit Reduktionsmitteln wie einfachen oder komplexen Leichtmetallhydriden, insbesondere einem Alkalimetallcyanborhydrid, wie Natriumcyanborhydrid. Die reduktive Aminierung mit einem Alkalimetallcyanborhydrid wird vorzugsweise in einem inerten Lösungsmittel, wie Methanol oder Acetonitril, insbesondere in Gegenwart einer Säure, wie Salzsäure oder Eisessig durchgeführt und wird vorteilhafterweise dann angewendet, wenn im Ausgangsmaterial eine Doppelbindung vorliegt.

Verfahren h) wird für den Fall, dass $Z^1$ oder $Z^2$ für reaktives verestertes Hydroxy W zusammen mit Wasserstoff oder $C_{1-4}$-Alkyl steht, mit oder ohne Base wie Triethylamin oder Kaliumcarbonat in einem inerten Lösungsmittel durchgeführt, wie für N-Alkylierungen im Stand der Technik geläufig.

Ausgangsverbindungen der Formel XI, z.B. solche, in denen $Z^2$ Oxo bedeutet, können durch Alkylierung eines Enaminderivats eines gegebenenfalls substituierten Chroman-3-ons oder Thiochroman-3-ons mit einem geeignet substituierten reaktiven 3-(verestertes Hydroxy)-propylamin-Derivat, z.B. einem gegebenenfalls substituierten 3-Halogenpropylamin, gewonnen werden. Diese Verbindungen können dann reduziert und in die entsprechenden Verbindungen der Formel XI umgewandelt werden, in denen $Z^2$ reaktives verestertes Hydroxy zusammen mit Wasserstoff bedeutet.

Alternativ kann ein gegebenenfalls substituiertes 4-(3-Aminopropyl)-2H-[1]-benzopyran oder -benzothiopyran mit Halogen, vorzugsweise mit Brom in einem inerten Lösungsmittel wie Essigester bei einer Temperatur zwischen ca. 0° und 100°C, sodann mit einer Base, vorteilhafterweise einem tertiären Amin wie Triethylamin oder Pyridin behandelt werden. Man erhält so eine Verbindung der Formel XI, die eine 1,10b-Doppelbindung aufweist und worin $Z^2$ Halogen zusammen mit Wasserstoff bedeutet, die, vorzugsweise in situ, in eine Verbindung der Formel I übergeführt wird.

Die Ausgangsverbindungen für diese Halogenierung lassen sich durch Cyclisieren beispielsweise eines N-Acyl-6-(gegebenenfalls substituiertes phenoxy)-4-hexinylamins, z.B. von 1-Phthalimido-6-(gegebenenfalls substituiertes phenoxy)-4-hexinylamin in Gegenwart einer organischen Base wie Diethylanilin in einem

polaren Lösungsmittel wie Dimethylformamid oder N-Methylpyrrolidon, vorzugsweise bei erhöhter Temperatur, insbesondere innerhalb eines Bereiches von 150-250° C, herstellen, indem man anschliessend die Aminoschutzgruppe abspaltet, z.B. mit Hydrazin, wenn die Aminogruppe als Phthalimidogruppe vorliegt. Man erhält auf diese Weise ein gegebenenfalls substituiertes 4-(3-Aminopropyl)-2H-[1]-benzopyran.

Das benötigte 4-Hexinylamin-Derivat lässt sich z.B. erhalten, indem man einen gegebenenfalls substituierten Phenylpropargylether mit einem reaktiven Derivat eines 1,3-Dihydroxypropans behandelt, z.B. mit 3-Brom-1-chlorpropan, in Gegenwart einer starken Base, wie Butyllithium, und nachfolgend z.B. ein Derivat von Ammoniak, wie Kaliumphthalimid zugibt. Verbindungen der Formel XI, in denen X Schwefel bedeutet, können auf analoge Weise ausgehend von einem entsprechenden N-Acyl-6-(gegebenenfalls substituiertes phenylthio)-4-hexinylamin hergestellt werden.

Zur Herstellung der Ausgangsverbindungen der Formel XI, worin $Z^1$ Oxo bedeutet, wird ein geeignet substituiertes Chroman-3-on oder Thiochroman-3-on zuerst unter basischen Bedingungen z.B. mit einem als Acetal geschützten 3-halogensubstituierten Propionaldehyd kondensiert. Das erhaltene Produkt wird unter Bedingungen reduktiver Aminierung mit Ammoniak, einem $C_{1-4}$-Alkylamin oder einem Benzyl- oder Phenethylamin umgesetzt, und durch Säurebehandlung wird die Schutzgruppe abgespalten.

Ausgangsverbindungen der Formel XI, worin $Z^1$ reaktives verestertes Hydroxy bedeutet, können hergestellt werden, indem man zuerst ein Enaminderivat eines gegebenenfalls substituierten Chroman-3-ons oder Thiochroman-3-ons z.B. mit einem reaktiven veresterten Derivat von 1,3-Dihydroxypropan, wie 1-Brom-3-chlorpropan, alkyliert, und das erhaltene substituierte Chroman-3-on oder Thiochroman-3-on reduktiv mit Ammoniak, einem $C_{1-4}$-Alkylamin oder einem Benzyl- oder Phenethylamin aminiert.

Verfahren i) wird vorzugsweise in Gegenwart einer Protonensäure, wie polyphosphoriger Säure, oder einer Lewissäure, wie Bortrifluorid oder Aluminiumchlorid, in Anwesenheit oder Abwesenheit eines geeigneten wasserfreien organischen Lösungsmittels nach für Friedel-Crafts-Alkylierungen bekannten Bedingungen durchgeführt. Bei Verwendung von Ausgangsverbindungen der Formel XII, worin $Y^5$ für Oxo steht, werden Verbindungen der Formel Ia erhalten. Bedeutet $Y^5$ hingegen reaktives verestertes Hydroxy W gemeinsam mit Wasserstoff oder $C_{1-4}$-Alkyl, werden Verbindungen der Formel I erhalten.

Ausgangsverbindungen der Formel XII lassen sich durch Kondensation eines gegebenenfalls substituierten Phenols oder Thiophenols mit beispielsweise einem gegebenenfalls substituierten reaktiven Ester von 2-Hydroxymethyl-3-oxopiperidin, wie dessen Chlor- oder Mesyloxyderivat, gewinnen.

Im Verfahren j) ist die α-Carbanionen stabilisierende Abgangsgruppe E in einem Ausgangsmaterial der Formel XIII beispielsweise eine Triarylphosphoranyliden- oder eine Di-$C_{1-7}$-Alkylphosphono-Gruppe, z.B. Triphenylphosphoranyliden oder Diethylphosphono. Diese Cyclisierung wird unter den bekannten allgemein üblichen Bedingungen einer Wittig-Reaktion in Anwesenheit einer starken Base, wie Natriumhydrid in einem Lösungsmittel wie Dichlormethan oder Toluol, bei einer Temperatur vorzugsweise zwischen -10° und +50° C durchgeführt.

Die Ausgangsverbindungen der Formel XIII können durch Behandeln eines gegebenenfalls substituierten 3-Aminochroman-4-ons oder 3-Aminothiochroman-4-ons mit z.B. einem 3-(Di-$C_{1-7}$-alkylphosphono)-propylhalogenid, wie 3-(Diethylphosphono)-propylbromid, hergestellt werden. Die gegebenenfalls substituierten 3-Aminochroman-4-one oder 3-Aminothiochroman-4-one werden auf an sich bekannte Weise hergestellt, z.B. durch Behandeln entsprechender 3-Halogenchroman-4-one oder 3-Halogenthiochroman-4-one mit z.B. Hexamethylentetramin und darauf mit Säure, oder mit einem Amin der Formel $RNH_2$ (R wie vorstehend definiert).

Verbindungen dieser Erfindung, die z.B. nach einer der vorstehend beschriebenen Methoden hergestellt worden sind, können auf an sich bekannte Weise und wie nachstehend noch näher erläutert, ineinander übergeführt werden.

Verbindungen der Formel Ia oder Ib lassen sich auf im Stand der Technik für die Sättigung von Doppelbindungen bekannte Weise in Verbindungen der Formel I überführen, z.B. durch katalytische Hydrierung, wie mit Wasserstoff in Gegenwart eines Hydrierungskatalysators, z.B. Palladium, in einem polaren Lösungsmittel, wie Ethanol, bei atmosphärischem oder erhöhtem Druck. Die Sättigung einer derartigen Kohlenstoff-Kohlenstoff-Doppelbindung kann auch durch Metallreduktion mit einem Alkalimetall bewirkt werden, wie z.B. Natrium in einem polaren Lösungsmittel, wie Ammoniak oder Tetrahydrofuran, unter im Stand der Technik bekannten Bedingungen oder wie in den Beispielen illustriert.

Verbindungen der Formel Ia, insbesondere solche, in denen R für $C_{1-4}$-Alkyl steht, können durch Behandeln mit einer starken Base, z.B. mit Butyllithium, danach mit einem reaktiven Ester eines $C_{1-4}$-Alkanols, z.B. einem $C_{1-4}$-Alkylhalogenid, in Verbindungen der Formel Ib übergeführt werden, in denen $R^5$ für $C_{1-4}$-Alkyl steht.

Die Behandlung einer Verbindung der Formel Ia mit einer starken Base wie Butyllithium, danach mit einem reaktiven Derivat der Kohlensäure, z.B. mit einem Diester wie einem Kohlensäuredi-$C_{1-4}$-alkylester,

wie Kohlensäurediethylester, mit einem Halogenkohlensäureester, wie Chlorkohlensäureethylester, einem Alkalimetallcyanat oder einem $C_{1-4}$-Alkylisocyanat und, wenn erwünscht, sodann Hydrolyse oder N-Alkylierung des erhaltenen Produkts, ergibt Verbindungen der Formel Ia, in denen $R^1$ an das C-2-Kohlenstoffatom gebunden ist und beispielsweise für Carboxy, $C_{1-4}$-Alkoxycarbonyl, Carbamoyl oder Mono- oder Di-$C_{1-4}$-Alkylcarbamoyl steht. Diese Verbindungen können auch nach Verfahren, die hierin beschrieben sind, in die entsprechenden Verbindungen der Formel I umgewandelt werden.

Verbindungen der Formeln I, Ia oder Ib, in denen R Wasserstoff bedeutet, können durch Umsetzung mit einem reaktiven veresterten $C_{1-4}$-Alkanol, Benzylalkohol oder Phenylethanol, z.B. einem Halogenid, in Verbindungen der Formeln I, Ia oder Ib umgewandelt werden, in denen R $C_{1-4}$-Alkyl, Benzyl oder Phenethyl bedeutet. Dabei werden die Verbindungen der Formeln I, Ia oder Ib vorzugsweise als Säureadditionssalze isoliert. Diese Umwandlung lässt sich auch auf dem Weg reduktiver Alkylierung erzielen, z.B. mit Formaldehyd und Ameisensäure, wobei man eine entsprechende Verbindung enthält, in der R Methyl bedeutet, oder mit einem $C_{1-4}$-Alkylcarboxaldehyd, Benzaldehyd oder Phenylacetaldehyd in Gegenwart eines Reduktionsmittels, wie Natriumcyanborhydrid.

Verbindungen der Formeln I, Ia oder Ib, in denen R für $C_{1-4}$-Alkyl, insbesondere für Methyl steht, können durch katalytische Luftoxidation in Verbindungen der Formeln I, Ia oder Ib umgewandelt werden, in denen R für Wasserstoff steht, z.B. mit Palladium auf Kohle, wobei man einen Alkohol, wie Methanol, als Lösungsmittel verwendet, vorzugsweise bei Raumtemperatur. Man kann entsprechende Verbindungen auch mit Halogenkohlensäure-$C_{1-7}$-alkylestern, z.B. mit Chlorkohlensäureethylester, umsetzen, erhält N-Acylverbindungen, die sich ihrerseits zu den unsubstituierten Verbindungen, in denen R für Wasserstoff steht, hydrolysieren lassen, z.B. mit einer Base, wie einem Alkalimetallhydroxid, z.B. einer wässrigen oder wässrig-alkoholischen Lösung von Natriumhydroxid.

Verbindungen der Formel I, in denen R Methyl bedeutet, können durch Umsetzen der entsprechenden Verbindungen der Formel I, in denen R Wasserstoff bedeutet, mit einem Halogenkohlensäure-$C_{1-7}$-alkyl- oder -phenyl-$C_{1-7}$-alkylester, wie Chlorkohlensäureethylester, erhalten werden. Man gewinn so zuerst Verbindungen der Formel I, in denen R für $C_{1-7}$-Alkoxycarbonyl oder Phenyl-$C_{1-7}$-alkoxycarbonyl steht, und reduziert diese Acylderivate mit einfachen oder komplexen Leichtmetallhydriden wie Lithiumaluminiumhydrid, Natrium-tri-tert.-butoxy- oder Bis-(methoxyethoxy)-aluminiumhydrid.

Verbindungen der Formeln I, Ia oder Ib, in denen Ring A beispielsweise durch Acyloxy substituiert ist, wie durch $C_{1-4}$-Alkanoyloxy oder Aroyloxy, können durch Hydrolyse mit z.B. wässriger Säure, wie Salzsäure, oder mit wässrigem Alkali, wie Lithium- oder Natriumhydroxid, in Verbindungen der Formel I, Ia oder Ib übergeführt werden, in denen Ring A durch Hydroxy substituiert ist.

Umgekehrt können Verbindungen der Formel I, Ia oder Ib, in denen z.B. der Ring A durch Hydroxy substituiert ist, in Verbindungen der Formel I, Ia oder Ib, in denen Ring A durch Acyloxy wie $C_{1-4}$-Alkanoyloxy oder Aroyloxy substituiert ist, umgewandelt werden, indem man erstere mit einer entsprechenden Carbonsäure oder mit einem reaktiven Derivat davon auf für Acylierungen (Veresterungen) an sich bekannte Weise kondensiert.

Die Umwandlung von Verbindungen der Formeln I, Ia und Ib, in denen Ring A durch verethertes Hydroxy, wie $C_{1-4}$-Alkoxy, substituiert ist, in Verbindungen der Formel I, Ia oder Ib, in denen Ring A durch Hydroxy substituiert ist, wird auf an sich bekannte Weise durchgeführt, z.B. mit einer Mineralsäure, wie Iodwasserstoffsäure, oder, vorzugsweise für Verbindungen, in denen $C_{1-4}$-Alkoxy für Methoxy steht, mit Bortribromid in Dichlormethan oder mit Natrium-oder Lithiumdiphenylphosphid in Tetrahydrofuran.

Die Umwandlung von Verbindungen der Formel I, Ia oder Ib, in denen Ring A durch Benzyloxy substituiert ist, in Verbindungen der Formel I, Ia oder Ib, in denen Ring A durch Hydroxy substituiert ist, wird vorzugsweise durch Hydrogenolyse unter Verwendung von Wasserstoff in Gegenwart eines Katalysators, z.B. Palladium, durchgeführt.

Verbindungen der Formel I, Ia oder Ib, in denen R für Benzyl steht, können z.B. mit Wasserstoff in Gegenwart eines Hydrogenolyse-Katalysators, wie Palladium auf Kohle, zu den entsprechenden Verbindungen, in denen R für Wasserstoff steht, umgesetzt werden.

Ungesättigte Verbindungen, die einen Alkenyl- oder Alkinylrest tragen, können ebenfalls mit katalytisch aktiviertem Wasserstoff reduziert werden. Man erhält so Verbindungen der Formel I oder Zwischenprodukte, die den entsprechenden Alkylrest tragen.

Im Zusammenhang mit den vorstehend erwähnten Reaktionen kann es, wie schon erwähnt, vorteilhaft sein, die möglicherweise reaktiven Substituenten, wie Amino, Carboxy oder Hydroxy, oder andere störende Substituenten, in geeigneter Weise zu schützen. Man bedient sich dabei an sich bekannter Schutzgruppen-Techniken, wie z.B. nachstehend illustriert, um Nebenreaktionen zu vermeiden, indem man solche Substituenten vor der gewünschten Reaktion schützt und danach, wenn nötig, die Schutzgruppen wieder abspaltet, um die gewünschten Verbindungen, z.B. solche der Formel I oder auch Zwischenprodukte, zu erhalten.

So kann eine freie Aminogruppe, die zumindest ein Wasserstoffatom am Stickstoff trägt, in Form eines leicht spaltbaren Amides geschützt werden, z.B. als Acylderivat, wie als Benzyloxycarbonyl- oder als tert.-Butoxycarbonyl-Verbindung, oder durch jede andere leicht abspaltbare N-Schutzgruppe.

Eine Carboxygruppe lässt sich in Form eines leicht spaltbaren Esters schützen, z.B. als Benzyl- oder tert.-Butylester, oder als einer, der sonst üblicherweise verwendet wird.

Eine Hydroxygruppe kann als Ester geschützt vorliegen, z.B. als Acylderivat, wie als $C_{1-7}$-Alkanoyl-, Benzyloxycarbonyl- oder $C_{1-7}$-Alkoxycarbonylester. Ferner kann eine solche Hydroxygruppe als Ether geschützt werden, z.B. als 2-Tetrahydropyranyl- oder als Benzylether.

In einer erhaltenen Verbindung der Formel I oder einem Zwischenprodukt, in dem eine oder mehrere funktionelle Gruppen geschützt sind, werden solche geschützten funktionellen Gruppen, z.B. Amino-, Hydroxy- oder Carboxygruppen, auf an sich bekannte Weise freigesetzt, z.B. durch Solvolyse, insbesondere saure Hydrolyse, oder durch Reduktion, insbesondere Hydrogenolyse.

Die vorstehend erwähnten Umsetzungen werden nach Standardverfahren durchgeführt, in Gegenwart von oder ohne Verdünnungsmittel, vorzugsweise solchen, die gegenüber den Reagentien inert sind und sie zu lösen vermögen, von Katalysatoren, Kondensationsmitteln oder anderen Stoffen und/oder unter Schutzgasatmosphäre, bei niedrigen Temperaturen, Raumtemperatur oder erhöhter Temperatur, vorzugsweise im Bereich des Siedepunktes der verwendeten Lösungsmittel, bei atmosphärischem oder erhöhtem Druck.

Die Erfindung umfasst ferner jede Variante der erwähnten Verfahren, nach der ein Zwischenprodukt, das sich auf irgendeiner Stufe isolieren lässt, als Ausgangsstoff verwendet wird, um damit die weiteren Reaktionsschritte durchzuführen, oder nach der Ausgangsstoffe unter den Reaktionsbedingungen gebildet werden oder nach denen Ausgangsstoffe in Form ihrer Salze verwendet werden oder als optisch reine Antipoden. Immer wenn es wünschenswert ist, werden vor Ausführung der beschriebenen Verfahren möglicherweise störende reaktive funktionelle Gruppen auf geeignete Weise geschützt, wie vorstehend und in den Beispielen illustriert.

Vorzugsweise werden in diesen Umsetzungen solche Ausgangsstoffe verwendet, die zur Bildung der vorstehend als bevorzugt bezeichneten Verbindungen führen.

Die Erfindung bezieht sich auch auf neue Ausgangsstoffe und Verfahren zu deren Herstellung.

Je nach Wahl der Ausgangsstoffe und Verfahren lassen sich die neuen Verbindungen in Form eines der möglichen Isomere oder als Isomerengemisch gewinnen, z.B. als reine geometrische Isomere (cis oder trans), als reine optische Isomere (als Antipoden), oder als Mischungen optischer Isomere, wie als Racemate, oder als Mischungen geometrischer Isomere.

Verbindungen der Formel I, II oder III mit einer trans-4a,10b-Ring-Verknüpfung können aus Verbindungen der Formel I, II oder III mit einer cis-4a,10b-Ring-Verknüpfung hergestellt werden, indem man sie mit einer starken Base, wie Kalium-tert.-butylat, in einem nicht wässrigen Lösungsmittel, wie Dimethylsulfoxid, behandelt, und, wenn erwünscht, die trans-verknüpfte Verbindung aus einer Mischung der Isomere, z.B. durch Chromatographie oder durch Kristallisation, abtrennt.

Erhaltene Gemische von geometrischen Isomeren oder von Diastereomeren von Verbindungen der Formel I, Ia oder Ib oder von Zwischenprodukten dafür können auf an sich bekannte Weise in die einzelnen racemischen oder optisch aktiven Isomere aufgetrennt werden, z.B. durch fraktionierte Destillation oder Kristallisation und/oder durch Chromatographie.

Racemische Produkte der Formel I, Ia oder Ib sowie basische Zwischenprodukte können z.B. durch Auftrennung von ihren diastereomeren Salzen in die optischen Antipoden aufgetrennt werden, z.B. durch fraktioniertes Kristallisieren ihrer d- oder l-(Tartrate, Dibenzoyltartrate, Mandelate oder Camphersulfonate).

Jedes saure Zwischenprodukt, das eine zur Bildung von Salzen befähigte saure Gruppe aufweist, kann z.B. durch Trennung der mit d- oder oder l-(α-Methylbenzylamin, Cinchonidin, Cinchonin, Chinin, Chinidin, Ephedrin, Dehydroabietylamin, Brucin oder Strychnin) gebildeten Salze in reiner Form isoliert werden.

Vorzugsweise wird das aktivere Isomer, wie ein Antipode, von den Verbindungen dieser Erfindung isoliert.

Die Verbindungen dieser Erfindung werden entweder als freie Verbindungen oder als Salze erhalten. Jede erhaltene Base kann in ein entsprechendes Säureadditionssalz umgewandelt werden, vorzugsweise unter Verwendung einer pharmazeutisch annehmbaren Säure oder eines Anion-Austauschpräparates. Ferner können erhaltene Salze in die entsprechenden freien Basen übergeführt werden, z.B. unter Verwendung einer stärkeren Base, wie eines Metall- oder Ammoniumhydroxids oder irgendeines basischen Salzes, z.B. eines Alkalimetallhydroxids oder -carbonats, oder eines Kation-Austauschpräparates. Diese oder andere Salze, beispielsweise Pikrate, können auch zur Reinigung der erhaltenen Basen dienen, die Basen werden dafür in die Salze übergeführt. Wegen der engen Beziehungen zwischen den freien Verbindungen und ihren Salzen ist im Rahmen dieser Erfindung im Zusammenhang mit der Erwähnung einer Verbindung immer auch ein ihr entsprechendes Salz mit gemeint und umfasst, soweit das nach den Umständen möglich und

sinnvoll ist.

Die Verbindungen, wie auch ihre Salze, können auch in Form ihrer Hydrate erhalten werden oder andere Lösungsmittel enthalten, die für ihre Kristallisation verwendet wurden.

Die vorliegende Erfindung bezieht sich zusätzlich auf die Verwendung der Verbindungen der Formel I, Ia oder Ib, von deren pharmazeutisch annehmbaren nicht toxischen Säureadditionssalzen oder von pharmazeutischen Präparaten davon, als Medikamente in Säugern, insbesondere als psychotrope Mittel zur Behandlung von Krankheiten des zentralen Nervensystems, die auf Modulierung von psychotropen Rezeptoren des zentralen Nervensystems ansprechen, wie auf Stimulierung von präsynaptischen Dopaminrezeptoren oder von Serotoninrezeptoren. Sie lassen sich z.B. als neuroleptische (antipsychotische) Mittel zur Behandlung von psychotischen Zuständen oder als antidepressive Mittel zur Behandlung von Depression einsetzen.

Die vorliegende Erfindung erstreckt sich auch auf die Verwendung der Verbindungen dieser Erfindung zur Herstellung von pharmazeutischen Präparaten, insbesondere solchen pharmazeutischen Präparaten, die eine psychotrope Rezeptoren modulierende Wirkung aufweisen, z.B. eine präsynaptische Dopaminrezeptoren oder Seretoninrezeptoren modulierende, wie insbesondere stimulierende Wirksamkeit.

Die pharmazeutischen Präparate dieser Erfindung sind zur enteralen wie oralen oder rektalen, transdermalen oder parenteralen Verabreichung an Säuger, Menschen eingeschlossen, geeignet. Sie dienen zur Behandlung von Krankheiten, die auf Stimulierung präsynaptischer Dopaminrezeptoren ansprechen, wie von psychotischen Krankheiten, oder auf Stimulierung von Serotoninrezeptoren, wie von Depression. Es bedarf dazu einer wirksamen Menge einer pharmakologisch aktiven Verbindung der Formel I, Ia oder Ib oder eines pharmazeutisch annehmbaren Salzes davon, allein oder in Kombination mit einem oder mehreren pharmazeutisch annehmbaren Hilfsstoffen.

Die pharmakologisch aktiven Verbindungen dieser Erfindung lassen sich zur Herstellung von pharmazeutischen Präparaten verwenden, die eine wirksame Menge davon zusammen mit Hilfs- oder Trägerstoffen enthalten, die zur enteralen oder parenteralen Verabreichung geeignet sind. Bevorzugt sind Tabletten und Gelatinekapseln, die die aktive Komponente gemeinsam mit a) Verdünnungsmitteln, z.B. Laktose, Dextrose, Sucrose, Mannit, Sorbit, Cellulose und oder Glycin, b) Gleitmitteln, z.B. Silica, Talk, Stearinsäure, deren Magnesium- oder Calciumsalze und/oder Polyethylenglykol, für Tabletten auch c) Bindemitteln, z.B. Magnesiumaluminiumsilikat, Stärke, Gelatine, Traganth, Methylcellulose, Natriumcarboxymethylcellulose und/oder Polyvinylpyrrolidon, wenn erwünscht d) Sprengmitteln, z.B. Stärken, Agar, Alginsäure oder Natriumalginat, oder schäumende Mischungen, und/oder e) Absorbentien, Farbstoffen, Geschmacks- und Süssstoffen enthalten. Injizierbare Präparate sind vorzugsweise wässrige isotonische Lösungen oder Suspensionen; Suppositorien werden vorteilhaft aus Fettemulsionen oder Suspensionen hergestellt. Diese Präparate können sterilisiert sein und/oder Zusatzstoffe enthalten, wie Konservierungs-, Stabilisierungs-, Netz- oder Emulgiermittel, Lösungsvermittler, Salze zur Regulierung des osmotischen Drucks und/oder Puffer. Zusätzlich können die Präparate auch andere therapeutisch wertvolle Substanzen enthalten. Besagte Präparate werden mittels konventioneller Misch-, Granulier- und Ueberzugsverfahren hergestellt. Sie enthalten etwa 0,1 bis 75 %, vorzugsweise etwa 1 bis 50 % des aktiven Bestandteils.

Geeignete Formen zur transdermalen Verabreichung umfassen eine wirksame Menge einer Verbindung der Formel I, Ia oder Ib und einen Träger. Vorteilhafte Träger umfassen absorbierbare pharmakologisch annehmbare Lösungsmittel, die den Transport durch die Haut des Behandelten unterstützen sollen. Charakteristischerweise bestehen transdermale Anwendungsformen aus einer Bandage, die eine Rückfront enthält, ein Reservoir, in dem sich der Wirkstoff befindet, gegebenenfalls mit Trägerstoffen, gegebenenfalls eine die Abgabegeschwindigkeit regulierende Barriere, damit der Wirkstoff an die Haut des Behandelten während einer längeren Zeit in kontrollierter und vorherbestimmbarer Weise abgegeben wird, sowie ein Mittel, um die Anwendungsform auf der Haut sicher zu halten.

Insbesondere bezieht sich die Erfindung auch auf ein Verfahren zur Behandlung von psychotropen Krankheiten in Säugern, die z.B. auf Stimulierung präsynaptischer Dopaminrezeptoren oder auf Stimulierung von Serotoninrezeptoren ansprechen, wozu man eine wirksame Menge einer Verbindung dieser Erfindung, z.B. der Formel I, Ia oder Ib, oder von pharmazeutisch annehmbaren Salzen davon als pharmakologisch aktive Substanzen verwendet, vorzugsweise in Form einer der vorstehend beschriebenen pharmazeutischen Zusammensetzungen. Die Dosis der verabreichten aktiven Substanz hängt ab von der Art des Warmblüters (Säugers), dessen Körpergewicht, Alter und individueller Verfassung, sowie von der Art der Verabreichung.

Eine Einheitsdosis für einen Säuger von 50 bis 70 kg kann zwischen etwa 10 und 100 mg aktiven Bestandteil enthalten.

Die folgenden Beispiele sollen die Erfindung illustrieren und sind nicht geeignet, die Erfindung zu beschränken, etwa auf den Umfang der Beispiele. Temperaturen werden in Celsiusgraden angegeben. Alle Verdampfungen werden, wenn nicht ausdrücklich anderweitig beschrieben, unter vermindertem Druck,

vorzugsweise zwischen 2 und 13 kPa, vorgenommen.

Beispiel 1:

Zu einer Lösung von 9,0 g 9-Methoxy-4-propyl-1,2,3,5-tetrahydro-4H-[1]-benzopyrano[3,4-b]pyridin-1-on in 90 ml trockenem Pyridin werden bei 0° 0,90 g Lithiumaluminiumhydrid gegeben. Nach 20 Minuten bei 30° wird der Ansatz mit 1,8 ml 10 %iger Natronlauge versetzt, die Mischung wird mit Essigester verdünnt, über Magnesiumsulfat getrocknet und filtriert. Der Filterkuchen wird mit 10 %igem Methanol/Dichlormethan gewaschen, und die Lösungsmittel der vereinigten Filtrate werden im Vakuum abgezogen. Die vorstehende Reduktion wird mit dem Rückstand wiederholt, wobei man anstelle von Pyridin Tetrahydrofuran als Lösungsmittel verwendet. Das Produkt wird mit Ether behandelt. Man erhält ein weisses Pulver, das mit 28 ml Pyridin und 6,3 ml Phosphoroxychlorid 30 Minuten bei 65° gehalten wird. Der Ansatz wird auf eine Mischung aus Eis und Natriumcarbonatlösung gegeben, und das Produkt wird mit Essigester extrahiert. Nach Trocknen über Magnesiumsulfat wird das Lösungsmittel im Vakuum abgezogen. Man erhält als Oel 9-Methoxy-4-propyl-2,3,4a,5-tetrahydro-4H-[1]-benzopyrano[3,4-b]pyridin; NMR: $\delta$ 0.90 (3H,t), 3.75 (3H,s).

Das Ausgangsmaterial wird wie folgt hergestellt: Zu einer gut gerührten Lösung von 60 g 6-Methoxy-2H-[1]-benzopyran [J. Org. Chem. 39, 881 (1974)] in 300 ml Aceton und 150 ml Wasser werden 70 g N-Bromsuccinimid während 5 Minuten portionsweise zugegeben. Nach 10 Minuten bei Raumtemperatur wird der Ansatz mit Wasser verdünnt und das Produkt mit Ether extrahiert. Nachdem die vereinigten etherischen Phasen mit Wasser gewaschen sind, werden sie über Magnesiumsulfat getrocknet, und das Lösungsmittel wird im Vakuum abgezogen. Der kristalline Rückstand wird mit Ether/Hexan behandelt. Man erhält trans-3-Brom-4-hydroxy-6-methoxy-3,4-dihydro-2H-[1]-benzopyran, Fp. 98-99°.

Zu einer Suspension von 2,0 g Natriumhydrid in 100 ml trockenem Tetrahydrofuran wird unter Rühren tropfenweise eine Lösung von 20 g trans-3-Brom-4-hydroxy-6-methoxy-3,4-dihydro-2H-[1]-benzopyran in 200 ml trockenem Tetrahydrofuran gegeben. Nach 30 Minuten Rühren bei Raumtemperatur wird der Ansatz durch Filtercel filtriert und das Lösungsmittel im Vakuum abgezogen. Der Rückstand wird in 100 ml Toluol aufgenommen, 1,0 g wasserfreies Zinkiodid wird zugegeben, und die Mischung wird 1 Stunde auf 80° erhitzt. Der Ansatz wird unter Verwendung von Dichlormethan über 120 g Silicagel filtriert. Das erhaltene Produkt wird aus Ether umkristallisiert. Man erhält 6-Methoxy-2H-[1]-benzopyran-3-on, Fp. 67-72°.

Eine Mischung von 8,9 g 6-Methoxy-2H-[1]-benzopyran-3-on, 7,46 g 3-(Propylamino)-propionsäuremethylester und 0,8 ml Trifluoressigsäure in 80 ml Toluol wird 7 Stunden in einem Dean Stark Apparat unter Rückfluss erhitzt. Weitere Portionen von je 0,8 ml Trifluoressigsäure werden nach 2, 4 und 6 Stunden zugegeben. Der Ansatz wird gekühlt und das Lösungsmittel unter Vakuum abgezogen. Der Rückstand wird aus Ether/Methanol kristallisiert. Man erhält 9-Methoxy-4-propyl-1,2,3,5-tetrahydro-4H-[1]-benzopyrano[3,4-b]pyridin-1-on, Fp. 97-100°.

Beispiel 2:

a) Zu einer Lösung von 6,8 g 9-Methoxy-4-propyl-2,3,4a,5-tetrahydro-4H-[1]-benzopyrano[3,4-b]pyridin in 50 ml Tetrahydrofuran und 200 ml flüssigem Ammoniak werden 0,45 g Wasser gegeben, danach 2,8 g Natrium bei -70°. Die Mischung wird 5 Minuten bei -33° gerührt, nach dieser Zeit lässt sich eine permanente blaue Farbe beobachten. Die Mischung wird mit überschüssigem Ammoniumchlorid versetzt, und man lässt das Ammoniak abdampfen. Nach Verdünnung mit Wasser werden die Produkte mit Ether extrahiert, die organische Phase wird getrocknet und das Lösungsmittel im Vakuum abgezogen. Das Rohprodukt wird in Ethanol gelöst und mit über-schüssigem ethanolischem Chlorwasserstoff behandelt. Es wird abgekühlt, um das Salz auszukristallisieren. Man erhält das trans-9-Methoxy-4-propyl-1,2,3,4a,5,10b-hexahydro-4H-[1]-benzopyrano[3,4-b]-pyridinhydrochlorid, Fp. 252-254°.

b) Die Mutterlauge der obigen Kristallisation wird zur Trockne eingeengt. Man erhält das cis-9-Methoxy-4-propyl-1,2,3,4a,5,10b-hexahydro-4H-[1]-benzopyrano[3,4-b]pyridinhydrochlorid, Fp. 200-202°.

Beispiel 3:

Zu einer Lösung von 5,0 g 9-Methoxy-4-propyl-2,3,4a,5-tetrahydro-4H-[1]-benzopyrano[3,4-b]pyridin in 150 ml trockenem Tetrahydrofuran werden 8,8 ml 2,2M Butyllithium in Hexan bei -78° gegeben. Die resultierende orange Lösung wird 30 Minuten bei 0° gerührt. Nach erneutem Abkühlen auf -78° werden 3,0 g Methyliodid zugegeben, und erneut wird die Reaktionslösung 30 Minuten bei 0° gerührt. Der Ansatz wird auf Wasser gegeben, die Produkte werden mit Ether extrahiert, die organische Schicht wird getrocknet und die Lösungsmittel im Vakuum abgezogen. Chromatographie an 400 g Silicagel (Ether/Hexan, 1:4) ergibt

nacheinander:

a) cis-9-Methoxy-2-methyl-4-propyl-2,3,4a,5-tetrahydro-4H-[1]-benzopyrano[3,4-b]pyridin,

b) cis und trans-9-Methoxy-10b-methyl-4-propyl-3,4a,5,10b-tetrahydro-4H-[1]-benzopyrano[3,4-b]pyridin und

c) trans-9-Methoxy-2-methyl-4-propyl-2,3,4a,5-tetrahydro-4H-[1]-benzopyrano[3,4-b]pyridin (trans bezüglich der 2 und 4a Positionen).

Auf analoge Weise werden hergestellt:

d) cis und trans-7-Methoxy-4,10b-dimethyl-3,4a,5,10b-tetrahydro-4H-[1]-benzopyrano[3,4-b]pyridin,

e) cis und trans-7-Methoxy-2,4-dimethyl-2,3,4a,5-tetrahydro-4H-[1]-benzopyrano[3,4-b]pyridin.

Beispiel 4:

a) Zu einer Lösung von 4,5 g trans-9-Methoxy-4-propyl-1,2,3,4a,5,10b-hexahydro-4H-[1]-benzopyrano-[3,4-b]pyridin in 30 ml heissem Isopropanol werden 6,48 g (-)-Dibenzoylweinsäuremonohydrat gegeben. Nach Kühlen scheidet sich ein weisses kristallines Dibenzoylweinsäuresalz ab, das dreimal aus Ethanol bis zum konstanten Schmelzpunkt von 174-175° umkristallisiert wird. Das Salz wird in die freie Base übergeführt, indem es zwischen Ether und verdünnte Natriumbicarbonatlösung verteilt wird, der Etherextrakt wird über Magnesiumsulfat getrocknet und das Lösungsmittel entfernt. Man erhält (+)-trans-9-Methoxy-4-propyl-1,2,3,4a,5,10b-hexahydro-4-[1]-benzopyrano[3,4-b]pyridin, als Oel, $[\alpha]_D^{25}$ + 71,97° (c = 1,0, 0,1 N Salzsäure).

b) Die vereinigten Mutterlaugen der Kristallisationen aus Ethanol unter a) werden zur Trockne eingeengt und die zurückbleibenden Salze werden in die freie Base wie unter a) beschrieben übergeführt. Behandlung mit einem Aequivalent (+)-Dibenzoylweinsäuremonohydrat in Isopropanol gibt ein Salz, das dreimal aus Ethanol umkristallisiert wird, man erhält das optisch aktive Salz mit einem Schmelzpunkt von 176-178°. Ueberführung in die freie Base wie unter a) beschrieben ergibt (-)-trans-9-Methoxy-4-propyl-1,2,3,4a,5,10b-hexahydro-4H-[1]-benzopyrano[3,4-b]pyridin, als Oel, $[\alpha]_D^{25}$ -70,26° (c = 1,0, 0,1 N Salzsäure).

Beispiel 5:

a) Aus 4,27 g Diphenylphosphin und 8,9 ml 2,2 M Butyllithium in Hexan in 30 ml trockenem Tetrahydrofuran wird eine Lösung von Lithiumdiphenylphosphid hergestellt. Zu dieser Lösung werden 3,0 g trans-9-Methoxy-4-propyl-1,2,3,4a,5,10b-hexahydro-4H-[1]-benzopyrano[3,4-b]-pyridin gegeben, und die Mischung wird 5 Stunden unter Rückfluss erhitzt. Der Ansatz wird mit Ether verdünnt, und das Produkt wird mit 2N Salzsäure extrahiert. Nach Neutralisierung der wässrigen Phase mit Natriumcarbonat wird das Produkt mit Essigester extrahiert, die organische Phase über Magnesiumsulfat getrocknet und das Lösungsmittel im Vakuum abgezogen. Der Rückstand wird in heissem Ethanol gelöst, ein Ueberschuss einer 5,4 M Lösung von Chlorwasserstoff in Ethanol wird zugegeben. Kühlen führt zur Kristallisation von trans-9-Hydroxy-4-propyl-1,2,3,4a,5,10b-hexahydro-4H-[1]-benzopyrano[3,4-b]-pyridinhydrochlorid, Fp. 292-294°.

b) Auf analoge Weise wird aus (+)-trans-9-Methoxy-4-propyl-1,2,3,4a,5,10b-hexahydro-4H-[1]-benzopyrano[3,4-b]pyridin das (+)-trans-9-Hydroxy-4-propyl-1,2,3,4a,5,10b-hexahydro-4H-[1]-benzopyrano[3,4-b]pyridinhydrochlorid, $[\alpha]_D^{25}$ +81.79° (c = 1,0 Wasser), Fp. 292° (Z) hergestellt.

c) Auf analoge Weise wird aus (-)-trans-9-Methoxy-4-propyl-1,2,3,4a,5,10b-hexahydro-4H-[1]-benzopyrano[3,4-b]pyridin das (-)-trans-9-Hydroxy-4-propyl-1,2,3,4a,5,10b-hexahydro-4H-[1]-benzopyrano[3,4-b]pyridinhydrochlorid, $[\alpha]_D^{25}$ -80,35° (c = 1,0 Wasser), Fp. 292° (Z) hergestellt.

d) Auf analoge Weise wird aus 9-Methoxy-4-propyl-2,3,4a,5-tetrahydro-4H-[1]-benzopyrano[3,4-b]pyridin das 9-Hydroxy-4-propyl-2,3,4a,5-tetrahydro-4H-[1]-benzopyrano[3,4-b]pyridinhydrochlorid, Fp. 265-266° hergestellt.

e) Auf analoge Weise ergeben cis und trans-9-Methoxy-2-methyl-4-propyl-2,3,4a,5-tetrahydro-4H-[1]-benzopyrano[3,4-b]pyridin das cis und trans-9-Hydroxy-2-methyl-4-propyl-2,3,4a,5-tetrahydro-4H-[1]-benzopyrano[3,4-b]pyridinhydrochlorid, Fp. 248-250° und Fp. 237-240°.

Beispiel 6:

a) Eine Lösung von 1,5 g einer Mischung von cis und trans-9-Methoxy-10b-methyl-4-propyl-3,4a,5,10b-tetrahydro-4H-[1]-benzopyrano[3,4-b]pyridin (Beispiel 3b) in 30 ml Ethanol wird in Anwesenheit von 500 mg 10 % Palladium auf Kohle als Katalysator bei 300 kPa 3 Stunden hydriert. Man erhält cis und trans-

9-Methoxy-10b-methyl-4-propyl-1,2,3,4a,5,10b-hexahydro-4H-[1]-benzopyrano[3,4-b]pyridin als Isomerengemisch.

b) Das Produkt von a) wird dann mit einer Lösung von 1,85 g Diphenylphosphin und 3,85 ml 2,2 M Butyllithium in 15 ml Tetrahydrofuran 5 Stunden unter Rückfluss erhitzt. Die Produkte werden mit 3N Salzsäure extrahiert, die wässrige Phase wird mit Base neutralisiert und mit Chloroform extrahiert. Nach Trocknen über Magnesiumsulfat wird das Lösungsmittel im Vakuum abgezogen und der Rückstand an 60 g Silicagel mit Ether/Hexan (1:1) als Eluierungsmittel chromatographiert. Man erhält nach Ueberführung in das Hydrochlorid trans-9-Hydroxy-10b-methyl-4-propyl-1,2,3,4a,5,10b-hexahydro-4H-[1]-benzopyrano[3,4-b]pyridinhydrochlorid, Fp. 238-241°, und cis-9-Hydroxy-10b-methyl-4-propyl-1,2,3,4a,5,10b-hexahydro-4H-[1]-benzopyrano[3,4-b]pyridinhydrochlorid, Fp. 244-245°.

c) Auf analoge Weise führt eine Mischung von cis und trans-7-Methoxy-4,10b-dimethyl-3,4a,5,10b-tetrahydro-4H-[1]-benzopyrano-[3,4-b]pyridin zu cis- und trans-7-(Methoxy und Hydroxy)-4,10b-dimethyl-1,2,3,4a,5,10b-hexahydro-4H-[1]-benzopyrano[3.4-b]pyridin; trans-7-Hydroxy-4,10b-dimethyl-1,2,3,4a,5,10b-hexahydro-4H-[1]-benzopyrano[3,4-b]pyridinhydrochlorid hat einen Fp. von 252-253°.

Beispiel 7:

Die folgenden Verbindungen werden analog zu den vorstehend beschriebenen Beispielen hergestellt:

a) 7-Methoxy-4-propyl-2,3,4a,5-tetrahydro-4H-[1]-benzopyrano[3,4-b]-pyridin [NMR: δ 0.90 (3H,t), 3.80 (3H,s)], wobei man ausgeht von 8-Methoxy-2H-[1]-benzopyran [J. Org. Chem. 39, 881 (1974)] über 8-Methoxy-2H-benzopyran-3-on, Fp. 78-80°;

b) 7-Hydroxy-4-propyl-2,3,4a,5-tetrahydro-4H-[1]-benzopyrano-[3,4-b]pyridinhydrochlorid, Fp. 195-196°;

c) trans-7-Hydroxy-4-propyl-1,2,3,4a,5,10b-hexahydro-4H-[1]-benzopyrano[3,4-b]pyridinhydrochlorid, Fp. 260-262°;

d) cis-7-Hydroxy-4-propyl-1,2,3,4a,5,10b-hexahydro-4H-[1]-benzopyrano[3,4-b]pyridinhydrochlorid, Fp. 220-223°;

e) (+)-trans-7-Hydroxy-4-propyl-1,2,3,4a,5,10b-hexahydro-4H-[1]-benzopyrano[3,4-b]pyridinhydrochlorid, Fp. 283-286°, $[\alpha]_D^{25}$ + 85,73° (c = 1,0, Wasser); Dibenzoylweinsäuresalz, Fp. 203-204°;

f) (-)-trans-7-Hydroxy-4-propyl-1,2,3,4a,5,10b-hexahydro-4H-[1]-benzopyrano[3,4-b]pyridinhydrochlorid, Fp. 285-287°, $[\alpha]_D^{25}$ -88,69° (c = 1,0, Wasser);

g) 7-Methoxy-4-methyl-2,3,4a,5-tetrahydro-4H-[1]-benzopyrano-[3,4-b]pyridinhydrochlorid, Fp. 236-239°, durch Kondensation von 8-Methoxy-2H-[1]-benzopyran-3-on mit 3-(Methylamino)-propionsäureethylester und weitere Schritte wie in Beispiel 1;

h) trans-7-Methoxy-4-methyl-1,2,3,4a,5,10b-hexahydro-4H-[1]-benzopyrano[3,4-b]pyridinhydrochlorid, Fp. 236-238°;

i) trans und cis-4-Butyl-7-hydroxy-1,2,3,4a,5,10b-hexahydro-4H-[1]-benzopyrano[3,4-b]-pyridinhydrochlorid, Fp.252-253° und 160-162°, durch Kondensation von 8-Methoxy-2H-[1]-benzopyran-3-on mit 3-(Butylamino)propionsäuremethylester und weitere Schritte wie in den Beispielen 1, 2 und 5;

j) 9-Hydroxy-4-methyl-2,3,4a,5-tetrahydro-4H-[1]-benzopyrano-[3,4-b]pyridinhydrochlorid, Fp. 265-268° (Z);

k) 7-Hydroxy-4-methyl-2,3,4a,5-tetrahydro-4H-[1]-benzopyrano-[3,4-b]pyridinhydrochlorid, Fp. 257-259°;

l) 8,9-Dihydroxy-4-propyl-2,3,4a,5-tetrahydro-4H-[1]-benzopyrano-[3,4-b]pyridinhydrochlorid, Fp. 205-206°;

m) 4-Methyl-2,3,4a,5-tetrahydro-4H-[1]-benzopyrano[3,4-b]pyridinhydrochlorid, Fp. 242-244°;

n) trans-7-Hydroxy-4-methyl-1,2,3,4a,5,10b-hexahydro-4H-[1]-benzopyrano[3,4-b]pyridinhydrochlorid, Fp. 275-277°;

o) cis-7-Hydroxy-4-methyl-1,2,3,4a,5,10b-hexahydro-4H-[1]-benzopyrano[3,4-b]pyridinhydrochlorid, Fp. 238-240°;

p) trans-9-Hydroxy-4-methyl-1,2,3,4a,5,10b-hexahydro-4H-[1]-benzopyrano[3,4-b]pyridinhydrochlorid, Fp. > 260° (Z);

q) trans-8,9-Dihydroxy-4-propyl-1,2,3,4a,5,10b-hexahydro-4H-[1]-benzopyrano[3,4-b]pyridinhydrochlorid, Fp. 290° (Z);

r) 4-Benzyl-9-methoxy-2,3,4a,5-tetrahydro-4H-[1]-benzopyrano-[3,4-b]pyridin durch Kondensation von 6-Methoxy-2H-[1]-benzopyran-3-on mit 3-Benzylaminopropionsäureethylester und weitere Schritte wie in Beispiel 1;

s) 4-Benzyl-9-hydroxy-2,3,4a,5-tetrahydro-4H-[1]-benzopyrano[3,4-b]-pyridin;

t) 4-Benzyl-9-methoxy-1,2,3,4a,5,10b-hexahydro-4H-[1]-benzopyrano-[3,4-b]pyridin;

u) 4-Benzyl-9-hydroxy-1,2,3,4a,5,10b-hexahydro-4H-[1]-benzopyrano-[3,4-b]pyridin.

Beispiel 8:

Zu einer Suspension von 3,0 g 9-Methoxy-4-propyl-1,2,3,5-tetrahydro-4H-[1]-benzothiopyrano[3,4-b]-pyridin-1-on in 150 ml wasserfreiem Ether werden 750 mg Lithiumaluminiumhydrid in einer Portion gegeben. Nach einstündigem Rühren bei Raumtemperatur wird der Ansatz mit 1,5 ml 10 %iger Natronlauge versetzt, filtriert und der Filterkuchen mit Ether gewaschen. Nach Abziehen des Lösungsmittels wird der Rückstand in 20 ml Ethanol aufgenommen, 5 ml Essigsäure und 1,5 g Natriumcyanborhydrid werden zugegeben. Nach zweistündigem Rühren bei Raumtemperatur wird der Ansatz in eine Natriumcarbonatlösung gegeben, und die Produkte werden mit Ether extrahiert. Nach Trocknen der organischen Phase wird das Lösungsmittel im Vakuum abgezogen, und der Rückstand wird an 60 g Silicagel chromatographiert, wobei man Ether/Dichlormethan als Eluierungsmittel verwendet. Man erhält der Reihe nach:

a) 9-Methoxy-4-propyl-2,3,4a,5-tetrahydro-4H-[1]-benzothiopyrano-[3,4-b]pyridin, charakterisiert als Hydrochlorid, Fp. 215-216°;

b) cis-9-Methoxy-4-propyl-1,2,3,4a,5,10b-hexahydro-4H-[1]-benzothiopyrano[3,4-b]pyridin; und

c) trans-9-Methoxy-4-propyl-1,2,3,4a,5,10b-hexahydro-4H-[1]-benzothiopyrano[3,4-b]pyridin.

Das Ausgangsmaterial wird wie folgt hergestellt: Eine Mischung von 7,5 g 6-Methoxythiochroman-3-on [J. Org. Chem. 34, 1566 (1969)], 5,8 g 3-(Propylamino)-propionsäuremethylester und 0,8 ml Trifluoressigsäure in 80 ml Toluol wird 22 Stunden in einem Dean Stark Apparat unter Rückfluss erhitzt. Weitere Portionen von je 0,8 ml Trifluoressigsäure werden nach 2, 5 und 16 Stunden zugegeben. Das Lösungsmittel wird entfernt, der Rückstand wird in Ether aufgenommen, und das Produkt wird mit 3 N Salzsäure extrahiert. Nach Neutralisation wird die wässrige Phase mit Essigester extrahiert, die organische Phase getrocknet und das Lösungsmittel abgezogen. Das Produkt wird aus einer kleinen Menge Methanol kristallisiert. Man erhält 9-Methoxy-4-propyl-1,2,3,5-tetrahydro-4H-[1]-benzothiopyrano[3,4-b)pyridin-1-on, Fp. 108-110°.

Auf analoge Weise lassen sich erhalten:

d) 4-Propyl-2,3,4a,5-tetrahydro-4H-[1]-benzothiopyrano[3,4-b]-pyridinhydrochlorid, Fp. 231-235°, ausgehend von Thiochroman-3-on;

e) trans-4-Propyl-1,2,3,4a,5,10b-hexahydro-4N-[1]-benzothiopyrano[3,4-b]pyridinhydrochlorid;

f) 7-Methoxy-4-propyl-2,3,4a,5-tetrahydro-4H-[1]-benzothiopyrano-[3,4-b]pyridinhydrochlorid, Fp. 244-246°, ausgehend von 8-Methoxythiochroman-3-on;

g) trans-7-Methoxy-4-propyl-1,2,3,4a,5,10b-hexahydro-4H-[1]-benzothiopyrano[3,4-b]pyridin.

Das Ausgangsmaterial für die Verbindung von Beispiel 8f, 8-Methoxythiochroman-3-on wird wie folgt hergestellt: Zu einer Mischung von 28 g o-Methoxythiophenol, 50 g 45 % Kaliumhydroxid in 70 ml Dimethylsulfoxid werden 33 g α-(Brommethyl)-acrylsäure in 30 ml Dimethylsulfoxid gegeben, wobei die Temperatur bei 50 - 60° gehalten wird. Nach 1 Stunde wird der Ansatz in verdünnte Salzsäure gegeben, die Produkte werden mit Ether extrahiert. Die etherische Phase wird mit Natriumbicarbonatlösung extrahiert. Nach Ansäuern der wässrigen Bicarbonatphase wird α-(o-Methoxyphenylthiomethyl)acrylsäure, Fp. 101-104°, erhalten.

Eine Mischung von 22,5 g α-(o-Methoxyphenylthiomethyl)acrylsäure, 2,53 g Triethylamin und 200 ml o-Dichlorbenzol wird 12 Stunden auf 195° erhitzt. Nach Verdünnen mit Ether werden die Produkte mit Natriumbicarbonatlösung extrahiert. Ansäuern der basischen Extrakte ergibt 8-Methoxy-3,4-dihydro-2H-[1]-benzothiopyran-3-carbonsäure, Fp. 138-144°.

Zu einer Lösung von 10 g 8-Methoxy-3,4-dihydro-2H-[1]-benzothiopyran-3-carbonsäure in 200 ml Dichlormethan werden portionsweise 6,2 g N-Chlorsuccinimid gegeben. Nach 10 Minuten werden 60 g Silicagel zugefügt. Der Ansatz wird 15 Minuten gerührt, über 40 g Silicagel filtriert, wobei Ether/Dichlormethan (1:1) als Elutionsmittel verwendet werden. Die Lösungsmittel werden auf 100 ml konzentriert, 5 ml Triethylamin und 5 g Chlorkohlensäureethylester werden zugegeben. Nach Einengen werden 5 g Natriumazid in 60 ml Dimethylformamid zugegeben. Der Ansatz wird 1 Stunde gerührt. Nach Verdünnen mit Wasser werden die Produkte mit Ether extrahiert, der etherische Extrakt getrocknet und zur Trockne eingeengt. 150 ml 10 %ige Schwefelsäure werden zugegeben, und der Ansatz wird 2 Stunden unter Rückfluss erhitzt. Der Ansatz wird mit Ether extrahiert, der etherische Extrakt mit verdünnter Natriumbicarbonatlösung gewaschen, getrocknet und zur Trockne eingeengt. Kristallisation aus Methanol ergibt 8-Methoxythiochroman-3-on, Fp. 60°.

Beispiel 9:

a) Zu einer Lösung von 1,8 ml Diphenylphosphin in 25 ml Tetrahydrofuran werden bei 0° 4,7 ml 2,1 M Butyllithium in Hexan gegeben, danach 1,3 g 9-Methoxy-4-propyl-2,3,4a,5-tetrahydro-4H-[1]-

benzothiopyrano[3,4-b]pyridin. Nach fünfstündigem Erhitzen unter Rückfluss unter Stickstoffatmosphäre wird der Ansatz mit Ether verdünnt und das Produkt mit 10 %iger Natronlauge extrahiert. Die wässrige Phase wird neutralisiert und das Produkt mit Essigester extrahiert. Nach dem Trocknen wird das Lösungsmittel im Vakuum abgezogen. Der Rückstand wird in Ethanol aufgenommen, mit ethanolischem Chlorwasserstoff angesäuert, und die erhaltenen Kristalle werden gesammelt. Es handelt sich um 9-Hydroxy-4-propyl-2,3,4a,5-tetrahydro-4H-[1]-benzothiopyrano[3,4-b]pyridinhydrochlorid, Fp. 275-277°.

Auf analoge Weise werden hergestellt:

b) cis-9-Hydroxy-4-propyl-1,2,3,4a,5,10b-hexahydro-4H-[1]-benzothiopyrano[3,4-b]pyridinhydrochlorid, Fp. 246-248°;

c) trans-9-Hydroxy-4-propyl-1,2,3,4a,5,10b-hexahydro-4H-[1]-benzothiopyrano[3,4-b]pyridinhydrochlorid, Fp. 285-286°;

d) 7-Hydroxy-4-propyl-2,3,4a,5-tetrahydro-4H-[1]-benzothiopyrano-[3,4-b]pyridinhydrochlorid, Fp. 225-228°;

e) trans-7-Hydroxy-4-propyl-1,2,3,4a,5,10b-hexahydro-4H-[1]-benzothiopyrano[3,4-b]pyridinhydrochlorid;

Beispiel 10:

a) Zu einer Lösung von 550 mg trans-9-Hydroxy-4-propyl-1,2,3,4a,5,10b-hexahydro-4H-[1]-benzopyrano-[3,4-b]pyridinhydrochlorid in 10 ml Dichlormethan werden 1,4 g Diisopropylethylamin und 680 mg Benzoylchlorid gegeben, und die Mischung wird 2 Stunden gerührt. Nach Verdünnen mit Dichlormethan wird der Ansatz mit Wasser und gesättigter Natriumbicarbonatlösung gewaschen, über Magnesiumsulfat getrocknet und das Lösungsmittel im Vakuum abgezogen. Zugabe ausreichender Mengen ethanolischen Chlorwasserstoffs führt zum Ausfallen von trans-9-Benzoyloxy-4-propyl-1,2,3,4a,5,10b-hexahydro-4H-[1]-benzopyrano[3,4-b]pyridinhydrochlorid, Fp. 238-240°.

Auf analoge Weise werden hergestellt:

b) trans-7-Benzoyloxy-4-propyl-1,2,3,4a,5,10b-hexahydro-4H-[1]-benzopyrano[3,4-b]pyridinhydrochlorid;

c) 9-Benzoyloxy-4-propyl-2,3,4a,5-tetrahydro-4H-[1]-benzopyrano-[3,4-b]pyridin;

d) trans-9-Nicotinoyloxy-4-propyl-1,2,3,4a,5,10b-hexahydro-4H-[1]-benzopyrano[3,4-b]pyridin;

e) trans-9-Pivaloyloxy-4-propyl-1,2,3,4a,5,10b-hexahydro-4H-[1]-benzopyrano[3,4-b]pyridinhydrochlorid, Fp. 229-230°;

f) trans-9-Dimethylaminocarbonyloxy-4-propyl-1,2,3,4a,5,10b-hexahydro-4H-[1]-benzopyrano[3,4-b]-pyridinhydrochlorid, Fp. 185-187°;

g) trans-9-Acetoxy-4-propyl-1,2,3,4a,5,10b-hexahydro-4H-[1]-benzopyrano[3,4-b]pyridinhydrochlorid;

h) 9-Benzoyloxy-4-propyl-2,3,4a,5-tetrahydro-4H-[1]-benzothiopyrano-[3,4-b]pyridinhydrochlorid;

i) 9-Acetoxy-4-propyl-1,2,3,4a,5,10b-hexahydro-4H-[1]-benzothiopyrano[3,4-b]pyridinhydrochlorid;

j) 4-Butyl-9-(2-methylpropionyloxy)-1,2,3,4a,5,10b-hexahydro-4H-[1]-benzopyrano[3,4-b]pyridin.

Beispiel 11:

a) Zu einer gut gerührten Lösung von 1,16 g 9-Methoxy-4-methyl-2,3,4a,5-tetrahydro-4H-[1]-benzopyrano[3,4-b]pyridin in 15 ml Tetrahydrofuran werden 3,4 ml 1,5 H Butyllithium in Hexan bei 0° gegeben. Nach 5 Minuten bei 0° wird der Ansatz auf -70° abgekühlt und 0,6 ml tert.-Butylisocyanat werden zugegeben. Nach Erwärmen auf Raumtemperatur wird der Ansatz mit Wasser verdünnt, und die Produkte werden mit Ether extrahiert. Nach Trocknen über Magnesiumsulfat werden die Lösungsmittel im Vakuum abgezogen, und der Rückstand wird aus Ether/Hexan kristallisiert. Man erhält cis-2-(N-t-Butylcarbamoyl)-9-methoxy-4-methyl-2,3,4a,5-tetrahydro-4H-[1]-benzopyrano[3,4-b]pyridin, das mit ethanolischem Chlorwasserstoff in cis-2-(N-t-Butylcarbamoyl)-9-methoxy-4-methyl-2,3,4a,5-tetrahydro-4H-[1]-benzopyrano[3,4-b]pyridinhydrochlorid, Fp. 188-189° (aus Ethanol/Ether) übergeführt wird.

b) Eine Lösung von 950 mg cis-2-(N-t-Butylcarbamoyl)-9-methoxy-4-methyl-2,3,4a,5-tetrahydro-4H-[1]-benzopyrano[3,4-b]pyridin in 50 ml 0,1 M Natriumethanolat in Ethanol wird 15 Minuten auf 70° erhitzt. Nach Abziehen des Lösungsmittels im Vakuum wird der Rückstand über 20 g Silicagel chromatographiert, wobei Ether/Hexan (1:1) als Eluierungsmittel verwendet werden. Man erhält trans-2-(N-t-Butylcarbamoyl)-9-methoxy-4-methyl-2,3,4a,5-tetrahydro-4H-[1]-benzopyrano[3,4-b]pyridin, kristallisiert als Hydrochlorid, Fp. 238-240°.

Beispiel 12:

a) Zu einer Lösung von 500 mg trans-7-Hydroxy-4-methyl-1,2,3,4a,5,10b-hexahydro-4H-[1]-benzopyrano-

[3,4-b]pyridin in 5 ml Essigsäure werden 365 mg Brom gegeben, die Mischung wird 4 Stunden bei Raumtemperatur gerührt. Nach Verdünnen mit Wasser, Neutralisieren der wässrigen Phase, Extraktion mit Dichlormethan und Trocknen des Extrakts über Magnesiumsulfat wird das Lösungsmittel im Vakuum abgezogen. Der Rückstand wird in Ethanol aufgenommen und mit ethanolischem Chlorwasserstoff angesäuert. Man erhält trans-10-Brom-7-hydroxy-4-methyl-1,2,3,4a,5,10b-hexahydro-4H-[1]-benzopyrano-[3,4-b]pyridinhydrochlorid, Fp. 282-284°.

b) Auf analoge Weise führt die Umsetzung von trans-7-Methoxy-4-methyl-1,2,3,4a,5,10b-hexahydro-4H-[1]-benzopyrano[3,4-b]pyridin mit 2 Aequivalenten Brom zu trans-8,10-Dibrom-7-methoxy-4-methyl-1,2,3,4a,5,10b-hexahydro-4H-[1]-benzopyrano[3,4-b]pyridinhydrochlorid, Fp. 234-237°.

Beispiel 13:

a) Zu einer Lösung von 219 mg trans-7-Hydroxy-4-methyl-1,2,3,4a,5,10b-hexahydro-4H-[1]-benzopyrano-[3,4-b]pyridin in 5 ml Tetrahydrofuran und 1 ml Dimethylformamid werden unter Rühren 75 mg 50 % Natriumhydriddispersion in Mineralöl gegeben. Danach folgen 115 mg Allylbromid, und die Mischung wird 3 Stunden unter Rückfluss gerührt. Nach Verdünnen mit Ether wird der Ansatz mit Wasser gewaschen, über Magnesiumsulfat getrocknet, und das Lösungsmittel wird im Vakuum abgezogen. Der Rückstand wird in Essigester aufgenommen und mit ethanolischem Chlorwasserstoff behandelt. Man erhält trans-7-Allyloxy-4-methyl-1,2,3,4a,5,10b-hexahydro-4H-[1]-benzopyrano[3,4-b]pyridinhydrochlorid, Fp. 229-230°.

Auf analoge Weise werden hergestellt:

b) trans-9-Benzyloxy-4-propyl-1,2,3,4a,5,10b-hexahydro-4H-[1]-benzopyrano[3,4-b]pyridin durch Alkylierung von trans-9-Hydroxy-4-propyl-1,2,3,4a,5,10b-hexahydro-4H-[1]-benzopyrano[3,4-b]pyridin mit Benzylbromid;

c) trans-9-Propargyloxy-4-propyl-1,2,3,4a,5,10b-hexahydro-4H-[1]-benzopyrano[3,4-b]pyridin;

d) trans-7-Butoxy-4-propyl-2,3,4a,5-tetrahydro-4H-[1]-benzopyrano-[3,4-b]pyridin;

e) 7-Allyloxy-4-propyl-2,3,4a,5-tetrahydro-4H-[1]-benzothiopyrano-[3,4-b]pyridin;

f) trans-9-Ethoxy-4-propyl-1,2,3,4a,5,10b-hexahydro-4H-[1]-benzopyrano[3,4-b]pyridin.

Beispiel 14:

Eine Lösung von 1,5 g trans-4-Benzyl-9-methoxy-1,2,3,4a,5,10b-hexahydro-4H-[1]-benzopyrano[3,4-b]-pyridin in 30 ml Ethanol wird bei einem Druck von 300 kPa in Anwesenheit von 0,5 g 10 % Palladium auf Kohle hydriert, bis 1 Mol Wasserstoff verbraucht ist. Der Ansatz wird filtriert, zur Trockne eingeengt, man erhält 9-Methoxy-1,2,3,4a,5,10b-hexahydro-4H-[1]-benzopyrano[3,4-b]pyridin.

Beispiel 15:

Zu einer Suspension von 3,8 g Lithiumaluminiumhydrid in 100 ml Tetrahydrofuran werden zuerst tropfenweise 3,6 g konzentrierte Schwefelsäure bei -5 bis -10° gegeben, danach langsam und unter Rühren 5,0 g. 9-Methoxy-4-propionyl-1,2,3,4a,5,10b-hexahydro-4H-[1]-benzopyrano[3,4-b]pyridin. Man lässt den Ansatz auf Raumtemperatur kommen, rührt 18 Stunden und erhitzt am Schluss 1 Stunde unter Rückfluss. Nach Abkühlen wird der Ansatz mit Essigester versetzt, danach mit einer kleinen Menge Wasser und 3 N Natronlauge, filtriert und im Vakuum eingeengt. Der Rückstand wird in Essigester aufgenommen und die Essigesterlösung mit Wasser gewaschen, getrocknet und eingeengt. Man erhält 9-Methoxy-4-propyl1,2,3,4a,5,10b-hexahydro-4H-[1]-benzopyrano[3,4-b]pyridin.

Das Ausgangsmaterial wird hergestellt, indem man 9-Methoxy-1,2,3,4a,5,10b-hexahydro-4H-[1]-benzopyrano[3,4-b]pyridin in Anwesenheit von 2 Aequivalenten pulverisiertem Kaliumcarbonat in Dichlormethanlösung mit 1 Mol Propionsäurechlorid über Nacht bei Raumtemperatur behandelt.

Beispiel 16:

Zu einer Suspension von 20 g 4-(3-Aminopropyl)-6-methoxy-2H-[1]-benzopyranhydrochlorid in 200 ml Essigester werden 12,5 g Brom bei Raumtemperatur gegeben. Nach 15 Minuten bei Raumtemperatur werden 24 g Triethylamin zugesetzt, so dass 4-(3-Aminopropyliden)-3-brom-6-methoxy-2H-[1]-benzopyran gebildet wird. Der Ansatz wird 2 Stunden unter Rückfluss erhitzt. Nach Waschen mit Wasser und Trocknen wird das Lösungsmittel im Vakuum abgezogen. Der Rückstand wird in Ethanol aufgenommen und mit ethanolischem Chlorwasserstoff angesäuert. Man erhält 9-Methoxy-2,3,4a,5-tetrahydro-4H-[1]-benzopyrano-

[3,4-b]pyridinhydrochlorid, Fp. 285-286°.

Das Ausgangsmaterial wird wie folgt hergestellt: Zu einer Lösung von 26,9 g 4-Methoxyphenylpropargylether in 300 ml Tetrahydrofuran bei -70° werden 74 ml 2,3 M Butyllithium in Hexan gegeben. Nach 20 Minuten bei -70° werden 26,2 g 3-Brom-1-chlorpropan in 86 ml Hexamethylphosphorsäuretriamid gegeben, und der Ansatz wird 1 Stunde bei 0° gerührt. Man gibt den Ansatz auf Wasser, extrahiert das Produkt mit Ether und entfernt das Lösungsmittel im Vakuum nach Trocknen über Magnesiumsulfat. Flüchtige Bestandteile werden bei 150°/13 Pa entfernt. Der Rückstand wird in 400 ml Dimethylformamid aufgenommen, 45 g Kaliumphthalimid werden zugegeben, und der Ansatz wird 16 Stunden auf 50° erhitzt. Der Ansatz wird auf Wasser gegeben und das Produkt mit Ether extrahiert. Nach Trocknen wird das Lösungsmittel im Vakuum abgezogen, und der Rückstand wird aus Isopropanol kristallisiert. Man erhält 6-(4-Methoxyphenoxy)-1-phthalimido-4-hexinylamin, Fp. 51-53°.

Eine Mischung von 40 g 6-(4-Methoxyphenoxy)-1-phthalimido-4-hexinylamin und 8,5 g N,N-Diethylanilin in 400 ml N-Methylpyrrolidinon wird 30 Stunden auf 210° erhitzt. Man gibt den Ansatz in Wasser, das 57 ml 1N Salzsäure enthält, extrahiert das Produkt mit Ether, trocknet und entfernt das Lösungsmittel im Vakuum. Der Rückstand wird aus Isopropanol kristallisiert, man erhält 4-(3-Phthalimidopropyl)-6-methoxy-2H-[1]-benzopyran, Fp. 78-80°.

Eine Mischung von 30 g 4-(3-Phthalimidopropyl)-6-methoxy-2H-[1]-benzopyran und 9 g Hydrazinhydrat in 600 ml Ethanol wird 2 Stunden unter Rückfluss erhitzt. Nach Entfernung eines Grossteils des Lösungsmittels im Vakuum wird 10 %ige Natronlauge zugesetzt und das Produkt mit Essigester extrahiert. Nach dem Trocknen werden die Essigesterlösungen mit Chlorwasserstoff angesäuert. Man erhält 4-(3-Aminopropyl)-6-methoxy-2H-[1]-benzopyranhydrochlorid, Fp. 141-142°.

Beispiel 17:

Eine Mischung von 14 g 9-Methoxy-2,3,4a,5-tetrahydro-4H-[1]-benzopyrano[3,4-b]pyridinhydrochlorid und 140 ml Ethanol und eine Lösung von 2,95 g Natriumcarbonat in 10 ml Wasser und 3 g 10 % Palladium auf Kohle Katalysator wird bei einem Druck von 300 kPa 16 Stunden bei 50° hydriert. Nach der Zugabe von 15 g Propionaldehyd wird die Hydrierung weitere 16 Stunden unter den gleichen Bedingungen fortgesetzt. Nach Filtrieren wird das Lösungsmittel im Vakuum abgezogen.Der Rückstand wird in Ethanol aufgenommen und mit ethanolischem Chlorwasserstoff angesäuert. Man erhält trans-9-Methoxy-4-propyl-1,2,3,4a,5,10b-hexahydro-4H-[1]-benzopyrano-[3,4-b]pyridinhydrochlorid, Fp. 252-254° (die Verbindung des Beispiels 2a).

Beispiel 18:

a) Eine Lösung von 400 mg trans-7-Allyloxy-4-methyl-1,2,3,4a,5,10b-hexahydro-4H-[1]-benzopyrano[3,4-b)pyridinhydrochlorid in 10 ml 1-Methyl-2-pyrrolidinon wird 2 Stunden auf 190° erhitzt, in Wasser gegeben, und die Produkte werden mit Essigester extrahiert. Nach Trocknen über Magnesiumsulfat wird das Lösungsmittel abgezogen. Der Rückstand wird in 20 ml Ethanol in Anwesenheit von 100 mg 10 % Palladium auf Kohle Katalysator 3 Stunden bei 300 kPa hydriert. Nach dem Filtrieren wird das Lösungsmittel entfernt und das Hydrochlorid mit ethanolischem Chlorwasserstoff hergestellt. Man erhält trans-7-Hydroxy-4-methyl-10-propyl-1,2,3,4a,5,10b-hexahydro-4H-[1]-benzopyrano[3,4-b]-pyridinhydrochlorid, Fp. 283-285°.

b) Die Mutterlauge wird zur Trockne eingeengt, der Rückstand in die freie Base übergeführt und an Silicagel mit Aceton/Hexan (1:3) chromatographiert. Man erhält trans-7-Hydroxy-4-methyl-8-propyl-1,2,3,4a,5,10b-hexahydro-4H-[1]-benzopyrano[3,4-b]pyridin.

Beispiel 19:

Eine Lösung von 628 mg trans-7-Methoxy-4-methyl-1,2,3,4a,5,10b-hexahydro-4H-[1]-benzopyrano[3,4-b]pyridin und 1 g Chlorkohlensäurephenylester in 20 ml Toluol wird 3 Stunden unter Rückfluss erhitzt. Nach Verdünnen mit Ether und Waschen mit Salzsäure wird das Lösungsmittel abgezogen und 1 g Kaliumhydroxid und 20 ml Dioxan werden zugesetzt. Nach zweistündigem Erhitzen unter Rückfluss wird der Ansatz mit Wasser verdünnt und das Produkt mit Ether extrahiert. Nach Trocknen und Entfernen des Lösungsmittels wird mittels ethanolischem Chlorwasserstoff das Hydrochlorid hergestellt. Man erhält trans-7-Methoxy-1,2,3,4a,5,10b-hexahydro-4H-[1]-benzopyrano[3,4-b]pyridinhydrochlorid, Fp. 207-210°.

Example 20:

a) Eine Lösung von 600 mg trans-8-Brom-7-methoxy-4-propyl-1,2,3,4a,5,10b-hexahydro-4H-[1]-benzopyrano[3,4-b]pyridin in 10 ml Tetrahydrofuran wird bei -78° mit 1,68 ml 2,1M Butyllithium behandelt. Nach 10 Minuten werden 27 ml Dimethylformamid zugegeben. Nach 30 Minuten bei 0° wird der Ansatz in Wasser gegeben, die Produkte werden mit Ether extrahiert. Nach Trocknen wird das Lösungsmittel entfernt. Der Rückstand wird mit 35 mg Natriumborhydrid in 5 ml Methanol behandelt. Nach 10 Minuten wird das Lösungsmittel entfernt und der Rückstand an 10 g Silicagel mit Aceton/Hexan (1:2) chromatographiert. Man erhält trans-8-Hydroxymethyl-7-methoxy-4-propyl-1,2,3,4a,5,10b-hexahydro-4H-[1]-benzopyrano[3,4-b]pyridin.

b) Die Umsetzung von trans-8-Hydroxymethyl-7-methoxy-4-propyl-1,2,3,4a,5,10b-hexahydro-4H-[1]-benzopyrano[3,4-b]pyridin mit Diphenylphosphin und Butyllithium analog zum Verfahren von Beispiel 9 und nachfolgende Behandlung mit Fumarsäure ergibt trans--7-Hydroxy-8-hydroxymethyl-4-propyl-1,2,3,4a,5,10b-hexahydro-4H-[1]-benzopyrano[3,4-b]pyridinfumarat, Fp. 198-200°.

Beispiel 21:

Eine Lösung von 1,0 g trans-10-Brom-7-hydroxy-4-methyl-1,2,3,4a,5,10b-hexahydro-4H-[1]-benzopyrano[3,4-b]pyridin in 10 ml Tetrahydrofuran wird zu 160 mg 50 % Natriumhydrid in 10 ml Tetrahydrofuran und 10 ml Dimethylformamid gegeben. Nach 10 Minuten bei Raumtemperatur werden 420 ml Dimethylsulfat zugesetzt und der Ansatz wird für 1/2 Stunde gerührt. Der Ansatz wird mit Wasser verdünnt und mit Ether extrahiert. Nach Trocknen über Magnesiumsulfat wird das Lösungsmittel im Vakuum abgezogen. Der Rückstand wird in ethanolischem Chlorwasserstoff gelöst und Ether wird zugesetzt. Man erhält trans-10-Brom-7-methoxy-4-methyl-1,2,3,4a,5,10b-hexahydro-4H-[1]-benzopyrano[3,4-b]-pyridinhydrochlorid, Fp. 265-268°.

Beispiel 22:

Die folgenden Verbindungen werden nach Verfahren hergestellt, die analog zu den in den vorstehenden Beispielen beschriebenen sind:

a) 7-Methoxy-2,4-dimethyl-2,3,4a,5-tetrahydro-4H-[1]-benzopyrano-[3,4-b]pyridinhydrochlorid, Fp. 236-239°,

b) trans-7-Methoxy-4-methyl-10-propyl-1,2,3,4a,5,10b-hexahydro-4H-[1]-benzopyrano[3,4-b]-pyridinhydrochlorid, Fp. 173-175°,

c) trans-7-Hydroxy-1,2,3,4a,5,10b-hexahydro-4H-[1]-benzopyrano-[3,4-b]pyridinhydrochlorid, Fp. 325° (Z);

d) trans-7-Methoxy-4-methyl-8-propyl-1,2,3,4a,5,10b-hexahydro-4H-[1]-benzopyrano[3,4-b]-pyridinhydrochlorid, Fp. 181-183°,

e) trans-2-Ethyl-9-methoxy-4-propyl-2,3,4a,5-tetrahydro-4H-[1]-benzopyrano[3,4-b]pyridinhydrochlorid, Fp. 210-212°,

f) cis-2-Ethyl-9-methoxy-4-propyl-2,3,4a,5-tetrahydro-4H-[1]-benzopyrano[3,4-b]pyridinhydrochlorid, Fp. 206-207°,

g) 4-Propyl-2,3,4a,5-tetrahydro-4H-[1]-benzopyrano[3,4-b]pyridinhydrochlorid, Fp. 257-259°,

h) 4-Butyl-2,3,4a,5-tetrahydro-4H-[1]-benzopyrano[3,4-b]pyridinhydrochlorid, Fp. 241-243°,

i) trans-4-Butyl-1,2,3,4a,5,10b-hexahydro-4H-[1]-benzopyrano-[3,4-b]pyridinhydrochlorid, Fp. 259-262°

j) cis-4-Butyl-1,2,3,4a,5,10b-hexahydro-4H-[1]-benzopyrano[3,4-b]-pyridinhydrochlorid, Fp. 186-189°,

k) trans-4-Propyl-1,2,3,4a,5,10b-hexahydro-4H-[1]-benzopyrano-[3,4-b]pyridinhydrochlorid, Fp. 257-262°,

l) trans-10b-Ethyl-9-hydroxy-4-propyl-1,2,3,4a,5,10b-hexahydro-4H-[1]-benzopyrano[3,4-b]-pyridinhydrochlorid, Fp. 242-244°,

m) trans-2β-Ethyl-9-methoxy-4-propyl-1,2,3,4a,5,10b-hexahydro-4H-[1]-benzopyrano[3,4-b]-pyridinhydrochlorid, Fp. 194-196°,

n) 10-Methoxy-4,8-dimethyl-2,3,4a,5-tetrahydro-4H-[1]-benzopyrano-[3,4-b]pyridinhydrochlorid, Fp. 244-245°,

o) 8-Methoxy-4,10-dimethyl-2,3,4a,5-tetrahydro-4H-[1]-benzopyrano-[3,4-b]pyridinhydrochlorid, Fp. 235-238°,

p) trans-10-Methoxy-4,8-dimethyl-1,2,3,4a,5,10b-hexahydro-4H-[1]-benzopyrano[3,4-b]-pyridinhydrochlorid, Fp. 228-232°,

q) 10-Methoxy-4-methyl-2,3,4a,5-tetrahydro-4H-[1]-benzopyrano-[3,4-b]pyridinhydrochlorid, Fp. 247-250°,

r) trans-10-Hydroxy-4-methyl-1,2,3,4a,5,10b-hexahydro-4H-[1]-benzopyrano[3,4-b]pyridinhydrochlorid, Fp.

302-305°,

s) trans-10-Benzyloxy-4-methyl-1,2,3,4a,5,10b-hexahydro-4H-[1]-benzopyrano[3,4-b]pyridinhydrochlorid, Fp. 218-220°,

t) trans-10-Methoxy-4-propyl-1,2,3,4a,5,10b-hexahydro-4H-[1]-benzopyrano[3,4-b]pyridinhydrochlorid, Fp. 252-255° (Z),

u) trans-10-Methoxy-4-methyl-1,2,3,4a,5,10b-hexahydro-4H-[1]-benzopyrano[3,4-b]pyridinhydrochlorid, Fp. 231-234°,

v) trans-10-Hydroxy-4-propyl-1,2,3,4a,5,10b-hexahydro-4H-[1]-benzopyrano[3,4-b]pyridinhydrochlorid, Fp. 295-297°,

w) trans-7-Methoxy-4-propyl-1,2,3,4a,5,10b-hexahydro-4H-[1]-benzopyrano[3,4-b]pyridinhydrochlorid, Fp. 249-251°,

x) trans-8-Hydroxy-4-propyl-1,2,3,4a,5,10b-hexahydro-4H-[1]-benzopyrano[3,4-b]pyridinhydrochlorid, Fp. 275-278 (Z).

Beispiel 23:

Eine Mischung von 3 g 9-Methoxy-4-propyl-1,2,3,5-tetrahydro-4H-[1]-benzopyrano[3,4-b]pyridin-3-on in 100 ml Tetrahydrofuran wird mit 1,0 g Lithiumaluminiumhydrid 3 Stunden unter Rückfluss erhitzt. Nach Zusatz von Wasser, Filtrieren und Entfernen des Lösungsmittels wird der Rückstand mit 1 g Natriumcyanborhydrid in 50 ml Ethanol und 2 ml Eisessig behandelt. Nach 4 Stunden bei Raumtemperatur wird der Ansatz zu einer wässrigen Natriumcarbonatlösung gegeben, das Produkt wird mit Ether extrahiert und das Lösungsmittel abgezogen. Der Rückstand wird in das Hydrochlorid übergeführt. Man erhält das cis und trans 9-Methoxy-4-propyl-1,2,3,4a,5,10b-hexahydro-4H-[1]-benzopyrano[3,4-b]pyridinhydrochlorid von Beispiel 2.

Das Ausgangsmaterial wird wie folgt hergestellt: Eine Lösung von 17,8 g 6-Methoxy-3-chromanon, 7,5 g Pyrrolidin und 0,1 ml Trifluoressigsäure in 200 ml Toluol wird 8 Stunden in einem Dean-Stark Apparat unter Rückfluss erhitzt. Nach Entfernen des Lösungsmittels im Vakuum werden 14,0 g Acrylamid zugegeben, und die Mischung wird 3 Stunden auf 80° erhitzt. Wasser wird zugesetzt, die organische Phase abgetrennt und zur Trockne eingeengt. Man erhält 9-Methoxy-1,2,3,5-tetrahydro-4H-[1]-benzopyrano[3,4-b]pyridin-3-on.

Eine Mischung von 2,31 g der vorstehenden Verbindung wird 1 Stunde mit 240 mg Natriumhydrid in Tetrahydrofuran unter Rückfluss erhitzt. 2,0 g Propyliodid werden zugesetzt, das Erhitzen wird 2 Stunden fortgesetzt. Wässrige Aufarbeitung und Extraktion ergibt 9-Methoxy-4-propyl-1,2,3,5-tetrahydro-4H-[1]-benzopyrano[3,4-b]pyridin-3-on.

Beispiel 24:

9-Methoxy-4-propyl-2,3,4a,5-tetrahydro-4H-[1]-benzopyrano[3,4-b]pyridin-2-on wird mit Lithiumaluminiumhydrid in Ether erhitzt. Das erhaltene Produkt wird in Anwesenheit von 10 % Palladium auf Kohle in Ethanol hydriert. Man erhält 9-Methoxy-4-propyl-1,2,3,4a,5,10b-hexahydro-4H-[1]-benzopyrano[3,4-b]pyridin.

Das Ausgangsmaterial wird wie folgt hergestellt: Eine Mischung von 2,57 g 3-Brom-6-methoxy-4-chromanon und 3,3 g 1-(Propylamino)-2-propanonethylenketal in 500 ml Toluol wird 17 Stunden unter Rückfluss erhitzt. Das erhaltene Produkt wird in 20 ml Nitromethan gelöst, und 50 ml 85 % Polyphosphorsäure werden zugesetzt. Nach 48 Stunden bei Raumtemperatur wird der Ansatz auf Eis gegeben. Man erhält 9-Methoxy-4-propyl-2,3,4a,5-tetrahydro-4H-[1]-benzopyrano-[3,4-b]pyridin-2-on.

Beispiel 25:

Eine Mischung von 2,0 g N-(3-Chlorpropyl)-propylamin und 1,78 g 6-Methoxy-3-chromanon in 100 ml Toluol wird in einem Dean-Stark Apparat mit Wasserabscheider 17 Stunden unter Rückfluss erhitzt. Man erhält nach Entfernung des Lösungsmittels 9-Methoxy-4-propyl-1,2,3,5-tetrahydro-4H-[1]-benzopyrano[3,4-b]pyridin. Reduktion mit Natriumcyanborhydrid in Ethanol in Anwesenheit von Eisessig ergibt 9-Methoxy-4-propyl-1,2,3,4a,5,10b-hexahydro-4H-[1]-benzopyrano[3,4-b]pyridin von Beispiel 2.

Beispiel 26:

Zu einer Lösung von 2,3 g trans-10-Brom-7-methoxy-4-methyl-1,2,3,4a,5,10b-hexahydro-4H-[1]-benzopyrano[3,4-b]pyridin (Beispiel 21) in 45 ml Tetrahydrofuran werden bei -70° 5,6 ml 2,6 M t-Butyllithium in Hexan gegeben. Nach 5 Minuten bei -70° werden 1,36 g Methyliodid zugesetzt. Nach

Erwärmen auf 0° wird der Ansatz mit Essigester verdünnt und mit Wasser gewaschen. Nach Trocknen über Magnesiumsulfat wird das Lösungsmittel im Vakuum abgezogen. Ansäuern des Rückstandes mit ethanolischer Chlorwasserstoffsäure ergibt trans-7-Methoxy-4,10-dimethyl-1,2,3,4a,5,10b-hexahydro-4H-[1]-benzopyrano[3,4-b]pyridinhydrochlorid, Fp. 277-278°.

Beispiel 27:

a) Eine Mischung von 21,6 l 47-49 % Bromwasserstoffsäure und 2,27 kg trans-9-Methoxy-4-propyl-1,2,3,4a,5,10b-hexahydro-4H-[1]-benzopyrano[3,4-b]pyridinhydrochlorid wird 2 Stunden unter Rühren auf 105-110° erhitzt. Die Hauptmenge Bromwasserstoffsäure wird dann durch Destillation (125°/400 Pa) entfernt, ein Gemisch von 10,5 l Methanol und 9,5 l Wasser wird zugegeben, die Suspension wird auf 75° erhitzt. Die heisse Mischung wird vorsichtig mit 2,2 l 4 N Natriumhydroxidlösung auf pH 8 eingestellt, auf 10° abgekühlt und filtriert. Die vereinigten Niederschläge werden fünfmal mit je 1 l Wasser gewaschen und getrocknet (68°/400 Pa). Man erhält trans-9-Hydroxy-4-propyl-1,2,3,4a,5,10b-hexahydro-4H-[1]-benzopyrano[3,4-b]pyridin, Fp. 194-196°.

b) 1,71 kg dieser freien Base werden bei 78° in absolutem Ethanol gelöst, und 300 g Aktivkohle werden der gerührten Lösung zugesetzt. Die Mischung wird heiss filtriert, das klare Filtrat wird bei 65-70° mit 630 ml 12 N Salzsäure behandelt, auf 10° abgekühlt und zur Abtrennung des kristallinen Produkts filtriert. Dieses Material wird zweimal mit je 500 ml absolutem Ethanol gewaschen und bei 70°/27 Pa getrocknet. Man erhält trans-9-Hydroxy-4-propyl-1,2,3,4a,5,10b-hexahydro-4H-[1]-benzopyrano[3,4-b]-pyridinhydrochlorid (siehe Beispiel 5a), Fp. 295-297,5° (Zersetzung).

Beispiel 28:

3,02 kg Brom werden bei 16-23° während 35 Minuten zu einer Mischung von 4,83 kg 4-(3-Aminopropyl)-8-methoxy-2H-[1]-benzopyranhydrochlorid und 58 l Dichlormethan gegeben. Nach beendeter Zugabe wird der Ansatz 3,5 Stunden bei Raumtemperatur gerührt. Nach Zusatz von 5,8 kg Triethylamin erhält man 4-(3-Aminopropyliden)-3-brom-8-methoxy-2H-[1]-benzopyran. Der Ansatz wird 4 Stunden auf 60° erhitzt und dann über Nacht bei Raumtemperatur gerührt. Die Mischung wird fünfmal mit je 12 l Wasser und dann mit 8 l gesättigter Kochsalzlösung gewaschen, über wasserfreiem Natriumsulfat getrocknet, filtriert und bei vermindertem Druck eingeengt. Das erhaltene Oel wird in 12 l Isopropanol aufgenommen, mit 400 g Aktivkohle behandelt und filtriert. Das Filtrat wird auf 10° gekühlt, mit 1,8 l 10 N ethanolischem Chlorwasserstoff behandelt und eine Stunde bei 5-10° gerührt. Die Suspension wird filtriert, das Produkt dreimal mit je 4 l Isopropanol gewaschen und 72 Stunden bei 40°/400 Pa getrocknet. Man erhält 7-Methoxy-2,3,4a,5-tetrahydro-4H-[1]-benzopyrano[3,4-b]pyridinhydrochlorid, Fp. 195-198° (Zersetzung).

Das Ausgangsmaterial wird analog zu der Stufenfolge von Beispiel 16 hergestellt, wobei man für die erste Umsetzung anstelle von 4-Methoxyphenyl-propargylether 2-Methoxyphenyl-propargylether verwendet.

Beispiel 29:

a) Eine gerührte Mischung von 4,2 kg 7-Methoxy-2,3,4a,5-tetrahydro-4H-[1]-benzopyrano[3,4-b]-pyridinhydrochlorid, 900 g wasserfreiem Natriumcarbonat, 42 l Ethanol, 3,02 l Wasser und 840 g 10 % Palladium auf Kohle (50 % mit Wasser versetzt) wird bei 50° und einem Druck von 300 kPa 10 Stunden hydriert. Der Ansatz wird abgekühlt und belüftet, 4 kg Propionaldehyd werden zugegeben, und die Hydrierung wird weitere 24 Stunden bei 50° fortgesetzt. Der Ansatz wird erneut abgekühlt und belüftet, mit 1 l 10 N Natronlauge auf pH 12 eingestellt und zur Entfernung des Katalysators filtriert. Der Filter wird mit 10 l Ethanol und 12 l Wasser gewaschen. Die vereinigten Filtrate werden unter vermindertem Druck eingeengt. Das erhaltene Oel wird in 18 l Essigester aufgenommen, und die Lösung wird fünfmal mit je 4 l Wasser gewaschen, danach mit 4 l gesättigter Kochsalzlösung. Nach Trocknen über 10 kg wasserfreiem Natriumsulfat wird filtriert und bei vermindertem Druck eingeengt. Man erhält rohes trans-7-methoxy-4-propyl-1,2,3,4a,5,10b-hexahydro-4H-[1]-benzopyrano[3,4-b]pyridin, das geringe Mengen des cis-Isomers enthält. Die rohe freie Base wird bei 5° in 12 l absolutem Ethanol aufgenommen und 1,15 l 12 N ethanolischer Chlorwasserstoff werden zugesetzt. Die Suspension wird 15 Minuten gerührt und filtriert. Das Produkt wird aus absolutem Ethanol umkristallisiert und 24 Stunden bei 50°/13 Pa getrocknet. Man erhält auf diese Weise reines trans-7-Methoxy-4-propyl-1,2,3,4a,5,10b-hexahydro-4H-[1]-benzopyrano-[3,4-b]pyridinhydrochlorid, Fp. 250-251° (Zersetzung).

b) Eine Mischung von 16,3 l 47-49 % Bromwasserstoffsäure und 1,63 kg trans-7-Methoxy-4-propyl-1,2,3,4a,5,10b-hexahydro-4H-[1]-benzopyrano[3,4-b]pyridinhydrochlorid wird 5 Stunden auf 115-120° erhitzt. Sie wird dann auf 16 kg Eis gegeben und mit einer Lösung von 6,75 kg Natriumhydroxid in 11 l Wasser auf pH 12 gebracht, wobei die Temperatur unter 30° gehalten wird. Der Ansatz wird dann mit 800 ml 37 % Salzsäure auf pH 9 eingestellt und viermal mit je 12 l Essigester extrahiert. Die vereinigten Extrakte werden mit 8 l gesättigter Kochsalzlösung gewaschen, über 5 kg wasserfreiem Natriumsulfat getrocknet, mit 230 g Aktivkohle behandelt, 30 Minuten gerührt und filtriert. Das Filtrat wird unter vermindertem Druck eingeengt, man erhält trans-7-Hydroxy-4-propyl-1,2,3,4a,5,10b-hexahydro-4H-[1]-benzopyrano[3,4-b]pyridin, Fp. 111-114°.

c) Eine Mischung von 1,3 kg trans-7-Hydroxy-4-propyl-1,2,3,4a,5,10b-hexahydro-4H-[1]-benzopyrano[3,4-b]pyridin und 7,8 l absolutem Ethanol wird auf 60° erhitzt, bis eine Lösung erhalten wird. Nach Filtrieren wird das Filtrat mit 525 ml 37 % Salzsäure behandelt und die Mischung über Nacht bei Raumtemperatur gerührt. Die Suspension wird auf 0-5° gekühlt und filtriert. Der Feststoff wird viermal mit je 250 ml absolutem Ethanol gewaschen und 4 Stunden bei 80°/400 Pa getrocknet. Man erhält trans-7-Hydroxy-4-propyl-1,2,3,4a,5,10b-hexahydro-4H-[1]-benzopyrano[3,4-b]pyridinhydrochlorid (wie in Beispiel 7c), Fp. 264-265,5° (Zersetzung).

Beispiel 30:

Die folgenden Verbindungen werden auf zu den vorstehenden Beispielen analoge Weise hergestellt:

a) 10-Methoxy-4-propyl-2,3,4a,5-tetrahydro-4H-[1]-benzopyrano-[3,4-b]pyridinhydrochlorid, Fp. 252-254°,

b) trans-8-Ethyl-7-methoxy-4-methyl-1,2,3,4a,5,10b-hexahydro-4H-[1]-benzopyrano[3,4-b]-pyridinhydrochlorid, Fp. 217-221°,

c) trans-7-Ethoxy-4,8-dimethyl-1,2,3,4a,5,10b-hexahydro-4H-[1]-benzopyrano[3,4-b]pyridinhydrochlorid, Fp. 248-251°,

d) trans-10-Methoxy-8-methyl-4-propyl-1,2,3,4a,5,10b-hexahydro-4H-[1]-benzopyrano[3,4-b]-pyridinhydrochlorid, Fp. 251-253°,

e) trans-10-Benzyloxy-8-methyl-4-propyl-1,2,3,4a,5,10b-hexahydro-4H-[1]-benzopyrano[3,4-b]-pyridinhydrochlorid, Fp. 214-215°,

f) trans-10-Hydroxy-8-methyl-4-propyl-1,2,3,4a,5,10b-hexahydro-4H-[1]-benzopyrano[3,4-b]-pyridinhydrochlorid, Fp. 287-291°,

g) trans-8-Brom-7-methoxy-4-propyl-1,2,3,4a,5,10b-hexahydro-4H-[1]-benzopyrano[3,4-b]-pyridinhydrochlorid, Fp. 210-211°,

h) trans-8-Ethyl-10-methoxy-4-propyl-1,2,3,4a,5,10b-hexahydro-4H-[1]-benzopyrano[3,4-b]-pyridinhydrochlorid, Fp. 231-233°,

i) trans-7-Brom-10-methoxy-4-propyl-1,2,3,4a,5,10b-hexahydro-4H-[1]-benzopyrano[3,4-b]-pyridinhydrochlorid, Fp. 267-270°,

j) trans-10-Ethoxy-4-propyl-1,2,3,4a,5,10b-hexahydro-4H-[1]-benzopyrano[3,4-b]pyridinhydrochlorid, Fp. 247-250°,

k) trans-10-Benzyloxy-4-propyl-1,2,3,4a,5,10b-hexahydro-4H-[1]-benzopyrano[3,4-b]pyridinhydrochlorid, Fp. 184-185°,

l) trans-10-Benzyloxy-7-brom-4-propyl-1,2,3,4a,5,10b-hexahydro-4H-[1]-benzopyrano[3,4-b]-pyridinhydrochlorid, Fp. 210-212°,

m) trans-7-Methoxy-8-methyl-4-propyl-1,2,3,4a,5,10b-hexahydro-4H-[1]-benzopyrano[3,4-b]-pyridinhydrochlorid, Fp. 243-246°,

n) trans-7-Hydroxy-4,10-dimethyl-1,2,3,4a,5,10b-hexahydro-4H-[1]-benzopyrano[3,4-b]pyridinhydrochlorid, Fp. 252-254°.

Beispiel 31:

Zu einer Lösung von 2,61 g cis-9-Methoxy-4-propyl-1,2,3,4a,5,10b-hexahydro-4H-[1]-benzopyrano[3,4-b]pyridin in 30 ml trockenem Dimethylsulfoxid werden 2,0 g Kalium-tert.-butylat gegeben. Nach 2 Tagen bei Raumtemperatur wird der Ansatz auf Wasser gegeben, und das Produkt wird mit Ether extrahiert. Nach Trocknen über Magnesiumsulfat wird das Lösungsmittel im Vakuum abgezogen. Man erhält eine 1:1-Mischung der cis- und trans-Isomere. Ansäuern mit ethanolischem Chlorwasserstoff ergibt das trans-9-Methoxy-4-propyl-1,2,3,4a,5,10b-hexahydro-4H-[1]-benzopyrano[3,4-b]pyridinhydrochlorid, Fp. 252-254°.

Beispiel 32:

Ein Gemisch von 2,19 g trans-7-Methoxy-1,2,3,4a,5,10b-hexahydro-4H-[1]-benzopyrano[3,4-b]pyridin, 0,80 g Allylchlorid und 2,0 g Calciumcarbonat in 20 ml trockenem Dimethylformamid wird 16 Stunden auf 80° erhitzt. Nach Verdünnen mit Wasser wird das Produkt mit Ether extrahiert. Nach Trocknen über Magnesiumsulfat wird das Lösungsmittel im Vakuum abgezogen. Ansäuern mit ethanolischem Chlorwasserstoff ergibt das trans-4-Allyl-7-methoxy-1,2,3,4a,5,10b-hexahydro-4H-[1]-benzopyrano[3,4-b]-pyridinhydrochlorid.

Die Hydrierung dieser Verbindung in Gegenwart von 500 mg 10 % Palladium auf Kohle als Katalysator bei einem Druck von 300 kPa während 6 Stunden bei Raumtemperatur in Wasser ergibt trans-7-Methoxy-4-propyl-1,2,3,4a,5,10b-hexahydro-4H-[1]-benzoyprano[3,4-b]-pyridinhydrochlorid.

Beispiel 33:

Herstellung von 1000 Kapseln mit einem Gehalt von jeweils 10 mg der aktiven Substanz des Beispiels 5a mit folgender Zusammensetzung:

| | |
|---|---|
| trans-9-Hydroxy-4-propyl-1,2,3,4a,5,10b-hexahydro-4H-[1]-benzopyrano[3,4-b]pyridinhydrochlorid | 10,0 g |
| Milchzucker | 207,0 g |
| Modifizierte Stärke | 80,0 g |
| Magnesiumstearat | 3,0 g |

Verfahren:

Sämtliche pulverigen Bestandteile werden mit einem Sieb von 0,6 mm Maschenweite gesiebt. Dann wird der Wirkstoff in einen geeigneten Mixer gegeben und bis zur Homogenität zuerst mit Magnesiumstearat, dann mit Milchzucker und Stärke vermischt. No. 2 Gelatinekapseln werden mit je 300 mg dieser Mischung gefüllt, wobei man eine Kapselabfüllmaschine verwendet.

Auf analoge Weise werden Kapseln hergestellt, die 10-200 mg der anderen offenbarten und in den Beispielen erwähnten Verbindungen enthalten.

Beispiel 34:

Herstellung von 10'000 Tabletten mit einem Gehalt von je 10 mg der aktiven Substanz des Beispiels 7c mit folgender Zusammensetzung:

| | |
|---|---|
| trans-7-Hydroxy-4-propyl-1,2,3,4a,5,10b-hexahydro-4H-[1]-benzopyrano[3,4-b]pyridinhydrochlorid | 100,00 g |
| Milchzucker | 2,535,00 g |
| Maisstärke | 125,00 g |
| Polyethylenglykol 6000 | 150,00 g |
| Magnesiumstearat | 40,00 g |
| gereinigtes Wasser | q.s |

Verfahren:

Sämtlichen pulvrigen Bestandteile werden mit einem Sieb von 0,6 mm Maschenweite gesiebt. Dann wird der Wirkstoff mit Milchzucker, Magnesiumstearat und mit der Hälfte der Stärke in einem geeigneten

Mischer vermischt. Die andere Hälfte der Stärke wird in 65 ml Wasser suspendiert, und die Suspension wird zur siedenden Lösung von Polyethylenglykol in 260 ml Wasser gegeben. Die erhaltene Paste wird zu den Pulvern gegeben und, gegebenenfalls unter Zugabe einer weiteren Wassermenge, granuliert. Das Granulat wird über Nacht bei 35° getrocknet, durch ein Sieb von 1,2 mm Maschenweite getrieben und zu Tabletten gepresst, die eine Bruchrille aufweisen.

Auf analoge Weise werden Tabletten hergestellt, die 10-200 mg einer der anderen offenbarten und in den Beispielen erwähnten Verbindungen enthalten.

## Ansprüche

1. Verbindungen der Formel

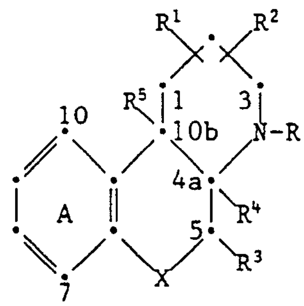

(I),

worin X Sauerstoff oder Schwefel bedeutet, Ring A unsubstituiert, durch einen bis zwei gleiche oder verschiedene Substituenten aus der Gruppe Hydroxy, Hydroxy-$C_1$-$C_4$-alkyl, $C_1$-$C_4$-Alkoxy, $C_2$-$C_4$-Alkenyloxy, $C_2$-$C_4$-Alkinyloxy, $C_3$-$C_6$-Cycloalkyl-$C_1$-$C_2$-alkoxy, Benzyloxy, $C_1$-$C_4$-Alkoxy-$C_1$-$C_4$-alkyl, $C_1$-$C_4$-Alkanoyloxy, Benzoyloxy, Benzolsulfonyloxy, 2-, 3- oder 4-Pyridylcarbonyloxy, $C_1$-$C_4$-Alkoxycarbonyloxy, Carbamoyloxy, Mono-oder Di-$C_1$-$C_4$-alkylcarbamoyloxy, $C_1$-$C_4$-Alkanoyloxy-$C_1$-$C_4$-alkyl, Halogen, $C_1$-$C_4$-Alkyl, Trifluormethyl, Amino, Mono- oder Di-$C_1$-$C_4$-alkylamino, $C_1$-$C_4$-Alkanoylamino, Benzoylamino und $C_1$-$C_4$-Alkoxycarbonylamino substituiert oder durch einen Substituenten $C_1$-$C_4$-Alkylendioxy substituiert ist, R Wasserstoff, $C_1$-$C_4$-Alkyl, Benzyl oder Phenethyl bedeutet, $R^1$ für Wasserstoff, $C_1$-$C_4$-Alkyl, $C_1$-$C_4$-Alkylthio-$C_1$-$C_4$-alkyl, Amino, $C_1$-$C_4$-Alkanoylamino, Benzoylamino, $C_1$-$C_4$-Alkoxycarbonylamino, (Amino-, Mono- oder Di-$C_1$-$C_4$-Alkylamino)-$C_1$-$C_4$-alkyl, Carboxy, $C_1$-$C_4$-Alkoxycarbonyl, Carbamoyl oder Mono-oder Di-$C_1$-$C_4$-alkylcarbamoyl steht und $R^2$ bis $R^5$ Wasserstoff oder $C_1$-$C_4$-Alkyl bedeuten, sowie die Dehydroderivate mit einer Doppelbindung in 1,2-Position oder in 1,10b-Position, wobei in letzterem Fall $R^5$ nicht vorhanden ist, und Salze von diesen Verbindungen.

2. Verbindungen der Formel I gemäss Anspruch 1, worin X Sauerstoff oder Schwefel bedeutet, Ring A unsubstituiert, durch einen bis zwei gleiche oder verschiedene Substituenten aus der Gruppe Hydroxy, $C_1$-$C_4$-Alkanoyloxy, Benzoyloxy, Nicotinoyloxy, $C_1$-$C_4$-Alkoxy, Hydroxymethyl, $C_1$-$C_4$-Alkyl, $C_1$-$C_4$-Alkanoyloxymethyl, $C_1$-$C_4$-Alkoxymethyl, Halogen und Trifluoromethyl substituiert oder durch einen Substituenten $C_1$-$C_4$-Alkylendioxy substituiert ist, R Wasserstoff oder $C_1$-$C_4$-Alkyl und $R^1$ bis $R^5$ Wasserstoff oder $C_1$-$C_4$-Alkyl bedeuten, deren 1,10b- und 1,2-Dehydroderivate sowie pharmazeutisch annehmbare Salze davon.

3. Verbindungen gemäss Anspruch 1 der Formel

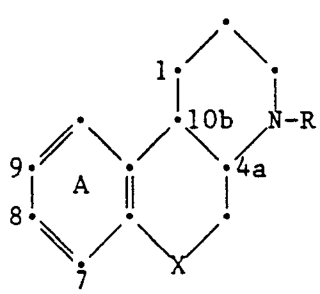

(II),

worin X Sauerstoff oder Schwefel bedeutet, Ring A unsubstituiert oder durch einen bis zwei gleiche oder verschiedene Substituenten aus der Gruppe Hydroxy, Hydroxymethyl, $C_1$-$C_4$-Alkanoyloxy, Benzoyloxy, Pyridylcarbonyloxy, $C_1$-$C_4$-Alkanoyloxymethyl, $C_1$-$C_4$-Alkyl, $C_1$-$C_4$-Alkoxy und Halogen substituiert ist und R $C_1$-$C_4$-Alkyl bedeutet, deren 1,10b-Dehydroderivate und pharmazeutisch annehmbare Salze davon.

**4.** Verbindungen der Formel II gemäss Anspruch 3, worin X Sauerstoff bedeutet, Ring A durch Hydroxy, $C_1$-$C_4$-Alkanoyloxy, Benzoyloxy oder Pyridylcarbonyloxy monosubstituiert ist und R $C_1$-$C_4$-Alkyl bedeutet, sowie pharmazeutisch annehmbare Salze davon.

**5.** Verbindungen gemäss Anspruch 1 der Formel

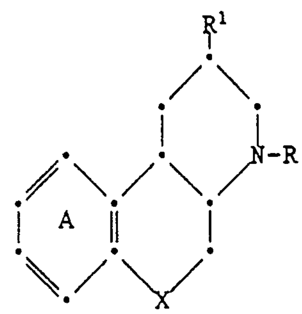

(III),

worin X Sauerstoff oder Schwefel bedeutet, Ring A unsubstituiert oder durch einen bis zwei gleiche oder verschiedene Substituenten aus der Gruppe Hydroxy, Hydroxymethyl, $C_1$-$C_4$-Alkanoyloxy, Benzoyloxy, Nicotinoyloxy, $C_1$-$C_4$-Alkanoyloxymethyl, $C_1$-$C_4$-Alkoxy und Halogen substituiert ist, R $C_1$-$C_4$-Alkyl bedeutet und $R^1$ für $C_1$-$C_4$-Alkylthio-$C_1$-$C_4$-alkyl, $C_1$-$C_4$-Alkanoylamino, (Amino, Mono- oder Di-$C_1$-$C_4$-alkylamino)-$C_1$-$C_4$-alkyl, Carboxy, $C_1$-$C_4$-Alkoxycarbonyl, Carbamoyl oder Mono- oder Di-$C_1$-$C_4$-Alkylcarbamoyl steht, und pharmazeutisch annehmbare Salze davon.

**6.** Verbindungen der Formel II gemäss Anspruch 3 mit einer 4a,10b-trans-Ringverknüpfung, in denen X Sauerstoff oder Schwefel bedeutet, R für $C_3$-$C_4$-Alkyl steht und Ring A in der 7-, 8- oder 9-Position durch Hydroxy, $C_1$-$C_4$-Alkanoyloxy, Benzoyloxy, Nicotinoyloxy, Hydroxymethyl oder $C_1$-$C_4$-Alkanoyloxymethyl monosubstituiert ist oder Ring A in 7,8-, 7,9- oder 8,9-Position disubstituiert ist, wobei ein Substituent Hydroxy, $C_1$-$C_4$-Alkanoyloxy, Benzoyloxy oder Nicotinoyloxy und der andere Hydroxy, $C_1$-$C_4$-Alkanoyloxy, Benzoyloxy, Nicotinoyloxy oder Halogen ist, deren 1,10b-Dehydroderivate und pharmazeutisch annehmbare Salze davon.

**7.** Verbindungen der Formel II gemäss Anspruch 3 mit 4a,10b-trans-Ringverknüpfung, in denen X Sauerstoff bedeutet, R für n-Propyl oder n-Butyl steht und Ring A in der 7-, 8- oder 9-Position durch Hydroxy, $C_1$-$C_4$-Alkanoyloxy, Benzoyloxy, Nicotinoyloxy, Hydroxymethyl oder $C_1$-$C_4$-Alkanoyloxymethyl substituiert ist, und pharmazeutisch annehmbare Salze davon.

**8.** Verbindungen der Formel II gemäss Anspruch 3 mit 4a,10b-trans-Ringverknüpfung, in denen X Sauerstoff bedeutet, R für n-Propyl oder n-Butyl steht und Ring A in der 7-, 8- oder 9-Position durch Hydroxy, $C_1$-$C_4$-Alkanoyloxy, Benzoyloxy oder Nicotinoyloxy substituiert ist, und pharmazeutisch annehmbare Salze davon.

**9.** Verbindungen der Formel II gemäss Anspruch 3 mit 4a,10b-trans-Ringverknüpfung, in denen X Sauerstoff bedeutet, R für Methyl, Ethyl oder n-Propyl steht, Ring A in der 7- oder 10-Position durch Hydroxy, $C_1$-$C_4$-Alkoxy, $C_1$-$C_4$-Alkanoyloxy, Benzoyloxy oder Nicotinoyloxy monosubstituiert ist oder Ring A in 7,8-, 7,10- oder 8,10-Position disubstituiert ist, wobei ein Substituent Hydroxy oder $C_1$-$C_4$-Alkoxy und der andere $C_1$-$C_4$-Alkyl oder Halogen ist, und pharmazeutisch annehmbare Salze davon.

**10.** Eine Verbindung gemäss Anspruch 1 aus der Gruppe trans-9-Hydroxy-4-propyl-1,2,3,4a,5,10b-hexahydro-4H-[1]-benzopyrano[3,4-b]pyridin, trans-7-Hydroxy-4-propyl-1,2,3,4a,5,10b-hexahydro-4H-[1]-benzopyrano-[3,4-b]pyridin und trans-10-Brom-7-methoxy-4-methyl-1,2,3,4a,5,10b-hexahydro-4H-[1]-

benzopyrano[3,4-b]pyridin, oder ein pharmazeutisch annehmbares Salz davon.

11. Eine Verbindung gemäss Anspruch 1 aus der Gruppe trans-10-Hydroxy-4-propyl-1,2,3,4a,5,10b-hexahydro-4H-[1]-benzopyrano[3,4-b]pyridin, trans-8-Ethyl-7-methoxy-4-methyl-1,2,3,4a,5,10b-hexahydro-4H-[1]-benzopyrano[3,4-b]pyridin, trans-7-Ethoxy-4,8-dimethyl-1,2,3,4a,5,10b-hexahydro-4H-[1]-benzopyrano[3,4-b]pyridin, trans-10-Ethoxy-4-propyl-1,2,3,4a,5,10b-hexahydro-4H-[1]-benzopyrano[3,4-b]pyridin und trans-7-Methoxy-4,10-dimethyl-1,2,3,4a,5,10b-hexahydro-4H-[1]-benzopyrano[3,4-b]-pyridin, oder ein pharmazeutisch annehmbares Salz davon.

12. trans-7-Hydroxy-4-propyl-1,2,3,4a,5,10b-hexahydro-4H-[1]-benzopyrano[3,4-b]pyridin gemäss Anspruch 1, oder ein pharmazeutisch annehmbares Salz davon.

13. trans-10-Methoxy-4-propyl-1,2,3,4a,5,10b-hexahydro-4H-[1]-benzopyrano[3,4-b]pyridin gemäss Anspruch 1, oder ein pharmazeutisch annehmbares Salz davon.

14. trans-7-Methoxy-4,10-dimethyl-1,2,3,4a,5,10b-hexahydro-4H-[1]-benzopyrano[3,4-b]pyridin gemäss Anspruch 1, oder ein pharmazeutisch annehmbares Salz davon.

15. trans-7-Hydroxy-4,10-dimethyl-1,2,3,4a,5,10b-hexahydro-4H-[1]-benzopyrano[3,4-b]pyridin gemäss Anspruch 1, oder ein pharmazeutisch annehmbares Salz davon.

16. Die in den Ansprüchen 1 bis 15 genannten Verbindungen zur Anwendung in einem Verfahren zur therapeutischen Behandlung des menschlichen oder tierischen Körpers.

17. Die in den Ansprüchen 1 bis 15 genannten Verbindungen als Neuroleptika oder Antidepressiva.

18. Pharmazeutische Präparate enthaltend Verbindungen der Ansprüche 1 bis 15 oder pharmazeutisch annehmbare Salze davon zusammen mit einem pharmazeutisch geeigneten Trägermaterial.

19. Verwendung der in den Ansprüchen 1 bis 15 genannten Verbindungen zur Herstellung von pharmazeutischen Präparaten.

20. Verfahren zur Herstellung von den in Anspruch 1 genannten Verbindungen der Formel I, in der alle Symbole die in Anspruch 1 angegebenen Bedeutungen haben, und deren Salzen, dadurch gekennzeichnet, dass man

a) eine Verbindung der Formel

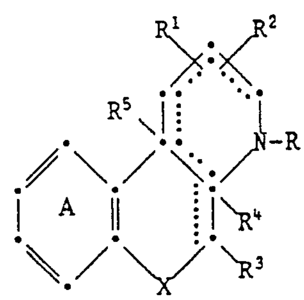

(IV),

in der gestrichelte Linien die Lage von einer, zwei oder drei nicht kumulierten Doppelbindungen angeben, X, eventuelle Substituenten von Ring A, R und $R^1$ bis $R^5$ wie vorstehend definiert sind, mit der Massgabe, dass $R^4$ und $R^5$ nur anwesend sind, wenn die Kohlenstoffatome, an die sie gebunden sind, nicht zu einer Doppelbindung gehören, mit einem geeigneten Reduktionsmittel reduziert, oder

b) eine Verbindung der Formel

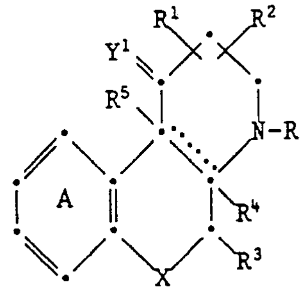

(V),

in der die gestrichelte Linie die Lage einer gegebenenfalls vorhandenen Doppelbindung angibt, X, eventuelle Substituenten von Ring A, R und $R^1$ bis $R^5$ wie vorstehend definiert sind, mit der Massgabe, dass $R^4$ und $R^5$ nur anwesend sind, wenn die Kohlenstoffatome, an die sie gebunden sind, nicht zu einer Doppelbindung gehören, und worin $Y^1$ Oxo oder geschütztes Oxo bedeutet oder worin $Y^1$ verethertes, verestertes oder freies Hydroxy zusammen mit Wasserstoff bedeutet, mit einem geeigneten Reduktionsmittel und/oder Eliminierungsreagens behandelt, oder

c) eine Verbindung der Formel

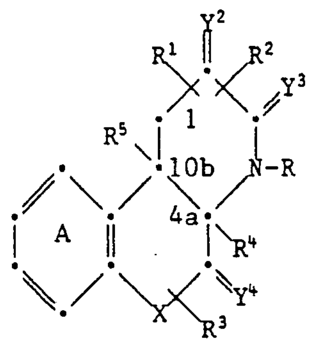

(VI),

worin X, eventuelle Substituenten von Ring A, R und $R^1$ bis $R^5$ wie vorstehend definiert sind, und worin einer der Reste $Y^2$, $Y^3$ und $Y^4$ Oxo oder geschütztes Oxo bedeutet, begleitet oder nicht begleitet von einer konjugierten endocyclischen Kohlenstoff-Kohlenstoff-Doppelbindung, oder für den Fall, dass $Y^3$ Oxo oder geschütztes Oxo bedeutet, auch begleitet von einer 1,10b- oder einer 4a,10b-Doppelbindung, und worin von den beiden verbleibenden Resten $Y^2$ bis $Y^4$, je nachdem, ob weitere Kohlenstoff-Kohlenstoff-Doppelbindungen vorhanden sind, $Y^2$ und $Y^3$ einen oder zwei Reste aus der Gruppe bestehend aus einem Rest $R^1$, einem Rest $R^2$ und Wasserstoff bedeuten, und/oder $Y^4$ für $R^3$ und Wasserstoff steht, mit einem geeigneten Reduktionsmittel reduziert, oder

d) in einer Verbindung der Formel

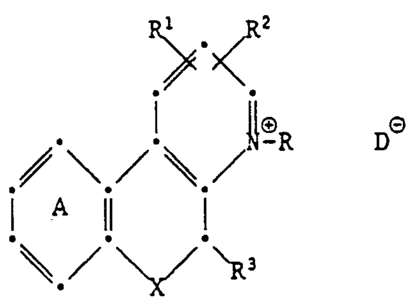

(VII),

worin X, eventuelle Substituenten von Ring A, R und $R^1$ bis $R^3$ wie vorstehend definiert sind und $D^{\ominus}$ das Anion einer organischen oder anorganischen Säure ist, den Pyridiniumring mit einem geeigneten Reduktionsmittel reduziert, oder

e) eine Verbindung der Formel

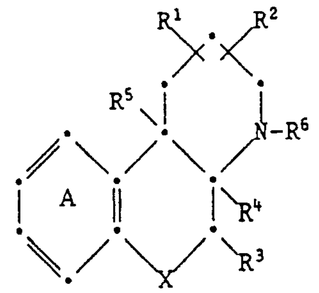

(VIII),

in der X, eventuelle Substituenten von Ring A und $R^1$ bis $R^5$ wie vorstehend definiert sind und $R^6$ $C_2$-$C_4$-Alkenyl, $C_2$-$C_4$-Alkinyl, $C_1$-$C_4$-Alkanoyl oder Benzoyl bedeutet, mit einem geeigneten Reduktionsmittel reduziert, oder

f) eine Verbindung der Formel

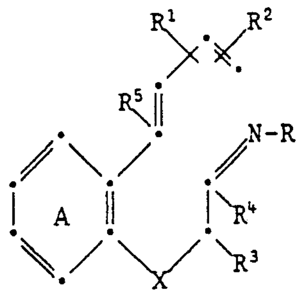

(IX),

in der X, eventuelle Substituenten von Ring A, R und $R^1$ bis $R^5$ wie vorstehend definiert sind, zu einer Verbindung der Formel

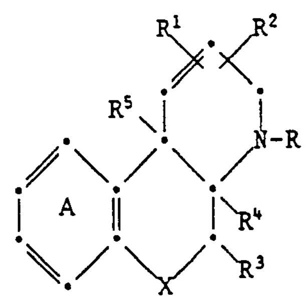

(Ib)

cyklisiert und das erhaltene Produkt gewünschtenfalls reduziert, oder

g) eine Verbindung der Formel

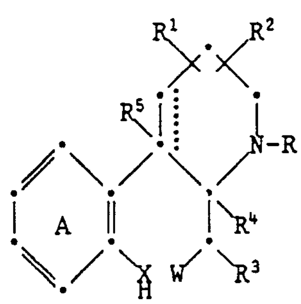

(X),

in der die gestrichelte Linie die Lage einer gegebenenfalls vorhandenen Doppelbindung angibt, X, eventuelle Substituenten von Ring A, R und $R^1$ bis $R^5$ wie vorstehend definiert sind, mit der

Massgabe, dass $R^5$ nur anwesend ist, wenn an der durch die gestrichelte Linie bezeichneten Position eine Einfachbindung vorliegt, und worin W für reaktives verestertes Hydroxy steht, cyclisiert, oder

h) eine Verbindung der Formel

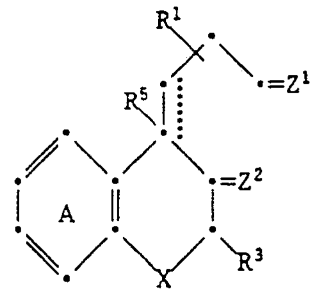

(XI),

in der die gestrichelte Linie die Lage einen gegebenenfalls vorhandenen Doppelbindung angibt, X, eventuelle Substituenten von Ring A, R und $R^1$ bis $R^5$ wie vorstehend definiert sind, und worin einer der Reste $Z^1$ und $Z^2$ Oxo oder reaktives verestertes Hydroxy zusammen mit Wasserstoff oder $C_1$-$C_4$-Alkyl bedeutet und der andere der beiden Reste $Z^1$ und $Z^2$ für -NHR zusammen mit Wasserstoff oder $C_1$-$C_4$-Alkyl steht, mit der Massgabe, dass $R^5$ nur anwesend ist, wenn an der durch die gestrichelte Linie bezeichneten Position eine Einfachbindung vorliegt, cyclisiert und das erhaltene Produkt gewünschtenfalls reduziert, oder

i) eine Verbindung der Formel

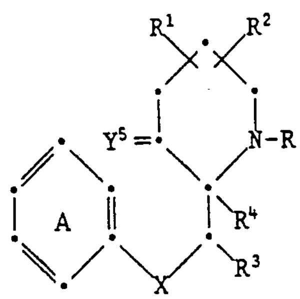

(XII),

in der X, eventuelle Substituenten von Ring A, R und $R^1$ bis $R^4$ wie vorstehend definiert sind, $Y^5$ für Oxo oder für reaktives verestertes Hydroxy zusammen mit Wasserstoff oder $C_1$-$C_4$-Alkyl steht, cyclisiert und das erhaltene Produkt gewünschtenfalls reduziert, oder

j) eine Verbindung der Formel

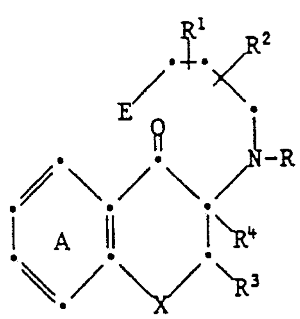

(XIII),

in der X, eventuelle Substituenten von Ring A, R und $R^1$ bis $R^4$ wie vorstehend definiert sind und E eine $\alpha$-Carbanionen stabilisierende Abgangsgruppe ist, cyclisiert und das erhaltene Produkt gewünschtenfalls reduziert,

und indem man diese Verfahren, wenn nötig, unter zwischenzeitlichem Schutz von reaktionsfähigen funktionellen Gruppen, deren Reaktivität sich störend auswirken würde, durchführt, und dann die

erhaltene Verbindung der Formel I oder die entsprechenden Dehydroderivate mit einer 1,2- oder 1,10b-Doppelbindung nach entsprechender Schutzgruppenabspaltung isoliert, und, wenn erwünscht, eine erhaltene Verbindung der Formel I oder das entsprechende Dehydroderivat mit einer 1,2- oder 1,10b-Doppelbindung in eine andere der vorstehend derfinierten Verbindungen umwandelt, und/oder, wenn erwünscht, eine erhaltene freie Verbindung in ein Salz oder ein erhaltenes Salz in die entsprechende freie Verbindung oder ein anderes Salz umwandelt, und/oder, wenn erwünscht, ein erhaltenes Gemisch von Isomeren oder Racematen in die einzelnen Isomere oder Racemate auftrennt, und/oder, wenn erwünscht, ein erhaltenes Racemat in die optischen Antipoden auftrennt.

Patentansprüche für folgenden Vertragsstaat: AT

1. Verfahren zur Herstellung von Verbindungen der Formel

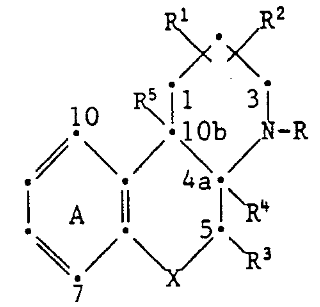

(I),

worin X Sauerstoff oder Schwefel bedeutet, Ring A unsubstituiert, durch einen bis zwei gleiche oder verschiedene Substituenten aus der Gruppe Hydroxy, Hydroxy-$C_1$-$C_4$-alkyl, $C_1$-$C_4$-Alkoxy, $C_2$-$C_4$-Alkenyloxy, $C_2$-$C_4$-Alkinyloxy, $C_3$-$C_6$-Cycloalkyl-$C_1$-$C_2$-alkoxy, Benzyloxy, $C_1$-$C_4$-Alkoxy-$C_1$-$C_4$-alkyl, $C_1$-$C_4$-Alkanoyloxy, Benzoyloxy, Benzolsulfonyloxy, 2-, 3- oder 4-Pyridylcarbonyloxy, $C_1$-$C_4$-Alkoxycarbonyloxy, Carbamoyloxy, Mono-oder Di-$C_1$-$C_4$-alkylcarbamoyloxy, $C_1$-$C_4$-Alkanoyloxy-$C_1$-$C_4$-alkyl, Halogen, $C_1$-$C_4$-Alkyl, Trifluormethyl, Amino, Mono- oder Di-$C_1$-$C_4$-alkylamino, $C_1$-$C_4$-Alkanoylamino, Benzoylamino und $C_1$-$C_4$-Alkoxycarbonylamino substituiert oder durch einen Substituenten $C_1$-$C_4$-Alkylendioxy substituiert ist, R Wasserstoff, $C_1$-$C_4$-Alkyl, Benzyl oder Phenethyl bedeutet, $R^1$ für Wasserstoff, $C_1$-$C_4$-Alkyl, $C_1$-$C_4$-Alkylthio-$C_1$-$C_4$-alkyl, Amino, $C_1$-$C_4$-Alkanoylamino, Benzoylamino, $C_1$-$C_4$-Alkoxycarbonylamino, (Amino-, Mono- oder Di-$C_1$-$C_4$-Alkylamino)-$C_1$-$C_4$-alkyl, Carboxy, $C_1$-$C_4$-Alkoxycarbonyl, Carbamoyl oder Mono-oder Di-$C_1$-$C_4$-alkylcarbamoyl steht und $R^2$ bis $R^5$ Wasserstoff oder $C_1$-$C_4$-Alkyl bedeuten, deren Dehydroderivaten mit einer Doppelbindung in 1,2-Position oder in 1,10b-Position, wobei in letzterem Fall $R^5$ nicht vorhanden ist, und von Salzen dieser Verbindungen, dadurch gekennzeichnet, dass man

a) eine Verbindung der Formel

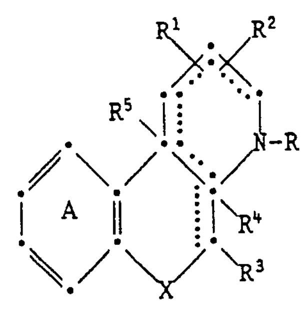

(IV),

in der gestrichelte Linien die Lage von einer, zwei oder drei nicht kumulierten Doppelbindungen angeben, X, eventuelle Substituenten von Ring A, R und $R^1$ bis $R^5$ wie vorstehend definiert sind, mit der Massgabe, dass $R^4$ und $R^5$ nur anwesend sind, wenn die Kohlenstoffatome, an die sie gebunden sind, nicht zu einer Doppelbindung gehören, mit einem geeigneten Reduktionsmittel reduziert, oder

b) eine Verbindung der Formel

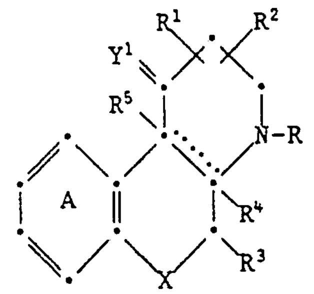

(V),

in der die gestrichelte Linie die Lage einer gegebenenfalls vorhandenen Doppelbindung angibt, X, eventuelle Substituenten von Ring A, R und $R^1$ bis $R^5$ wie vorstehend definiert sind, mit der Massgabe, dass $R^4$ und $R^5$ nur anwesend sind, wenn die Kohlenstoffatome, an die sie gebunden sind, nicht zu einer Doppelbindung gehören, und worin $Y^1$ Oxo oder geschütztes Oxo bedeutet oder worin $Y^1$ verethertes, verestertes oder freies Hydroxy zusammen mit Wasserstoff bedeutet, mit einem geeigneten Reduktionsmittel und/oder Eliminierungsreagens behandelt, oder

c) eine Verbindung der Formel

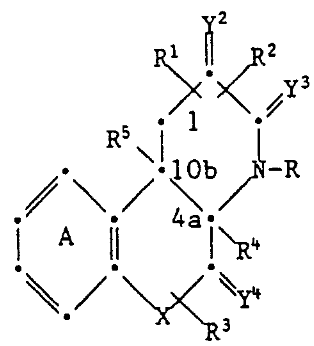

(VI),

worin X, eventuelle Substituenten von Ring A, R und $R^1$ bis $R^5$ wie vorstehend definiert sind, und worin einer der Reste $Y^2$, $Y^3$ und $Y^4$ Oxo oder geschütztes Oxo bedeutet, begleitet oder nicht begleitet von einer konjugierten endocyclischen Kohlenstoff-Kohlenstoff-Doppelbindung, oder für den Fall, dass $Y^3$ Oxo oder geschütztes Oxo bedeutet, auch begleitet von einer 1,10b- oder einer 4a,10b-Doppelbindung, und worin von den beiden verbleibenden Resten $Y^2$ bis $Y^4$, je nachdem, ob weitere Kohlenstoff-Kohlenstoff-Doppelbindungen vorhanden sind, $Y^2$ und $Y^3$ einen oder zwei Reste aus der Gruppe bestehend aus einem Rest $R^1$, einem Rest $R^2$ und Wasserstoff bedeuten, und/oder $Y^4$ für $R^3$ und Wasserstoff steht, mit einem geeigneten Reduktionsmittel reduziert, oder

d) in einer Verbindung der Formel

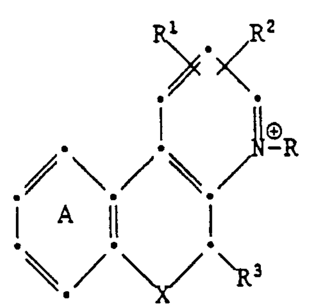

$D^{\ominus}$ (VII),

worin X, eventuelle Substituenten von Ring A, R und $R^1$ bis $R^3$ wie vorstehend definiert sind und $D^{\ominus}$ das Anion einer organischen oder anorganischen Säure ist, den Pyridiniumring mit einem geeigneten Reduktionsmittel reduziert, oder

e) eine Verbindung der Formel

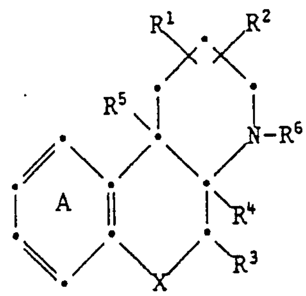

(VIII),

in der X, eventuelle Substituenten von Ring A und $R^1$ bis $R^5$ wie vorstehend definiert sind und $R^6$ $C_2$-$C_4$-Alkenyl, $C_2$-$C_4$-Alkinyl, $C_1$-$C_4$-Alkanoyl oder Benzoyl bedeutet, mit einem geeigneten Reduktionsmittel reduziert, oder

f) eine Verbindung der Formel

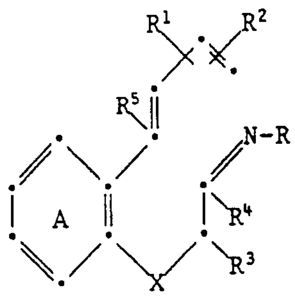

(IX),

in der X, eventuelle Substituenten von Ring A, R und $R^1$ bis $R^5$ wie vorstehend definiert sind, zu einer Verbindung der Formel

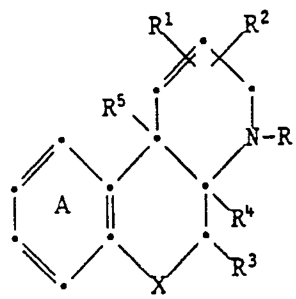

(Ib)

cyklisiert und das erhaltene Produkt gewünschtenfalls reduziert, oder

g) eine Verbindung der Formel

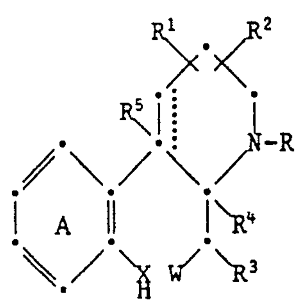

(X),

in der die gestrichelte Linie die Lage einer gegebenenfalls vorhandenen Doppelbindung angibt, X, eventuelle Substituenten von Ring A, R und $R^1$ bis $R^5$ wie vorstehend definiert sind, mit der Massgabe, dass $R^5$ nur anwesend ist, wenn an der durch die gestrichelte Linie bezeichneten

Position eine Einfachbindung vorliegt, und worin W für reaktives verestertes Hydroxy steht, cyclisiert, oder

h) eine Verbindung der Formel

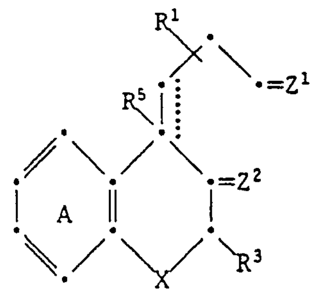

(XI),

in der die gestrichelte Linie die Lage einen gegebenenfalls vorhandenen Doppelbindung angibt, X, eventuelle Substituenten von Ring A, R und $R^1$ bis $R^5$ wie vorstehend definiert sind, und worin einer der Reste $Z^1$ und $Z^2$ Oxo oder reaktives verestertes Hydroxy zusammen mit Wasserstoff oder $C_1$-$C_4$-Alkyl bedeutet und der andere der beiden Reste $Z^1$ und $Z^2$ für -NHR zusammen mit Wasserstoff oder $C_1$-$C_4$-Alkyl steht, mit der Massgabe, dass $R^5$ nur anwesend ist, wenn an der durch die gestrichelte Linie bezeichneten Position eine Einfachbindung vorliegt, cyclisiert und das erhaltene Produkt gewünschtenfalls reduziert, oder

i) eine Verbindung der Formel

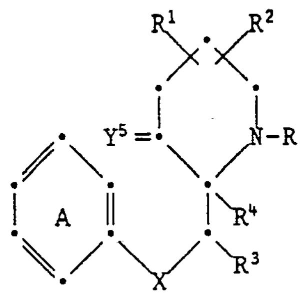

(XII),

in der X, eventuelle Substituenten von Ring A, R und $R^1$ bis $R^4$ wie vorstehend definiert sind, $Y^5$ für Oxo oder für reaktives verestertes Hydroxy zusammen mit Wasserstoff oder $C_1$-$C_4$-Alkyl steht, cyclisiert und das erhaltene Produkt gewünschtenfalls reduziert, oder

j) eine Verbindung der Formel

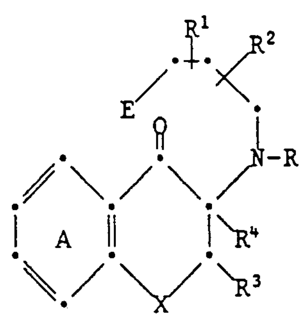

(XIII),

in der X, eventuelle Substituenten von Ring A, R und $R^1$ bis $R^4$ wie vorstehend definiert sind und E eine α-Carbanionen stabilisierende Abgangsgruppe ist, cyclisiert und das erhaltene Produkt gewünschtenfalls reduziert,

und indem man diese Verfahren, wenn nötig, unter zwischenzeitlichem Schutz von reaktionsfähigen funktionellen Gruppen, deren Reaktivität sich störend auswirken würde, durchführt, und dann die

erhaltene Verbindung der Formel I oder die entsprechenden Dehydroderivate mit einer 1,2- oder 1,10b-Doppelbindung nach entsprechender Schutzgruppenabspaltung isoliert, und, wenn erwünscht, eine erhaltene Verbindung der Formel I oder das entsprechende Dehydroderivat mit einer 1,2- oder 1,10b-Doppelbindung in eine andere der vorstehend derfinierten Verbindungen umwandelt, und/oder, wenn erwünscht, eine erhaltene freie Verbindung in ein Salz oder ein erhaltenes Salz in die entsprechende freie Verbindung oder ein anderes Salz umwandelt, und/oder, wenn erwünscht, ein erhaltenes Gemisch von Isomeren oder Racematen in die einzelnen Isomere oder Racemate auftrennt, und/oder, wenn erwünscht, ein erhaltenes Racemat in die optischen Antipoden auftrennt.

2. Verfahren gemäss Anspruch 1 zur Herstellung von Verbindungen der Formel I, worin X Sauerstoff oder Schwefel bedeutet, Ring A unsubstituiert, durch einen bis zwei gleiche oder verschiedene Substituenten aus der Gruppe Hydroxy, $C_1$-$C_4$-Alkanoyloxy, Benzoyloxy, Nicotinoyloxy, $C_1$-$C_4$-Alkoxy, Hydroxymethyl, $C_1$-$C_4$-Alkyl, $C_1$-$C_4$-Alkanoyloxymethyl, $C_1$-$C_4$-Alkoxymethyl, Halogen und Trifluoromethyl substituiert oder durch einen Substituenten $C_1$-$C_4$-Alkylendioxy substituiert ist, R Wasserstoff oder $C_1$-$C_4$-Alkyl und $R^1$ bis $R^5$ Wasserstoff oder $C_1$-$C_4$-Alkyl bedeuten, deren 1,10b- und 1,2-Dehydroderivaten sowie von pharmazeutisch annehmbaren Salzen dieser Verbindungen.

3. Verfahren gemäss Anspruch 1 zur Herstellung von Verbindungen der Formel

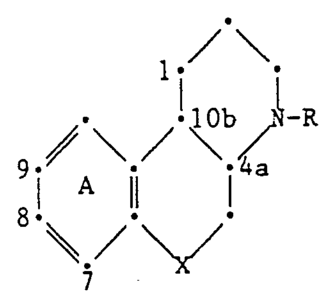 (II),

worin X Sauerstoff oder Schwefel bedeutet, Ring A unsubstituiert oder durch einen bis zwei gleiche oder verschiedene Substituenten aus der Gruppe Hydroxy, Hydroxymethyl, $C_1$-$C_4$-Alkanoyloxy, Benzoyloxy, Pyridylcarbonyloxy, $C_1$-$C_4$-Alkanoyloxymethyl, $C_1$-$C_4$-Alkyl, $C_1$-$C_4$-Alkoxy und Halogen substituiert ist und R $C_1$-$C_4$-Alkyl bedeutet, deren 1,10b-Dehydroderivaten sowie von pharmazeutisch annehmbaren Salzen dieser Verbindungen.

4. Verfahren gemäss Anspruch 3 zur Herstellung von Verbindungen der Formel II, worin X Sauerstoff bedeutet, Ring A durch Hydroxy, $C_1$-$C_4$-Alkanoyloxy, Benzoyloxy oder Pyridylcarbonyloxy monosubstituiert ist und R $C_1$-$C_4$-Alkyl bedeutet, sowie von pharmazeutisch annehmbaren Salzen dieser Verbindungen.

5. Verfahren gemäss Anspruch 1 zur Herstellung von Verbindungen der Formel

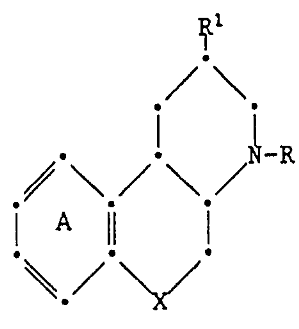 (III),

worin X Sauerstoff oder Schwefel bedeutet, Ring A unsubstituiert oder durch einen bis zwei gleiche oder verschiedene Substituenten aus der Gruppe Hydroxy, Hydroxymethyl, $C_1$-$C_4$-Alkanoyloxy, Benzoyloxy, Nicotinoyloxy, $C_1$-$C_4$-Alkanoyloxymethyl, $C_1$-$C_4$-Alkoxy und Halogen substituiert ist, R $C_1$-$C_4$-

Alkyl bedeutet und $R^1$ für $C_1$-$C_4$-Alkylthio-$C_1$-$C_4$-alkyl, $C_1$-$C_4$-Alkanoylamino, (Amino, Mono- oder Di-$C_1$-$C_4$-alkylamino)-$C_1$-$C_4$-alkyl, Carboxy, $C_1$-$C_4$-Alkoxycarbonyl, Carbamoyl oder Mono- oder Di-$C_1$-$C_4$-Alkylcarbamoyl steht, sowie von pharmazeutisch annehmbaren Salzen dieser Verbindungen.

6. Verfahren gemäss Anspruch 3 zur Herstellung von Verbindungen der Formel II mit einer 4a,10b-trans-Ringverknüpfung, in denen X Sauerstoff oder Schwefel bedeutet, R für $C_3$-$C_4$-Alkyl steht und Ring A in der 7-, 8- oder 9-Position durch Hydroxy, $C_1$-$C_4$-Alkanoyloxy, Benzoyloxy, Nicotinoyloxy, Hydroxymethyl oder $C_1$-$C_4$-Alkanoyloxymethyl monosubstituiert ist oder Ring A in 7,8-, 7,9-oder 8,9-Position disubstituiert ist, wobei ein Substituent Hydroxy, $C_1$-$C_4$-Alkanoyloxy, Benzoyloxy oder Nicotinoyloxy und der andere Hydroxy, $C_1$-$C_4$-Alkanoyloxy, Benzoyloxy, Nicotinoyloxy oder Halogen ist, deren 1,10b-Dehydroderivaten sowie von pharmazeutisch annehmbaren Salzen dieser Verbindungen.

7. Verfahren gemäss Anspruch 3 zur Herstellung von Verbindungen der Formel II mit 4a,10b-trans-Ringverknüpfung, in denen X Sauerstoff bedeutet, R für n-Propyl oder n-Butyl steht und Ring A in der 7-, 8- oder 9-Position durch Hydroxy, $C_1$-$C_4$-Alkanoyloxy, Benzoyloxy, Nicotinoyloxy, Hydroxymethyl oder $C_1$-$C_4$-Alkanoyloxymethyl substituiert ist, sowie von pharmazeutisch annehmbaren Salzen dieser Verbindungen.

8. Verfahren gemäss Anspruch 3 zur Herstellung von Verbindungen der Formel II mit 4a,10b-trans-Ringverknüpfung, in denen X Sauerstoff bedeutet, R für n-Propyl oder n-Butyl steht und Ring A in der 7-, 8- oder 9-Position durch Hydroxy, $C_1$-$C_4$-Alkanoyloxy, Benzoyloxy oder Nicotinoyloxy substituiert ist, sowie von pharmazeutisch annehmbaren Salzen dieser Verbindungen.

9. Verfahren gemäss Anspruch 3 zur Herstellung von Verbindungen der Formel II mit 4a,10b-trans-Ringverknüpfung, in denen X Sauerstoff bedeutet, R für Methyl, Ethyl oder n-Propyl steht, Ring A in der 7- oder 10-Position durch Hydroxy, $C_1$-$C_4$-Alkoxy, $C_1$-$C_4$-Alkanoyloxy, Benzoyloxy oder Nicotinoyloxy monosubstituiert ist oder Ring A in 7,8-, 7,10- oder 8,10-Position disubstituiert ist, wobei ein Substituent Hydroxy oder $C_1$-$C_4$-Alkoxy und der andere $C_1$-$C_4$-Alkyl oder Halogen ist, sowie von pharmazeutisch annehmbaren Salzen dieser Verbindungen.

10. Verfahren gemäss Anspruch 1 zur Herstellung von Verbindungen aus der Gruppe trans-9-Hydroxy-4-propyl-1,2,3,4a,5,10b-hexahydro-4H-[1]-benzopyrano[3,4-b]pyridin, trans-7-Hydroxy-4-propyl-1,2,3,4a,5,10b-hexahydro-4H-[1]-benzopyrano[3,4-b]pyridin und trans-10-Brom-7-methoxy-4-methyl-1,2,3,4a,5,10b-hexahydro-4H-[1]-benzopyrano[3,4-b]pyridin, sowie von pharmazeutisch annehmbaren Salzen dieser Verbindungen.

11. Verfahren gemäss Anspruch 1 zur Herstellung von Verbindungen aus der Gruppe trans-10-Hydroxy-4-propyl-1,2,3,4a,5,10b-hexahydro-4H-[1]-benzopyrano[3,4-b]pyridin, trans-8-Ethyl-7-methoxy-4-methyl-1,2,3,4a,5,10b-hexahydro-4H-[1]-benzopyrano[3,4-b]pyridin, trans-7-Ethoxy-4,8-dimethyl-1,2,3,4a,5,10b-hexahydro-4H-[1]-benzopyrano[3,4-b]pyridin, trans-10-Ethoxy-4-propyl-1,2,3,4a,5,10b-hexahydro-4H-[1]-benzopyrano[3,4-b]pyridin und trans-7-Methoxy-4,10-dimethyl-1,2,3,4a,5,10b-hexahydro-4H-[1]-benzopyrano[3,4-b]pyridin, sowie von pharmazeutisch annehmbaren Salzen dieser Verbindungen.

12. Verfahren gemäss Anspruch 1 zur Herstellung von trans-7-Hydroxy-4-propyl-1,2,3,4a,5,10b-hexahydro-4H-[1]-benzopyrano[3,4-b]pyridin, oder von pharmazeutisch annehmbaren Salzen dieser Verbindung.

13. Verfahren gemäss Anspruch 1 zur Herstellung von trans-10-Methoxy-4-propyl-1,2,3,4a,5,10b-hexahydro-4H-[1]-benzopyrano[3,4-b]pyridin, oder von pharmazeutisch annehmbaren Salzen dieser Verbindung.

14. Verfahren gemäss Anspruch 1 zur Herstellung von trans-7-Methoxy-4,10-dimethyl-1,2,3,4a,5,10b-hexahydro-4H-[1]-benzopyrano[3,4-b]pyridin, oder von pharmazeutisch annehmbaren Salzen dieser Verbindung.

15. Verfahren gemäss Anspruch 1 zur Herstellung von trans-7-Hydroxy-4,10-dimethyl-1,2,3,4a,5,10b-hexahydro-4H-[1]-benzopyrano[3,4-b]pyridin, oder von pharmazeutisch annehmbaren Salzen dieser Verbindung.

16. Verfahren zur Herstellung von pharmazeutischen Präparaten enthaltend nach Ansprüchen 1 bis 15 herstellbare Verbindungen oder pharmazeutisch annehmbare Salze davon, dadurch gekennzeichnet, dass man diese Verbindungen oder deren Salze mit einem pharmazeutisch geeigneten Trägermaterial mischt.

## Claims

1. A compound of formula

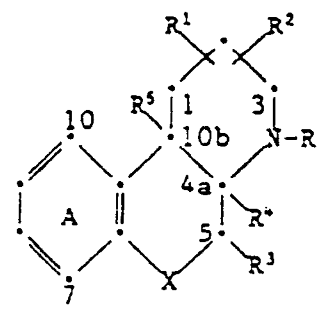

(I),

wherein X represents oxygen or sulfur, ring A is unsubstituted or substituted by one or two identical or different substituents from the group hydroxy, hydroxy-$C_1$-$C_4$alkyl, $C_1$-$C_4$alkoxy, $C_2$-$C_4$alkenyloxy, $C_2$-$C_4$alkynyloxy, $C_3$-$C_6$cycloalkyl-$C_1$-$C_2$alkoxy, benzyloxy, $C_1$-$C_4$alkoxy-$C_1$-$C_4$alkyl, $C_1$-$C_4$alkanoyloxy, benzoyloxy, benzenesulfonyloxy, 2-, 3- or 4-pyridylcarbonyloxy, $C_1$-$C_4$alkoxycarbonyloxy, carbamoyloxy, mono- or di-$C_1$-$C_4$alkylcarbamoyloxy, $C_1$-$C_4$alkanoyloxy-$C_1$-$C_4$alkyl, halogen, $C_1$-$C_4$alkyl, trifluoromethyl, amino, mono- or di-$C_1$-$C_4$alkylamino, $C_1$-$C_4$alkanoylamino, benzoylamino and $C_1$-$C_4$alkoxycarbonylamino, or by one substituent $C_1$-$C_4$-alkylenedioxy, R represents hydrogen, $C_1$-$C_4$alkyl, benzyl or phenethyl, $R^1$ represents hydrogen, $C_1$-$C_4$alkyl, $C_1$-$C_4$alkylthio-$C_1$-$C_4$alkyl, amino, $C_1$-$C_4$-alkanoylamino, benzoylamino, $C_1$-$C_4$alkoxycarbonylamino, (amino, mono- or di-$C_1$-$C_4$alkylamino)-$C_1$-$C_4$alkyl, carboxy, $C_1$-$C_4$alkoxycarbonyl, carbamoyl or mono- or di-$C_1$-$C_4$alkylcarbamoyl, and $R^2$ to $R^5$ represent hydrogen or $C_1$-$C_4$alkyl, as well as the dehydro derivatives thereof having a double bond at the 1,2-position, or at the 1,10b-position in which case $R^5$ is absent, or a salt of such a compound.

2. A compound of formula I according to claim 1, wherein X represents oxygen or sulfur, ring A is unsubstituted or substituted by one or two identical or different substituents from the group hydroxy, $C_1$-$C_4$alkanoyloxy, benzoyloxy, nicotinoyloxy, $C_1$-$C_4$alkoxy, hydroxymethyl, $C_1$-$C_4$alkyl, $C_1$-$C_4$alkanoyloxymethyl, $C_1$-$C_4$alkoxymethyl, halogen and trifluoromethyl, or by one substituent $C_1$-$C_4$alkylenedioxy, R represents hydrogen or $C_1$-$C_4$alkyl, and $R^1$ to $R^5$ represent hydrogen or $C_1$-$C_4$alkyl, the 1,10b- and 1,2-dehydro derivatives thereof, or a pharmaceutically acceptable salt thereof.

3. A compound according to claim 1 of formula

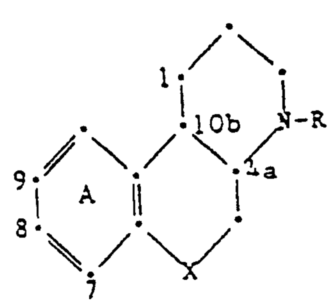

(II),

wherein X represents oxygen or sulfur, ring A is unsubstituted or substituted by one or two identical or different substituents from the group hydroxy, hydroxymethyl, $C_1$-$C_4$alkanoyloxy, benzoyloxy, pyridylcarbonyloxy, $C_1$-$C_4$alkanoyloxymethyl, $C_1$-$C_4$alkyl, $C_1$-$C_4$alkoxy and halogen, and R represents $C_1$-$C_4$alkyl, the 1,10b-dehydro derivative thereof, or a pharmaceutically acceptable salt thereof.

4. A compound of formula II according to claim 3, wherein X represents oxygen, ring A is monosubstituted by hydroxy, $C_1$-$C_4$alkanoyloxy, benzoyloxy or pyridylcarbonyloxy and R represents $C_1$-$C_4$alkyl, or a pharmaceutically acceptable salt thereof.

5. A compound according to claim 1 of formula

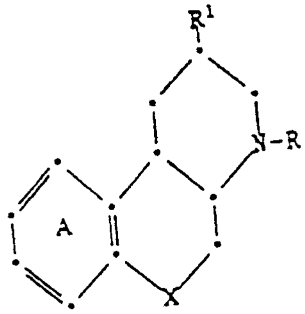

(III),

wherein X represents oxygen or sulfur, ring A is unsubstituted or substituted by one or two identical or different substituents from the group hydroxy, hydroxymethyl, $C_1$-$C_4$alkanoyloxy, benzoyloxy, nicotinoyloxy, $C_1$-$C_4$alkanoyloxymethyl, $C_1$-$C_4$alkoxy and halogen, R represents $C_1$-$C_4$alkyl, and $R^1$ represents $C_1$-$C_4$alkylthio-$C_1$-$C_4$alkyl, $C_1$-$C_4$alkanoylamino, (amino, mono- or di-$C_1$-$C_4$alkylamino)-$C_1$-$C_4$alkyl, carboxy, $C_1$-$C_4$alkoxycarbonyl, carbamoyl, mono- or di-$C_1$-$C_4$alkylcarbamoyl, or a pharmaceutically acceptable salt thereof.

6. A compound of formula II according to claim 3 with a 4a,10b-trans ring linkage, in which X represents oxygen or sulfur, R represents $C_3$-$C_4$alkyl and ring A is monosubstituted in the 7-, 8- or 9-position by hydroxy, $C_1$-$C_4$alkanoyloxy, benzoyloxy, nicotinoyloxy, hydroxymethyl or $C_1$-$C_4$alkanoyloxymethyl, or ring A is disubstituted in the 7,8-, 7,9- or 8,9-position, one substituent being hydroxy, $C_1$-$C_4$alkanoyloxy, benzoyloxy or nicotinoyloxy and the other hydroxy, $C_1$-$C_4$alkanoyloxy, benzoyloxy, nicotinoyloxy or halogen, the 1,10b-dehydro derivative thereof, or a pharmaceutically acceptable salt thereof.

7. A compound of formula II according to claim 3 with 4a,10b-trans ring linkage, in which X represents oxygen, R represents n-propyl or n-butyl and ring A is substituted in the 7-, 8- or 9-position by hydroxy, $C_1$-$C_4$alkanoyloxy, benzoyloxy, nicotinoyloxy, hydroxymethyl or $C_1$-$C_4$alkanoyloxymethyl, or a pharmaceutically acceptable salt thereof.

8. A compound of formula II according to claim 3 with 4a,10b-trans ring linkage, in which X represents oxygen, R represents n-propyl or n-butyl and ring A is substituted in the 7-, 8- or 9-position by hydroxy, $C_1$-$C_4$alkanoyloxy, benzoyloxy or nicotinoyloxy, or a pharmaceutically acceptable salt thereof.

9. A compound of formula II according to claim 3 with 4a,10b-trans ring linkage, in which X represents oxygen, R represents methyl, ethyl or n-propyl, ring A is monosubstituted in the 7- or 10-position by hydroxy, $C_1$-$C_4$alkoxy, $C_1$-$C_4$alkanoyloxy, benzoyloxy or nicotinoyloxy, or ring A is disubstituted in the 7,8-, 7,10- or 8,10-position, one substituent being hydroxy or $C_1$-$C_4$alkoxy and the other $C_1$-$C_4$alkyl or halogen, or a pharmaceutically acceptable salt thereof.

10. A compound according to claim 1 from the group trans-9-hydroxy-4-propyl-1,2,3,4a,5,10b-hexahydro-4H-[1]-benzopyrano[3,4-b]pyridine, trans-7-hydroxy-4-propyl-1,2,3,4a,5,10b-hexahydro-4H-[1]-benzopyrano[3,4-b]-pyridine and trans-10-bromo-7-methoxy-4-methyl-1,2,3,4a,5,10b-hexahydro-4H-[1]-benzopyrano[3,4-b]-pyridine, or a pharmaceutically acceptable salt thereof.

11. A compound according to claim 1 from the group trans-10-hydroxy-4-propyl-1,2,3,4a,5,10b-hexahydro-4H-[1]-benzopyrano[3,4-b]pyridine, trans-8-ethyl-7-methoxy-4-methyl-1,2,3,4a,5,10b-hexahydro-4H-[1]-benzopyrano-[3,4-b]pyridine, trans-7-ethoxy-4,8-dimethyl-1,2,3,4a,5,10b-hexahydro-4H-[1]-benzopyrano-[3,4-b]-pyridine, trans-10-ethoxy-4-propyl-1,2,3,4a,5,10b-hexahydro-4H-[1]-benzopyrano[3,4-b]pyridine and trans-7-methoxy-4,10-dimethyl-1,2,3,4a,5,10b-hexahydro-4H-[1]-benzopyrano[3,4-b]-pyridine, or a pharmaceutically acceptable salt thereof.

12. trans-7-Hydroxy-4-propyl-1,2,3,4a,5,10b-hexahydro-4H-[1]-benzopyrano[3,4-b]pyridine according to claim 1, or a pharmaceutically acceptable salt thereof.

13. trans-10-Methoxy-4-propyl-1,2,3,4a,5,10b-hexahydro-4H-[1]-benzopyrano[3,4-b]pyridine according to claim 1, or a pharmaceutically acceptable salt thereof.

14. trans-7-Methoxy-4,10-dimethyl-1,2,3,4a,5,10b-hexahydro-4H-[1]-benzopyrano[3,4-b]pyridine according to claim 1, or a pharmaceutically acceptable salt thereof.

15. trans-7-Hydroxy-4,10-dimethyl-1,2,3,4a,5,10b-hexahydro-4H-[1]-benzopyrano[3,4-b]pyridine according to claim 1, or a pharmaceutically acceptable salt thereof.

16. A compound defined in any one of claims 1 to 15 for use in a method for the therapeutic treatment of the human or animal body.

17. A compound defined in any one of claims 1 to 15 as a neuroleptic or antidepressive.

18. A pharmaceutical preparation comprising a compound according to any one of claims 1 to 15 or a pharmaceutically acceptable salt thereof together with a pharmaceutically acceptable carrier.

19. The use of a compound defined in any one of claims 1 to 15 for the manufacture of a pharmaceutical preparation.

20. A process for the manufacture of a compound of formula I defined in claim 1, in which all the symbols have the meanings given in claim 1, or a salt thereof, which comprises
a) reducing with a suitable reducing agent a compound of formula

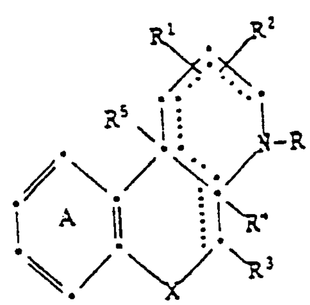

(IV),

wherein the dotted lines indicate the position of one, two or three non-cumulated double bonds, and X, optional substituents on ring A, R and $R^1$ to $R^5$ are as defined above provided that $R^4$ and $R^5$ are present only if the carbon atoms to which they are attached are not part of a double bond, or
b) reacting with a suitable reducing agent and/or elimination reagent a compound of formula

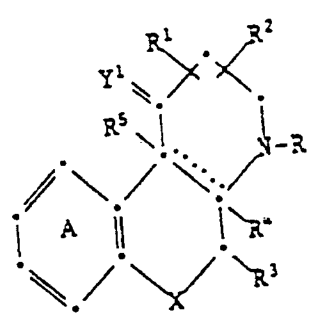

(V),

wherein the dotted line indicates the position of an optionally present double bond, X, optional substituents on ring A, R and $R^1$ to $R^5$ are as defined above provided that $R^4$ and $R^5$ are present only if the carbon atoms to which they are attached are not part of a double bond, and $Y'$ is oxo or

protected oxo, or $Y^1$ represents etherified, esterified or free hydroxy together with hydrogen, or
c) reducing with a suitable reducing agent a compound of formula

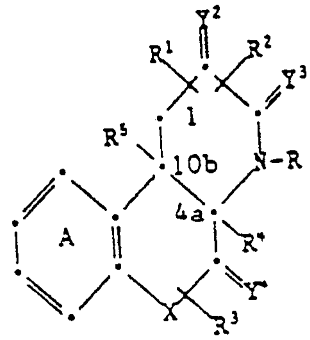

(VI),

wherein X, optional substituents on ring A, R and $R^1$ to $R^5$ are as defined above and one of the radicals $Y^2$, $Y^3$ and $Y^4$ is oxo or protected oxo, accompanied or not accompanied by a conjugated endocyclic carbon-carbon double bond, or in the case where $Y^3$ is oxo or protected oxo, also accompanied by a 1,10b- or a 4a,10b-double bond, and wherein of the two remaining radicals of $Y^2$ to $Y^4$, depending on whether additional carbon-carbon double bonds are present, $Y^2$ and $Y^3$ have the meaning of one or two of the radicals from the group comprising a radical $R^1$, a radical $R^2$ and hydrogen, and/or $Y^4$ represents $R^3$ and hydrogen, or
d) reducing with a suitable reducing agent the pyridinium ring in a compound of formula

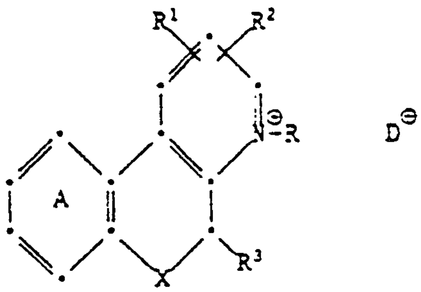

(VII),

wherein X, optional substituents on ring A, R and $R^1$ to $R^3$ are as defined above, and $D^{\ominus}$ is the anion of an organic or inorganic acid, or
e) reducing with a suitable reducing agent a compound of formula

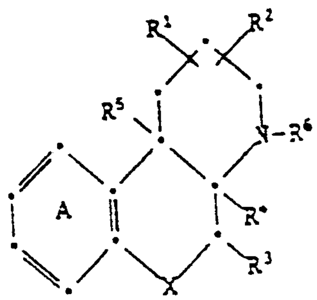

(VIII),

wherein X, optional substituents on ring A and $R^1$ to $R^5$ are as defined above and $R^6$ is $C_2$-$C_4$alkenyl, $C_2$-$C_4$-alkynyl, $C_1$-$C_4$alkanoyl or benzoyl, or
f) cyclising a compound of formula

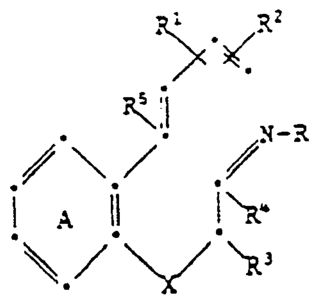

(IX),

wherein X, optional substituents on ring A, R and $R^1$ to $R^5$ are as defined above, to a compound of formula

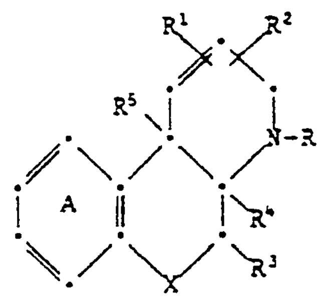

(Ib)

and, if desired, reducing the resulting product, or
g) cyclising a compound of formula

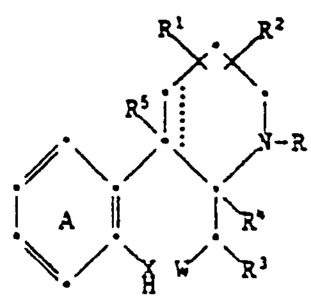

(X),

wherein the dotted line indicates the position of an optionally present double bond, X, optional substituents on ring A, R and $R^1$ to $R^5$ are as defined above provided that $R^5$ is present only if a single bond is present at the position designated by the dotted line, and wherein W represents reactive esterified hydroxy, or
h) cyclising a compound of formula

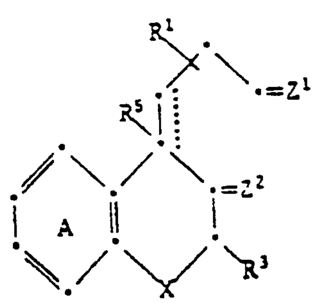

(XI),

wherein the dotted line indicates the position of an optionally present double bond, X, optional substituents on ring A, R and $R^1$ to $R^5$ are as defined above, and wherein one of the radicals $Z^1$ and

$Z^2$ is oxo, or reactive esterified hydroxy together with hydrogen or $C_1$-$C_4$alkyl, and the other of the two radicals $Z^1$ and $Z^2$ is -NHR together with hydrogen or $C_1$-$C_4$alkyl, provided that $R^5$ is present only if a single bond is present at the position designated by the dotted line, and, if desired, reducing the resulting product, or

i) cyclising a compound of formula

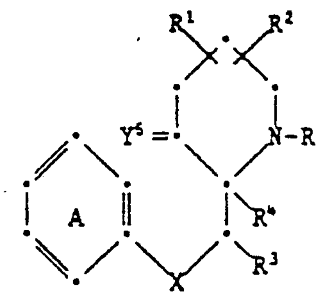

(XII),

wherein X, optional substituents on ring A, R and $R^1$ to $R^4$ are as defined above, $Y^5$ is oxo, or reactive esterified hydroxy together with hydrogen or $C_1$-$C_4$alkyl and, if desired, reducing the resulting product, or

j) cyclising a compound of formula

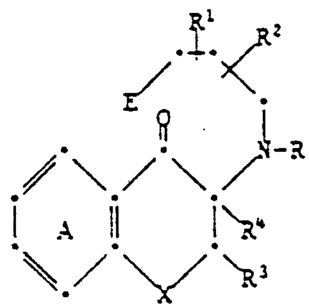

(XIII),

wherein X, optional substituents on ring A, R and $R^1$ to $R^4$ are as defined above and E represents an $\alpha$-carbanion-stabilizing leaving group and, if desired, reducing the resulting product,

and carrying out said processes while, if necessary, temporarily protecting any interfering reactive functional group(s), and then isolating the resulting compound of formula I, or the corresponding dehydro derivatives having a 1,2- or 1,10b-double bond, after appropriate removal of protecting groups and, if desired, converting a resulting compound of formula I, or the corresponding dehydro derivative having a 1,2- or 1,10b-double bond, into another of the compounds defined above and/or, if desired, converting a resulting free compound into a salt or a resulting salt into the corresponding free compound or into another salt and/or, if desired, separating a mixture of isomers or racemates obtained into the single isomers or racemates and/or, if desired, resolving a racemate obtained into the optical antipodes.

Claims for the following Contracting State: AT

**1.** A process for the manufacture of a compound of formula

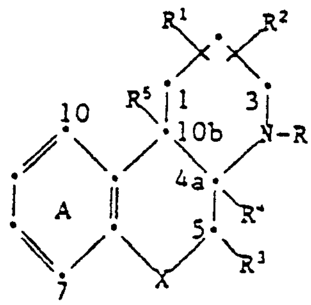

(I),

wherein X represents oxygen or sulfur, ring A is unsubstituted or substituted by one or two identical or different substituents from the group hydroxy, hydroxy-$C_1$-$C_4$alkyl, $C_1$-$C_4$alkoxy, $C_2$-$C_4$alkenyloxy, $C_2$-$C_4$alkynyloxy, $C_3$-$C_6$cycloalkyl-$C_1$-$C_2$alkoxy, benzyloxy, $C_1$-$C_4$alkoxy-$C_1$-$C_4$alkyl, $C_1$-$C_4$alkanoyloxy, benzoyloxy, benzenesulfonyloxy, 2-, 3- or 4-pyridylcarbonyloxy, $C_1$-$C_4$alkoxycarbonyloxy, carbamoyloxy, mono- or di-$C_1$-$C_4$alkylcarbamoyloxy, $C_1$-$C_4$alkanoyloxy-$C_1$-$C_4$alkyl, halogen, $C_1$-$C_4$alkyl, trifluoromethyl, amino, mono- or di-$C_1$-$C_4$alkylamino, $C_1$-$C_4$alkanoylamino, benzoylamino and $C_1$-$C_4$alkoxycarbonylamino or by one substituent $C_1$-$C_4$-alkylenedioxy, R represents hydrogen, $C_1$-$C_4$alkyl, benzyl or phenethyl, $R^1$ represents hydrogen, $C_1$-$C_4$alkyl, $C_1$-$C_4$alkylthio-$C_1$-$C_4$alkyl, amino, $C_1$-$C_4$-alkanoylamino, benzoylamino, $C_1$-$C_4$alkoxycarbonylamino, (amino, mono- or di-$C_1$-$C_4$alkylamino)-$C_1$-$C_4$alkyl, carboxy, $C_1$-$C_4$alkoxycarbonyl, carbamoyl or mono- or di-$C_1$-$C_4$alkylcarbamoyl, and $R^2$ to $R^5$ represent hydrogen or $C_1$-$C_4$alkyl, as well as the dehydro derivatives thereof having a double bond at the 1,2-position, or at the 1,10b-position in which case $R^5$ is absent, or a salt of such a compound, which comprises

a) reducing with a suitable reducing agent a compound of formula

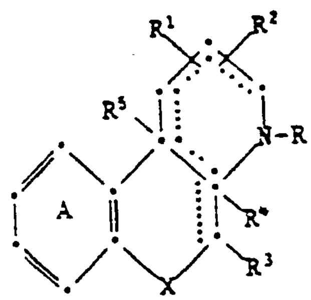

(IV),

wherein the dotted lines indicate the position of one, two or three non-cumulated double bonds, and X, optional substituents on ring A, R and $R^1$ to $R^5$ are as defined above provided that $R^4$ and $R^5$ are present only if the carbon atoms to which they are attached are not part of a double bond, or

b) reacting with a suitable reducing agent and/or elimination reagent a compound of formula

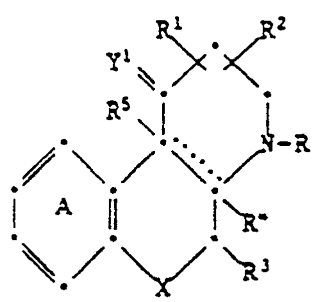

(V),

wherein the dotted line indicates the position of an optionally present double bond, X, optional substituents on ring A, R and $R^1$ to $R^5$ are as defined above provided that $R^4$ and $R^5$ are present only if the carbon atoms to which they are attached are not part of a double bond, and $Y^1$ is oxo or protected oxo, or Y' represents etherified, esterified or free hydroxy together with hydrogen, or

c) reducing with a suitable reducing agent a compound of formula

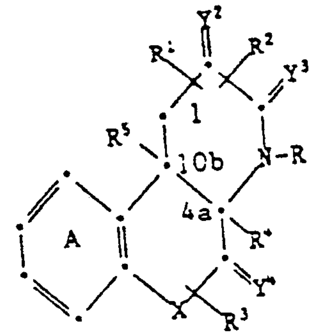

(VI),

wherein X, optional substituents on ring A, R and $R^1$ to $R^5$ are as defined above and one of the radicals $Y^2$, $Y^3$ and $Y^4$ is oxo or protected oxo, accompanied or not accompanied by a conjugated endocyclic carbon-carbon double bond, or in the case where $Y^3$ is oxo or protected oxo, also accompanied by a 1,10b- or a 4a,10b-double bond, and wherein of the two remaining radicals of $Y^2$ to $Y^4$, depending on whether additional carbon-carbon double bonds are present, $Y^2$ and $Y^3$ have the meaning of one or two of the radicals from the group comprising a radical $R^1$, a radical $R^2$ and hydrogen, and/or $Y^4$ represents $R^3$ and hydrogen, or

d) reducing with a suitable reducing agent the pyridinium ring in a compound of formula

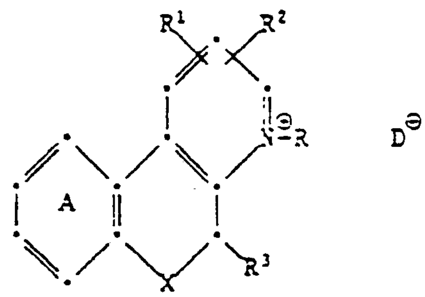

(VII),

wherein X, optional substituents on ring A, R and $R^1$ to $R^3$ are as defined above, and $D^{\ominus}$ is the anion of an organic or inorganic acid, or

e) reducing with a suitable reducing agent a compound of formula

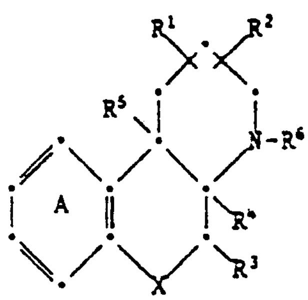

(VIII),

wherein X, optional substituents on ring A and $R^1$ to $R^5$ are as defined above and $R^6$ is $C_2$-$C_4$alkenyl, $C_2$-$C_4$-alkynyl, $C_1$-$C_4$alkanoyl or benzoyl, or

f) cyclising a compound of formula

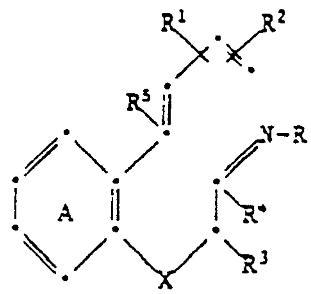

(IX),

wherein X, optional substituents on ring A, R and $R^1$ to $R^5$ are as defined above, to a compound of formula

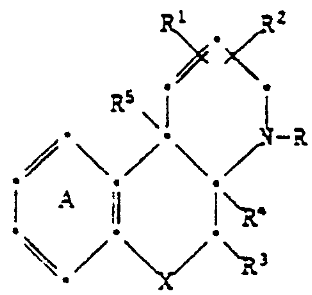

(Ib)

and, if desired, reducing the resulting product, or
g) cyclising a compound of formula

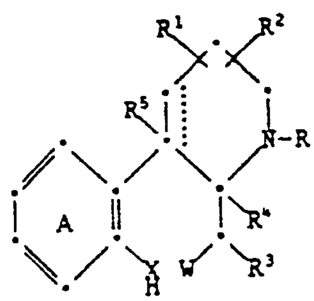

(X),

wherein the dotted line indicates the position of an optionally present double bond, X, optional substituents on ring A, R and $R^1$ to $R^5$ are as defined above provided that $R^5$ is present only if a single bond is present at the position designated by the dotted line, and wherein W represents reactive esterified hydroxy, or
h) cyclising a compound of formula

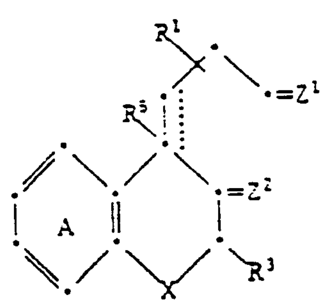

(XI),

wherein the dotted line indicates the position of an optionally present double bond, X, optional substituents on ring A, R and $R^1$ to $R^5$ are as defined above, and wherein one of the radicals $Z^1$ and

$Z^2$ is oxo, or reactive esterified hydroxy together with hydrogen or $C_1$-$C_4$alkyl, and the other of the two radicals $Z^1$ and $Z^2$ is -NHR together with hydrogen or $C_1$-$C_4$alkyl, provided that $R^5$ is present only if a single bond is present at the position designated by the dotted line, and, if desired, reducing the resulting product, or

i) cyclising a compound of formula

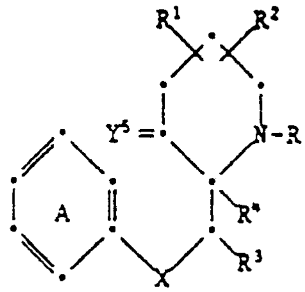

(XII),

wherein X, optional substituents on ring A, R and $R^1$ to $R^4$ are as defined above, $Y^5$ is oxo, or reactive esterified hydroxy together with hydrogen or $C_1$-$C_4$alkyl and, if desired, reducing the resulting product, or

j) cyclising a compound of formula

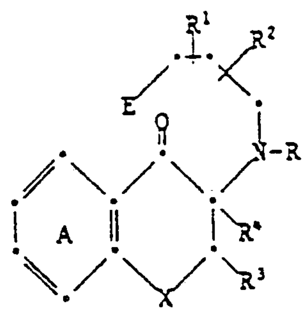

(XIII),

wherein X, optional substituents on ring A, R and $R^1$ to $R^4$ are as defined above and E represents an $\alpha$-carbanion-stabilizing leaving group and, if desired, reducing the resulting product,

and carrying out said processes while, if necessary, temporarily protecting any interfering reactive functional group(s), and then isolating the resulting compound of formula I, or the corresponding dehydro derivatives having a 1,2- or 1,10b-double bond, after appropriate removal of protecting groups and, if desired, converting a resulting compound of formula I, or the corresponding dehydro derivative having a 1,2- or 1,10b-double bond, into another of the compounds defined above and/or, if desired, converting a resulting free compound into a salt or a resulting salt into the corresponding free compound or into another salt and/or, if desired, separating a mixture of isomers or racemates obtained into the single isomers or racemates and/or, if desired, resolving a racemate obtained into the optical antipodes.

**2.** A process according to claim 1 for the manufacture of a compound of formula I, wherein X represents oxygen or sulfur, ring A is unsubstituted or substituted by one or two identical or different substituents from the group hydroxy, $C_1$-$C_4$alkanoyloxy, benzoyloxy, nicotinoyloxy, $C_1$-$C_4$alkoxy, hydroxymethyl, $C_1$-$C_4$alkyl, $C_1$-$C_4$alkanoyloxymethyl, $C_1$-$C_4$alkoxymethyl, halogen and trifluoromethyl, or by one substituent $C_1$-$C_4$alkylenedioxy, R represents hydrogen or $C_1$-$C_4$alkyl, and $R^1$ to $R^5$ represent hydrogen or $C_1$-$C_4$alkyl, the 1,10b- and 1,2-dehydro derivatives thereof, or a pharmaceutically acceptable salt thereof.

**3.** A process according to claim 1 for the manufacture of a compound of formula

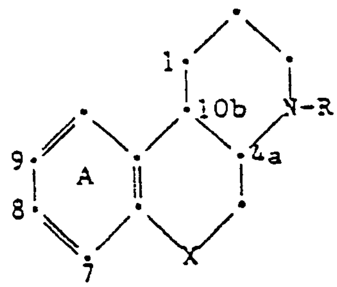

(II),

wherein X represents oxygen or sulfur, ring A is unsubstituted or substituted by one or two identical or different substituents from the group hydroxy, hydroxymethyl, $C_1$-$C_4$alkanoyloxy, benzoyloxy, pyridylcarbonyloxy, $C_1$-$C_4$alkanoyloxymethyl, $C_1$-$C_4$alkyl, $C_1$-$C_4$alkoxy and halogen, and R represents $C_1$-$C_4$alkyl, the 1,10b-dehydro derivative thereof, or a pharmaceutically acceptable salt thereof.

**4.** A process according to claim 3 for the manufacture of a compound of formula II, wherein X represents oxygen, ring A is monosubstituted by hydroxy, $C_1$-$C_4$alkanoyloxy, benzoyloxy or pyridylcarbonyloxy and R represents $C_1$-$C_4$alkyl, or a pharmaceutically acceptable salt thereof.

**5.** A process according to claim 1 for the manufacture of a compound of formula

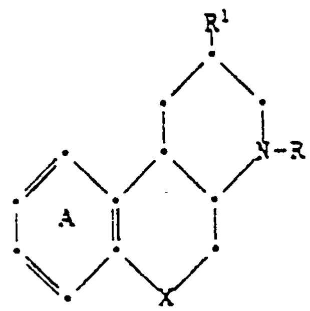

(III),

wherein X represents oxygen or sulfur, ring A is unsubstituted or substituted by one or two identical or different substituents from the group hydroxy, hydroxymethyl, $C_1$-$C_4$alkanoyloxy, benzoyloxy, nicotinoyloxy, $C_1$-$C_4$alkanoyloxymethyl, $C_1$-$C_4$alkoxy and halogen, R represents $C_1$-$C_4$alkyl, and $R^1$ represents $C_1$-$C_4$alkylthio-$C_1$-$C_4$alkyl, $C_1$-$C_4$alkanoylamino, (amino, mono- or di-$C_1$-$C_4$alkylamino)-$C_1$-$C_4$alkyl, carboxy, $C_1$-$C_4$alkoxycarbonyl, carbamoyl, mono- or di-$C_1$-$C_4$alkylcarbamoyl, or a pharmaceutically acceptable salt thereof.

**6.** A process according to claim 3 for the manufacture of a compound of formula II with a 4a,10b-trans ring linkage, in which X represents oxygen or sulfur, R represents $C_3$-$C_4$alkyl and ring A is monosubstituted in the 7-, 8- or 9-position by hydroxy, $C_1$-$C_4$alkanoyloxy, benzoyloxy, nicotinoyloxy, hydroxymethyl or $C_1$-$C_4$-alkanoyloxymethyl, or ring A is disubstituted in the 7,8-, 7,9- or 8-9-position, one substituent being hydroxy, $C_1$-$C_4$alkanoyloxy, benzoyloxy or nicotinoyloxy and the other hydroxy, $C_1$-$C_4$alkanoyloxy, benzoyloxy, nicotinoyloxy or halogen, the 1,10b-dehydro derivative thereof, or a pharmaceutically acceptable salt thereof.

**7.** A process according to claim 3 for the manufacture of a compound of formula II with 4a,10b-trans ring linkage, in which X represents oxygen, R represents n-propyl or n-butyl and ring A is substituted in the 7-, 8- or 9-position by hydroxy, $C_1$-$C_4$alkanoyloxy, benzoyloxy, nicotinoyloxy, hydroxymethyl or $C_1$-$C_4$alkanoyloxymethyl, or a pharmaceutically acceptable salt thereof.

**8.** A process according to claim 3 for the manufacture of a compound of formula II with 4a,10b-trans ring linkage, in which X represents oxygen, R represents n-propyl or n-butyl and ring A is substituted in the 7-, 8- or 9-position by hydroxy, $C_1$-$C_4$alkanoyloxy, benzoyloxy or nicotinoyloxy, or a pharmaceutically acceptable salt thereof.

9. A process according to claim 3 for the manufacture of a compound of formula II with 4a,10b-trans ring linkage, in which X represents oxygen, R represents methyl, ethyl or n-propyl, ring A is monosubstituted in the 7- or 10-position by hydroxy, $C_1$-$C_4$alkoxy, $C_1$-$C_4$alkanoyloxy, benzoyloxy or nicotinoyloxy, or ring A is disubstituted in the 7,8-, 7,10- or 8,10-position, one substituent being hydroxy or $C_1$-$C_4$alkoxy and the other $C_1$-$C_4$alkyl or halogen, or a pharmaceutically acceptable salt thereof.

10. A process according to claim 1 for the manufacture of a compound from the group trans-9-hydroxy-4-propyl-1,2,3,4a,5,10b-hexahydro-4H-[1]-benzopyrano[3,4-b]-pyridine, trans-7-hydroxy-4-propyl-1,2,3,4a,5,10b-hexahydro-4H-[1]-benzopyrano[3,4-b]pyridine and trans-10-bromo-7-methoxy-4-methyl-1,2,3,4a,5,10b-hexahydro-4H-[1]-benzopyrano[3,4-b]pyridine, or a pharmaceutically acceptable salt thereof.

11. A process according to claim 1 for the manufacture of a compound from the group trans-10-hydroxy-4-propyl-1,2,3,4a,5,10b-hexahydro-4H-[1]-benzopyrano[3,4-b]-pyridine, trans-8-ethyl-7-methoxy-4-methyl-1,2,3,4a,5,10b-hexahydro-4H-[1]-benzopyrano[3,4-b]-pyridine, trans-7-ethoxy-4,8-dimethyl-1,2,3,4a,5,10b-hexahydro-4H-[1]-benzopyrano[3,4-b]pyridine, trans-10-ethoxy-4-propyl-1,2,3,4a,5,10b-hexahydro-4H-[1]-benzopyrano[3,4-b]-pyridine and trans-7-methoxy-4,10-dimethyl-1,2,3,4a,5,10b-hexahydro-4H-[1]-benzopyrano[3,4-b]-pyridine, or a pharmaceutically acceptable salt thereof.

12. A process according to claim 1 for the manufacture of trans-7-hydroxy-4-propyl-1,2,3,4a,5,10b-hexahydro-4H-[1]-benzopyrano[3,4-b]pyridine or a pharmaceutically acceptable salt thereof.

13. A process according to claim 1 for the manufacture of trans-10-methoxy-4-propyl-1,2,3,4a,5,10b-hexahydro-4H-[1]-benzopyrano[3,4-b]pyridine or a pharmaceutically acceptable salt thereof.

14. A process according to claim 1 for the manufacture of trans-7-methoxy-4,10-dimethyl-1,2,3,4a,5,10b-hexahydro-4H-[1]-benzopyrano[3,4-b]pyridine or a pharmaceutically acceptable salt thereof.

15. A process according to claim 1 for the manufacture of trans-7-hydroxy-4,10-dimethyl-1,2,3,4a,5,10b-hexahydro-4H-[1]-benzopyrano[3,4-b]pyridine or a pharmaceutically acceptable salt thereof.

16. A process for the manufacture of a pharmaceutical preparation comprising a compound that can be manufactured according to any one of claims 1 to 15 or a pharmaceutically acceptable salt thereof, which comprises mixing the compound or salt with a pharmaceutically acceptable carrier.

**Revendications**

1. Composés de formule

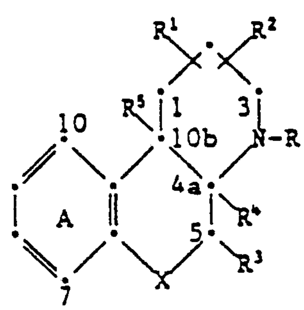

(I),

dans laquelle X représente l'oxygène ou le soufre, le noyau A est non substitué ou porte un ou deux substituants identiques ou différents pris parmi les groupes hydroxy, hydroxyalkyle en C 1-C 4, alcoxy en C 1-C 4, alcényloxy en C 2-C 4, alcynyloxy en C 2-C 4, (cycloalkyle en C 3-C 6)-alcoxy en C 1-C 2, benzyloxy, (alcoxy en C 1-C 4)-alkyle en C 1-C 4, alcanoyloxy en C 1-C 4, benzoyloxy, benzène-sulfonyloxy, 2-, 3- ou 4-pyridylcarbonyloxy, (alcoxy en C 1-C 4)-carbonyloxy, carbamoyloxy, mono- ou di-(alkyle en C 1-C 4)-carbamoyloxy, (alcanoyloxy en C 1-C 4)-alkyle en C 1-C 4, les halogènes, les groupes alkyle en C 1-C 4, trifluorométhyle, amino, mono- ou di-(alkyle en C 1-C 4)-amino, alcanoylamino en C 1-C 4, benzoylamino et (alcoxy en C 1-C 4)-carbonylamino ou porte un substituant alkylène-dioxy en C 1-C 4, R représente l'hydrogène, un groupe alkyle en C 1-C 4, benzyle ou phénéthyle, $R^1$ représente l'hydrogène, un groupe alkyle en C 1-C 4, (alkylthio en C 1-C 4)-alkyle en C 1-C 4, amino, alcanoylamino en C 1-C 4, benzoylamino, (alcoxy en C 1-C 4)-carbonylamino, (amino-, mono- ou di-(alkyle en C 1-C 4)-amino)-alkyle en C 1-C 4, carboxy, (alcoxy en C 1-C 4)-carbonyle, carbamoyle ou mono- ou di-(alkyle en C 1-C 4)-carbamoyle, et les symboles $R^2$ à $R^5$ représentent l'hydrogène ou des groupes alkyle en C 1-C 4, et les dérivés déshydrogénés contenant une double liaison en position 1,2 ou en position 1,10b, auquel cas $R^5$ n'existe pas, et les sels de ces composés.

2. Composés de formule I selon la revendication 1 dans laquelle X représente l'oxygène ou le soufre, le noyau A est non substitué, ou porte un ou deux substituants identiques ou différents pris parmi les groupes hydroxy, alcanoyloxy en C 1-C 4, benzoyloxy, nicotinoyloxy, alcoxy en C 1-C 4, hydroxyméthyle, alkyle en C 1-C 4, (alcanoyle en C 1-C 4)-oxyméthyle, (alcoxy en C 1-C 4)-méthyle, les halogènes et les groupes trifluorométhyle ou porte un substituant alkylène-dioxy en C 1-C 4, R représente l'hydrogène ou un groupe alkyle en C 1-C 4 et les symboles $R^1$ à $R^5$ représentent l'hydrogène ou des groupes alkyle en C 1-C 4, leurs dérivés déshydrogénés en 1,10b et 1,2 et leurs sels acceptables pour l'usage pharmaceutique.

3. Composés selon la revendication 1, de formule

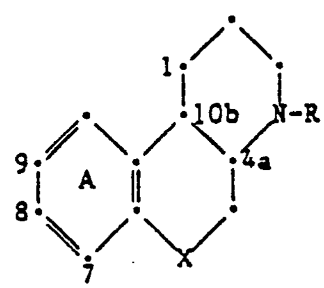

(II),

dans laquelle X représente l'oxygène ou le soufre, le noyau A est non substitué ou porte un ou deux substituants identiques ou différents pris parmi les groupes hydroxy, hydroxyméthyle, alcanoyloxy en C 1-C 4, benzoyloxy, pyridylcarbonyloxy, (alcanoyle en C 1-C 4)-oxyméthyle, alkyle en C 1-C 4, alcoxy en C 1-C 4 et les halogènes, et R représente un groupe alkyle en C 1-C 4, leurs dérivés déshydrogénés en 1,10b et leurs sels acceptables pour l'usage pharmaceutique.

4. Composés de formule II de la revendication 3, dans laquelle X représente l'oxygène, le noyau A porte un substituant hydroxy, alcanoyloxy en C 1-C 4, benzoyloxy ou pyridylcarbonyloxy et R représente un groupe alkyle en C 1-C 4, et leurs sels acceptables pour l'usage pharmaceutique.

5. Composés selon la revendication 1, de formule

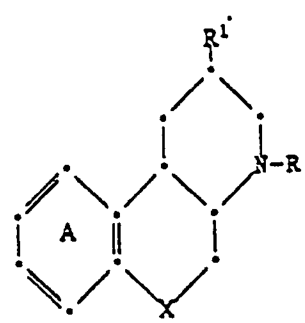

(III),

dans laquelle X représente l'oxygène ou le soufre, le noyau A est non substitué ou porte un ou deux substituants identiques ou différents pris parmi les groupes hydroxy, hydroxyméthyle, alcanoyloxy en C 1-C 4, benzoyloxy, nicotinoyloxy, (alcanoyle en C 1-C 4)-oxyméthyle, alcoxy en C 1-C 4 et les halogènes, R représente un groupe alkyle en C 1-C 4 et $R^1$ représente un groupe (alkylthio en C 1-C 4)-alkyle en C 1-C 4, alcanoylamino en C 1-C 4, (amino, mono- ou di-(alkyle en C 1-C 4)-amino))-alkyle

en C 1-C 4, carboxy, (alcoxy en C 1-C 4)-carbonyle, carbamoyle ou mono- ou di-(alkyle en C 1-C 4)-carbamoyle et leurs sels acceptables pour l'usage pharmaceutique.

**6.** Composés de formule II de la revendication 3, à liaison 4a,10b-trans entre les cycles, et dans lesquels X représente l'oxygène ou le soufre, R représente un groupe alkyle en C 3-C 4 et le noyau A porte un substituant en position 7, 8 ou 9 pris parmi les groupes hydroxy, alcanoyloxy en C 1-C 4, benzoyloxy, nicotinoyloxy, hydroxyméthyle ou (alcanoyle en C 1-C 4)-oxyméthyle ou bien le noyau A est disubstitué dans les positions 7,8; 7,9 ou 8,9, l'un de ces substituants étant un groupe hydroxy, alcanoyloxy en C 1-C 4, benzoyloxy ou nicotinoyloxy et l'autre un groupe hydroxy, alcanoyloxy en C 1-C 4, benzoyloxy, nicotinoyloxy ou un halogène, leurs dérivés déshydrogénés en 1,10b et leurs sels acceptables pour l'usage pharmaceutique.

**7.** Composés de formule II de la revendication 3, à liaison 4a,10b-trans entre les cycles, dans lesquels X représente l'oxygène, R représente un groupe n-propyle ou n-butyle et le noyau A porte un substituant hydroxy, alcanoyloxy en C 1-C 4, benzoyloxy, nicotinoyloxy, hydroxyméthyle ou (alcanoyle en C 1-C 4)-oxyméthyle en position 7, 8 ou 9, et leurs sels acceptables pour l'usage pharmaceutique.

**8.** Composés de formule II de la revendication 3, à liaison 4a,10b-trans entre les cycles, dans lesquels X représente l'oxygène, R représente un groupe n-propyle ou n-butyle et le noyau A est substitué en position 7, 8 ou 9 par un groupe hydroxy, alcanoyloxy en C 1-C 4, benzoyloxy ou nicotinoyloxy, et leurs sels acceptables pour l'usage pharmaceutique.

**9.** Composés de formule II selon la revendication 3, à liaison 4a,10b-trans entre les cycles, dans lesquels X représente l'oxygène, R représente un groupe méthyle, éthyle ou n-propyle, le noyau A porte un substituant en position 7 ou 10 pris parmi les groupes hydroxy, alcoxy en C 1-C 4, alcanoyloxy en C 1-C 4, benzoyloxy ou nicotinoyloxy, ou bien le noyau A est disubstitué dans les positions 7,8; 7,10 ou 8,10, l'un de ces substituants étant un groupe hydroxy ou alcoxy en C 1-C 4 et l'autre un groupe alkyle en C 1-C 4 ou un halogène, et leurs sels acceptables pour l'usage pharmaceutique.

**10.** Un composé selon la revendication 1, pris dans le groupe formé par la trans-9-hydroxy-4-propyl-1,2,3,4a,5,10b-hexahydro-4H-[1]-benzopyranno[3,4-b]-pyridine, la trans-7-hydroxy-4-propyl-1,2,3,4a,5-10b-hexahydro-4H-[1]-benzopyranno[3,4-b]pyridine et la trans-10-bromo-7-méthoxy-4-méthyl-1,2,3,4a,5,10b-hexahydro-4H-[1]-benzopyranno[3,4-b]pyridine, ou un sel acceptable pour l'usage pharmaceutique de l'un de ces composés.

**11.** Un composé selon la revendication 1, pris dans le groupe formé par la trans-10-hydroxy-4-propyl-1,2,3,4a,5,10b-hexahydro-4H-[1]-benzopyranno[3,4-b]pyridine, la trans-8-éthyl-7-méthoxy-4-méthyl-1,2,3,4a,5,10b-hexahydro-4H-[1]-benzopyranno[3,4-b]pyridine, la trans-7-éthoxy-4,8-diméthyl-1,2,3,4a,5,10b-hexahydro-4H-[1]-benzopyranno[3,4-b]pyridine, la trans-10-éthoxy-4-propyl-1,2,3,4a,5,10b-hexahydro-4H-[1]-benzopyranno[3,4-b]pyridine et la trans-7-méthoxy-4,10-diméthyl-1,2,3,4a,5,10b-hexahydro-4H-[1]benzopyranno[3,4-b]pyridine, ou un sel acceptable pour l'usage pharmaceutique de l'un de ces composés.

**12.** La trans-7-hydroxy-4-propyl-1,2,3,4a,5,10b-hexahydro-4H-[1]-benzopyranno[3,4-b]pyridine selon la revendication 1 ou l'un de ses sels acceptables pour l'usage pharmaceutique.

**13.** La trans-10-méthoxy-4-propyl-1,2,3,4a,5,10b-hexahydro-4H-[1]-benzopyranno[3,4-b]pyridine selon la revendication 1 ou l'un de ses sels acceptables pour l'usage pharmaceutique.

**14.** La trans-7-méthoxy-4,10-diméthyl-1,2,3,4a,5, 10b-hexahydro-4H-[1]-benzopyranno[3,4-b]pyridine selon la revendication 1 ou l'un de ses sels acceptables pour l'usage pharmaceutique.

**15.** La trans-7-hydroxy-4,10-diméthyl-1,2,3,4a,5, 10b-hexahydro-4H-[1]-benzopyranno[3,4-b]pyridine selon la revendication 1 ou l'un de ses sels acceptables pour l'usage pharmaceutique.

**16.** Les composés des revendications 1 à 15 pour l'utilisation dans un procédé pour le traitement thérapeutique de l'organisme humain ou animal.

17. Les composés des revendications 1 à 15, en tant que neuroleptiques ou antidépressifs.

18. Compositions pharmaceutiques contenant des composés des revendications 1 à 15 ou leurs sels acceptables pour l'usage pharmaceutique avec un véhicule approprié à l'usage pharmaceutique.

19. Utilisation des composés des revendications 1 à 15 pour la préparation de compositions pharmaceutiques.

20. Procédé de préparation des composés de formule I de la revendication 1, dans laquelle tous les symboles ont les significations indiquées dans la revendication 1, et de leurs sels, caractérisé en ce que
a) on réduit à l'aide d'un agent réducteur approprié un composé de formule

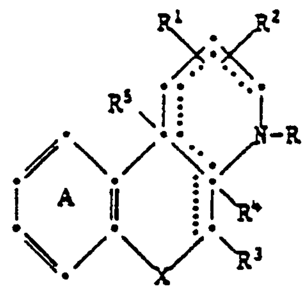

(IV),

dans laquelle les traits interrompus indiquent les positions d'une, deux ou trois doubles liaisons non cumulées, X, les substituants éventuels du noyau A, R et $R^1$ à $R^5$ sont tels que définis ci-dessus, sous réserve que $R^4$ et $R^5$ ne peuvent exister que si les atomes de carbone auxquels ils sont fixés n'appartiennent pas à une double liaison, ou bien
b) on traite par un agent réducteur et/ou un réactif d'élimination approprié un composé de formule

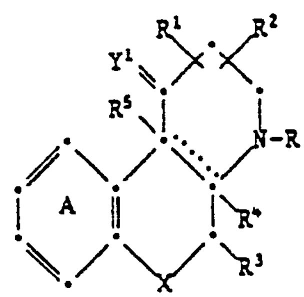

(V),

dans laquelle le trait interrompu indique la position d'une double liaison éventuelle, X, les substituants éventuels du noyau A, R et $R^1$ à $R^5$ sont tels que définis ci-dessus, sous réserve que $R^4$ et $R^5$ ne peuvent exister que si les atomes de carbone auxquels ils sont reliés n'appartiennent pas à une double liaison, et $Y^1$ représente un groupe oxo ou oxo protégé ou bien $Y^1$ représente un groupe hydroxy éthérifié, estérifié ou libre et l'hydrogène, ou bien
c) on réduit à l'aide d'un agent réducteur approprié un composé de formule

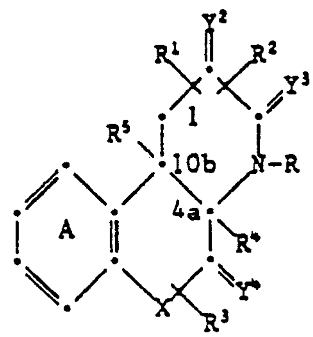

(VI),

dans laquelle X, Les substituants éventuels du noyau A, R et $R^1$ à $R^5$ sont tels que définis ci-dessus, et l'un des symboles $Y^2$, $Y^3$ et $Y^4$ représente un groupe oxo ou oxo protégé, accompagné ou non d'une double liaison carbone-carbone endocyclique conjuguée, ou bien, dans le cas où $Y^3$ représente un groupe oxo ou oxo protégé, également accompagné d'une double liaison 1,10b ou 4a,10b, et des deux symboles restants parmi $Y^2$, $Y^3$ et $Y^4$, selon qu'il y a ou non d'autres doubles liaisons carbone-carbone, $Y^2$ et $Y^3$ représentent un ou deux substituants du groupe consistant en un substituant $R^1$, un substituant $R^2$ et l'hydrogène, et/ou $Y^4$ représente $R^3$ et l'hydrogène, ou bien

d) dans un composé de formule

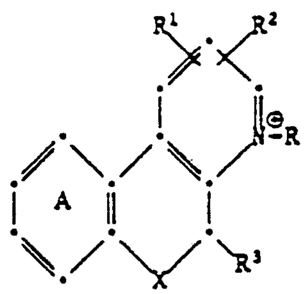

$D^{\ominus}$ (VII),

dans laquelle X, les substituants éventuels du noyau A, R et $R^1$ à $R^3$ sont tels que définis ci-dessus et $D^{\ominus}$ représente l'anion d'un acide organique ou minéral, on réduit le cycle pyridinium à l'aide d'un agent réducteur approprié, ou bien

e) on réduit à l'aide d'un agent réducteur approprié un composé de formule

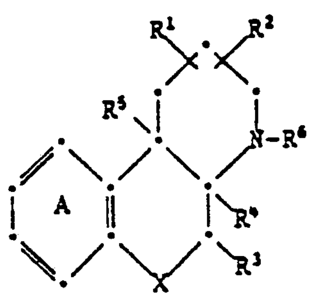

(VIII),

dans laquelle X, les substituants éventuels du noyau A et $R^1$ à $R^5$ sont tels que définis ci-dessus, et $R^6$ représente un groupe alcényle en C 2-C 4, alcynyle en C 2-C 4, alcanoyle en C 1-C 4 ou benzoyle, ou bien

f) on cyclise un composé de formule

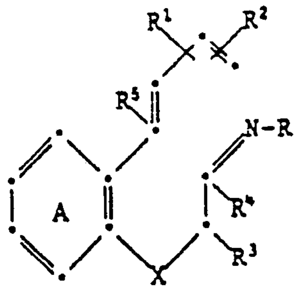

(IX),

dans laquelle X, les substituants éventuels du noyau A, R et $R^1$ à $R^5$ sont tels que définis ci-dessus, en un composé de formule

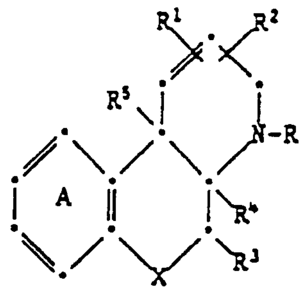

(Ib)

et si on le désire, on réduit ce composé, ou bien
g) on cyclise un composé de formule

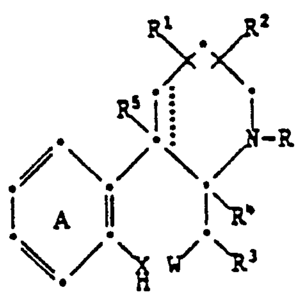

(X),

dans laquelle le trait interrompu indique la position d'une double liaison éventuelle, X, les substituants éventuels du noyau A, R et $R^1$ à $R^5$ sont tels que définis ci-dessus, sous réserve que $R^5$ ne peut exister que si, dans la position signalée par le trait interrompu, il y a une liaison simple, et W représente un groupe hydroxy estérifié réactif, ou bien
h) on cyclise un composé de formule

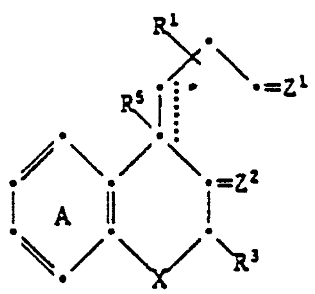

(XI),

dans laquelle le trait interrompu indique la position d'une double liaison éventuelle, X, les substituants éventuels du noyau A, R et $R^1$ à $R^5$ sont tels que définis ci-dessus, et l'un des symboles $Z^1$

et $Z^2$ représente un groupe oxo ou un groupe hydroxy estérifié réactif et l'hydrogène ou un groupe alkyle en C 1-C 4 et l'autre représente -NHR et l'hydrogène ou un groupe alkyle en C 1-C 4, sous réserve que $R^5$ ne peut être présent que lorsqu'il y a une liaison simple dans la position indiquée par le trait interrompu, et si on le désire, on réduit le composé obtenu, ou bien

i) on cyclise un composé de formule

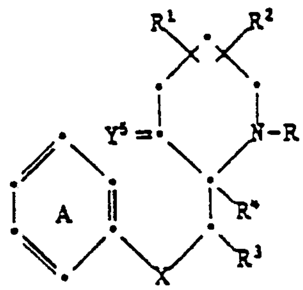

(XII),

dans laquelle X, les substituants éventuels du noyau A, R et $R^1$ à $R^4$ sont tels que définis ci-dessus, $Y^5$ représente un groupe oxo ou un groupe hydroxy estérifié réactif et l'hydrogène ou un groupe alkyle en C 1-C 4, et si on le désire, on réduit le composé obtenu, ou bien

j) on cyclise un composé de formule

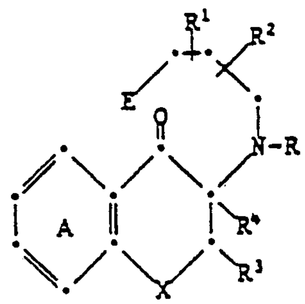

(XIII),

dans laquelle X, les substituants éventuels du noyau A, R et $R^1$ à $R^4$ sont tels que définis ci-dessus et E représente un groupe éliminable stabilisant un alpha-carbanion et si on le désire, on réduit le composé obtenu, toutes ces opérations étant effectuées, lorsque c'est nécessaire, avec protection transitoire des groupes fonctionnels réactifs dont la réactivité serait gênante, le composé de formule I ou son dérivé déshydrogéné à double liaison 1,2 ou 1,10b ainsi obtenu étant isolé après élimination des groupes protecteurs en question, et, si on le désire, on convertit un composé de formule I ainsi obtenu ou le dérivé déshydrogéné correspondant à double liaison 1,2 ou 1,10b en un autre composé tel que défini ci-dessus et/ou, si on le désire, on convertit un composé obtenu à l'état libre en un sel ou un sel ainsi obtenu en le composé libre correspondant ou en un autre sel et/ou, si on le désire, on sépare un mélange d'isomères ou de racémates ainsi obtenus en les isomères ou racémates individuels et/ou, si on le désire, on résout un racémate ainsi obtenu en les antipodes optiques.

Revendications pour l'Etat contractant suivant: AT

**1.** Procédé de préparation des composés de formule

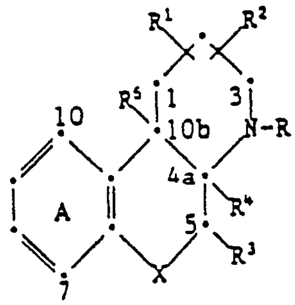

(I),

dans laquelle X représente l'oxygène ou le soufre, le noyau A est non substitué ou porte un à deux substituants identiques ou différents pris parmi les groupes hydroxy, hydroxyalkyle en C 1-C 4, alcoxy en C 1-C 4, alcényloxy en C 2-C 4, alcynyloxy en C 2-C 4, (cycloalkyle en C 3-C 6)-alcoxy en C 1-C 2, benzyloxy, (alcoxy en C 1-C 4)-alkyle en C 1-C 4, alcanoyloxy en C 1-C 4, benzoyloxy, benzènesulfonyloxy, 2-, 3- ou 4-pyridylcarbonyloxy, (alcoxy en C 1-C 4)-carbonyloxy, carbamoyloxy, mono- ou di-(alkyle en C 1-C 4)-carbamoyloxy, (alcanoyloxy en C 1-C 4)-alkyle en C 1-C 4, les halogènes, les groupes alkyle en C 1-C 4, trifluorométhyle, amino, mono- ou di-(alkyle en C 1-C 4)-amino, alcanoylamino en C 1-C 4, benzoylamino et (alcoxy en C 1-C 4)-carbonylamino ou porte un substituant alkylènedioxy en C 1-C 4, R représente l'hydrogène, un groupe alkyle en C 1-C 4, benzyle ou phénéthyle, $R^1$ représente l'hydrogène, un groupe alkyle en C 1-C 4, (alkylthio en C 1-C 4)-alkyle en C 1-C 4, amino, alcanoylamino en C 1-C 4, benzoylamino, (alcoxy en C 1-C 4)-carbonylamino, (amino-, mono- ou di-(alkyle en C 1-C 4)-amino))-alkyle en C 1-C 4, carboxy, (alcoxy en C 1-C 4)-carbonyle, carbamoyle ou mono- ou di-(alkyle en C 1-C 4)-carbamoyle et $R^2$ à $R^5$ représentent l'hydrogène ou des groupes alkyle en C 1-C 4, de leurs dérivés déshydrogénés ayant une double liaison en position 1,2 ou 1,10b, auquel cas le groupe $R^5$ n'existe pas, et des sels de ces composés, caractérisé en ce que

a) on réduit à l'aide d'un agent réducteur approprié un composé de formule

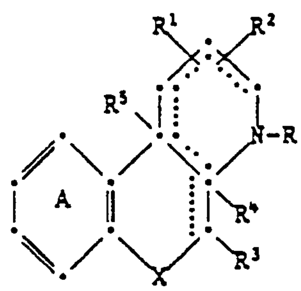

(IV),

dans laquelle les traits interrompus indiquent les positions d'une, deux ou trois doubles liaisons non cumulées, X, les substituants éventuels du noyau A, R et $R^1$ à $R^5$ sont tels que définis ci-dessus, sous réserve que $R^4$ et $R^5$ ne peuvent exister que si les atomes de carbone auxquels ils sont fixés n'appartiennent pas à une double liaison, ou bien

b) on traite par un agent réducteur et/ou un réactif d'élimination approprié un composé de formule

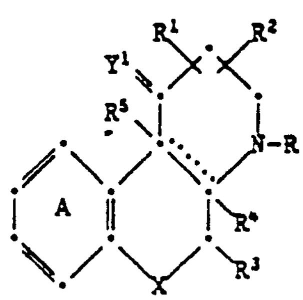

(V),

dans laquelle le trait interrompu indique la position d'une double liaison éventuelle, X, les substituants éventuels du noyau A, R et $R^1$ et $R^5$ sont tels que définis ci-dessus, sous réserve que $R^4$ et

$R^5$ ne peuvent exister que si les atomes de carbone auxquels ils sont reliés n'appartiennent pas à une double liaison, et $Y^1$ représente un groupe oxo ou oxo protégé ou bien $Y^1$ représente un groupe hydroxy éthérifié, estérifié ou libre et l'hydrogène, ou bien
c) on réduit à l'aide d'un agent réducteur approprié un composé de formule

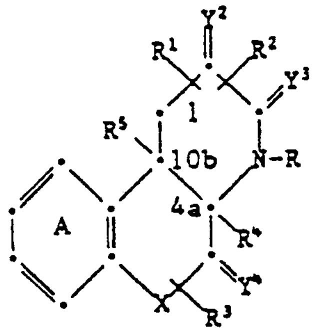

(VI),

dans laquelle X, les substituants éventuels du noyau A, R et $R^1$ à $R^5$ sont tels que définis ci-dessus, et l'un des symboles $Y^2$, $Y^3$ et $Y^4$ représente un groupe oxo ou oxo protégé, accompagné ou non d'une double liaison carbone-carbone endocyclique conjuguée, ou bien, dans le cas où $Y^3$ représente un groupe oxo ou oxo protégé, également accompagné d'une double liaison 1,10b ou 4a,10b, et des deux symboles restants parmi $Y^2$, $Y^3$ et $Y^4$, selon qu'il y a ou non d'autres doubles liaisons carbone-carbone, $Y^2$ et $Y^3$ représentent un ou deux substituants du groupe consistant en un substituant $R^1$, un substituant $R^2$ et l'hydrogène, et/ou $Y^4$ représente $R^3$ et l'hydrogène, ou bien
d) dans un composé de formule

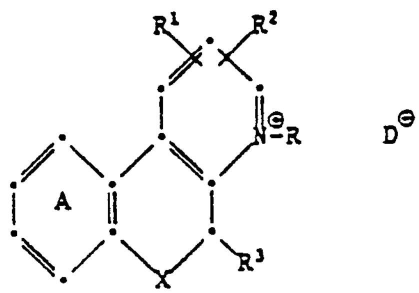

(VII),

dans laquelle X, les substituants éventuels du noyau A, R et $R^1$ à $R^3$ sont tels que définis ci-dessus et $D^{\ominus}$ représente l'anion d'un acide organique ou minéral, on réduit le cycle pyridinium à l'aide d'un agent réducteur approprié, ou bien
e) on réduit à l'aide d'un agent réducteur approprié un composé de formule

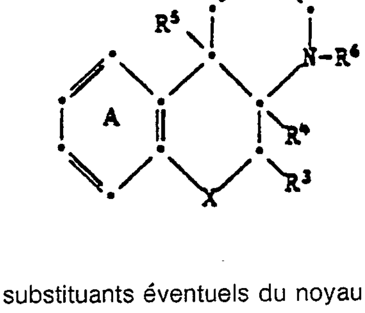

(VIII),

dans laquelle X, les substituants éventuels du noyau A et $R^1$ à $R^5$ sont tels que définis ci-dessus, et $R^6$ représente un groupe alcényle en C 2-C 4, alcynyle en C 2-C 4, alcanoyle en C 1-C 4 ou benzoyle, ou bien
f) on cyclise un composé de formule

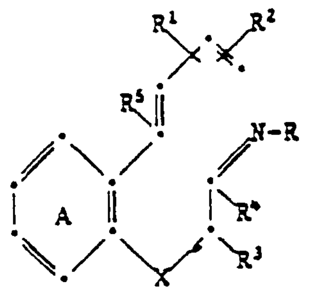

(IX),

dans laquelle X, les substituants éventuels du noyau A, R et $R^1$ à $R^5$ sont tels que définis ci-dessus, en un composé de formule

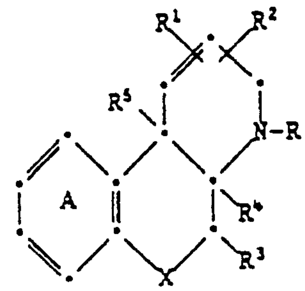

(Ib)

et si on le désire, on réduit ce composé, ou bien

g) on cyclise un composé de formule

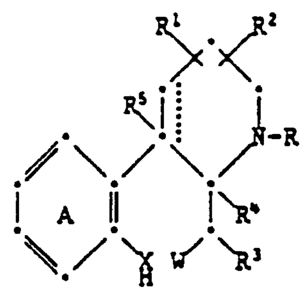

(X),

dans laquelle le trait interrompu indique la position d'une double liaison éventuelle, X, les substituants éventuels du noyau A, R et $R^1$ à $R^5$ sont tels que définis ci-dessus, sous réserve que $R^5$ ne peut exister que si, dans la position signalée par le trait interrompu, il y a une liaison simple, et W représente un groupe hydroxy estérifié réactif, ou bien

h) on cyclise un composé de formule

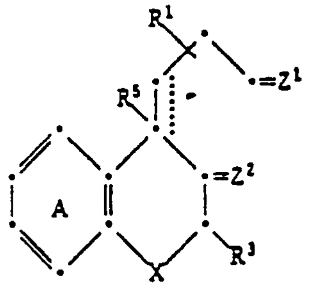

(XI),

dans laquelle le trait interrompu indique la position d'une double liaison éventuelle, X, les substituants éventuels du noyau A, R et $R^1$ à $R^5$ sont tels que définis ci-dessus, et l'un des symboles $Z^1$ et $Z^2$ représente un groupe oxo ou un groupe hydroxy estérifié réactif et l'hydrogène ou un groupe

alkyle en C 1-C 4 et l'autre représente -NHR et l'hydrogène ou un groupe alkyle en C 1-C 4, sous réserve que $R^5$ ne peut être présent que lorsqu'il y a une liaison simple dans la position indiquée par le trait interrompu, et si on le désire, on réduit le composé obtenu, ou bien

i) on cyclise un composé de formule

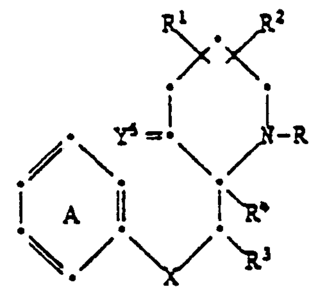

(XII),

dans laquelle X, les substituants éventuels du noyau A, R et $R^1$ à $R^4$ sont tels que définis ci-dessus, $Y^5$ représente un groupe oxo ou un groupe hydroxy estérifié réactif et l'hydrogène ou un groupe alkyle en C 1-C 4, et si on le désire, on réduit le composé obtenu, ou bien

j) on cyclise un composé de formule

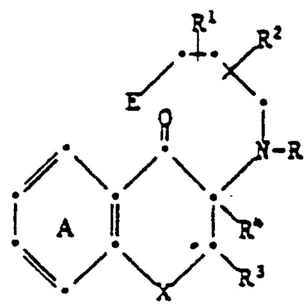

(XIII),

dans laquelle X, les substituants éventuels du noyau A, R et $R^1$ à $R^4$ sont tels que définis ci-dessus et E représente un groupe éliminable stabilisant un alpha-carbanion et si on le désire, on réduit le composé obtenu, toutes ces opérations étant effectuées, lorsque c'est nécessaire, avec protection transitoire des groupes fonctionnels réactifs dont la réactivité serait gênante, le composé de formule I ou son dérivé déshydrogéné à double liaison 1,2 ou 1,10b ainsi obtenu étant isolé après élimination des groupes protecteurs en question, et, si on le désire, on convertit un composé de formule I ainsi obtenu ou le dérivé déshydrogéné correspondant à double liaison 1,2 ou 1,10b en un autre composé tel que défini ci-dessus et/ou, si on le désire, on convertit un composé obtenu à l'état libre en un sel ou un sel ainsi obtenu en le composé libre correspondant ou en un autre sel et/ou, si on le désire, on sépare un mélange d'isomères ou de racémates ainsi obtenus en les isomères ou racémates individuels et/ou, si on le désire, on résout un racémate ainsi obtenu en les antipodes optiques.

**2.** Procédé selon la revendication 1 pour la préparation des composés de formule I dans laquelle X représente l'oxygène ou le soufre, le noyau A est non substitué, ou porte un ou deux substituants identiques ou différents pris parmi les groupes hydroxy, alcanoyloxy en C 1-C 4, benzoyloxy, nicotinoyloxy, alcoxy en C 1-C 4, hydroxyméthyle, alkyle en C 1-C 4, (alcanoyle en C 1-C 4)-oxyméthyle, (alcoxy en C 1-C 4)-méthyle, les halogènes et les groupes trifluorométhyle ou porte un substituant alkylène-dioxy en C 1-C 4, R représente l'hydrogène ou un groupe alkyle en C 1-C 4 et les symboles $R^1$ à $R^5$ représentent l'hydrogène ou des groupes alkyle en C 1-C 4, leurs dérivés déshydrogénés en 1,10b et 1,2 et leurs sels acceptables pour l'usage pharmaceutique.

**3.** Procédé selon la revendication 1 pour la préparation des composés de formule

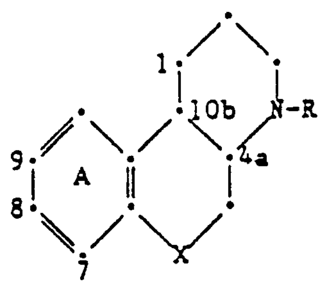

(II),

dans laquelle X représente l'oxygène ou le soufre, le noyau A est non substitué ou porte un ou deux substituants identiques ou différents pris parmi les groupes hydroxy, hydroxyméthyle, alcanoyloxy en C 1-C 4, benzoyloxy, pyridylcarbonyloxy, (alcanoyle en C 1-C 4)-oxyméthyle, alkyle en C 1-C 4, alcoxy en C 1-C 4 et les halogènes, et R représente un groupe alkyle en C 1-C 4, leurs dérivés déshydrogénés en 1,10b et leurs sels acceptables pour l'usage pharmaceutique.

4. Procédé selon la revendication 3 pour la préparation des composés de formule II dans laquelle X représente l'oxygène, le noyau A porte un substituant hydroxy, alcanoyloxy en C 1-C 4, benzoyloxy ou pyridylcarbonyloxy et R représente un groupe alkyle en C 1-C 4, et leurs sels acceptables pour l'usage pharmaceutique.

5. Procédé selon la revendication 1 pour préparer les composés de formule

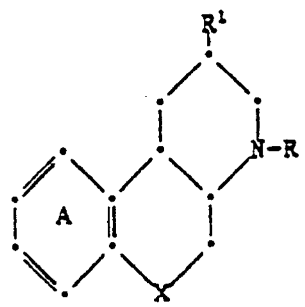

(III),

dans laquelle X représente l'oxygène ou le soufre, le noyau A est non substitué ou porte un ou deux substituants identiques ou différents pris parmi les groupes hydroxy, hydroxyméthyle, alcanoyloxy en C 1-C 4, benzoyloxy, nicotinoyloxy, (alcanoyle en C 1-C 4)-oxyméthyle, alcoxy en C 1-C 4 et les halogènes, R représente un groupe alkyle en C 1-C 4 et $R^1$ représente un groupe (alkylthio en C 1-C 4)-alkyle en C 1-C 4, alcanoylamino en C 1-C 4, (amino, mono- ou di-(alkyle en C 1-C 4)-amino))-alkyle en C 1-C 4, carboxy, (alcoxy en C 1-C 4)-carbonyle, carbamoyle ou mono- ou di-(alkyle en C 1-C 4)-carbamoyle et leurs sels acceptables pour l'usage pharmaceutique.

6. Procédé selon la revendication 3 pour la pré-paration des composés de formule II à liaison 4a,10b-trans entre les cycles, dans lesquels X représente l'oxygène ou le soufre, R représente un groupe alkyle en C 3-C 4 et le noyau A porte un substituant en position 7, 8 ou 9 pris parmi les groupes hydroxy, alcanoyloxy en C 1-C 4, benzoyloxy, nicotinoyloxy, hydroxyméthyle ou (alcanoyle en C 1-C 4)-oxyméthyle ou bien le noyau A est disubstitué dans les positions 7,8; 7,9 ou 8,9, l'un de ces substituants étant un groupe hydroxy, alcanoyloxy en C 1-C 4, benzoyloxy ou nicotinoyloxy et l'autre un groupe hydroxy, alcanoyloxy en C 1-C 4, benzoyloxy, nicotinoyloxy ou un halogène, leurs dérivés déshydrogénés en 1,10b et leurs sels acceptables pour l'usage pharmaceutique.

7. Procédé selon la revendication 3 pour préparer les composés de formule II à liaison 4a,10b-trans entre les cycles, dans lesquels X représente l'oxygène, R représente un groupe n-propyle ou n-butyle et le noyau A porte un substituant hydroxy, alcanoyloxy en C 1-C 4, benzoyloxy, nicotinoyloxy, hydroxyméthyle ou (alcanoyle en C 1-C 4)-oxyméthyle en position 7, 8 ou 9,, et leurs sels acceptables pour l'usage pharmaceutique.

8. Procédé selon la revendication 3 pour la préparation des composés de formule II à liaison 4a,10b-trans entre les cycles, dans lesquels X représente l'oxygène, R représente un groupe n-propyle ou n-butyle et le noyau A est substitué en position 7, 8 ou 9 par un groupe hydroxy, alcanoyloxy en C 1-C 4, benzoyloxy ou nicotinoyloxy, et leurs sels acceptables pour l'usage pharmaceutique.

9. Procédé selon la revendication 3 pour préparer les composés de formule II à liaison 4a,10b-trans entre les cycles, dans lesquels X représente l'oxygène, R représente un groupe méthyle, éthyle ou n-propyle, le noyau A porte un substituant en position 7 ou 10 pris parmi les groupes hydroxy, alcoxy en C 1-C 4, alcanoyloxy en C 1-C 4, benzoyloxy ou nicotinoyloxy, ou bien le noyau A est disubstitué dans les positions 7,8; 7,10 ou 8,10, l'un de ces substituants étant un groupe hydroxy ou alcoxy en C 1-C 4 et l'autre un groupe alkyle en C 1-C 4 ou un halogène, et leurs sels acceptables pour l'usage pharmaceutique.

10. Procédé selon la revendication 1 pour préparer les composés du groupe formé par la trans-9-hydroxy-4-propyl-1,2,3,4a,5,10b-hexahydro-4H-[1]-benzopyranno[3,4-b]-pyridine, la trans-7-méthoxy-4-propyl-1,2,3,4a,5,10b-hexahydro-4H-[1]-benzopyranno[3,4-b]pyridine et la trans-10-bromo-7-méthoxy-4-méthyl-1,2,3,4a,5,10b-hexahydro-4H-[1]benzopyranno[3,4-b]pyridine, ou un sel acceptable pour l'usage pharmaceutique de l'un de ces composés.

11. Procédé selon la revendication 1 pour préparer les composés du groupe formé par la trans-10-hydroxy-4-propyl-1,2,3,4a,5,10b-hexahydro-4H-[1]-benzopyranno[3,4-b]pyridine, la trans-8-éthyl-7-méthoxy-4-méthyl-1,2,3,4a, 5,10b-hexahydro-4H-[1]-benzopyranno[3,4-b]pyridine, la trans-7-éthoxy-4,8-diméthyl-1,2,3,4a,5,10b-hexahydro-4H-[1]-benzopyranno[3,4-b]pyridine, la trans-10-éthoxy-4-propyl-1,2,3,4a,5,10b-hexahydro-4H-[1]-benzopyranno-[3,4-b]pyridine et la trans-7-méthoxy-4,10-diméthyl-1,2,3,4a,5,10b-hexahydro-4H-[1]benzopyranno[3,4-b]pyridine, ou un sel acceptable pour l'usage pharmaceutique de l'un de ces composés.

12. Procédé selon la revendication 1 pour préparer la trans-7-hydroxy-4-propyl-1,2,3,4a,5,10b-hexahydro-4H-[1]-benzopyranno[3,4-b]pyridine selon la revendication 1 ou l'un de ses sels acceptables pour l'usage pharmaceutique.

13. Procédé selon la revendication 1 pour préparer la trans-10-méthoxy-4-propyl-1,2,3,4a,5,10b-hexahydro-4H-[1]-benzopyranno[3,4-b]pyridine selon la revendication 1 ou l'un de ses sels acceptables pour l'usage pharmaceutique.

14. Procédé selon la revendication 1 pour préparer la trans-7-méthoxy-4,10-diméthyl-1,2,3,4a,5,10b-hexahydro-4H-[1]-benzopyranno[3,4-b]pyridine selon la revendication 1 ou l'un de ses sels acceptables pour l'usage pharmaceutique.

15. Procédé selon la revendication 1 pour préparer la trans-7-hydroxy-4,10-diméthyl-1,2,3,4a,5,10b-hexahydro-4H-[1]-benzopyranno[3,4-b]pyridine selon la revendication 1 ou l'un de ses sels acceptables pour l'usage pharmaceutique.

16. Procédé de préparation de compositions pharmaceutiques contenant des composés qu'on peut préparer selon les revendications 1 à 15 ou les sels acceptables pour l'usage pharmaceutique de ces composés, caractérisé en ce que l'on mélange ces composés ou leurs sels avec un véhicule pharmaceutique approprié.